(19) Europäisches Patentamt
European Patent Office
Office européen des brevets

(11) Publication number: **0 386 611 A2**

(12) **EUROPEAN PATENT APPLICATION**

(21) Application number: **90103930.5**

(22) Date of filing: **01.03.90**

(51) Int. Cl.5: **C07K 5/02, C07C 271/22, C07C 237/22, C07D 295/20, C07F 7/18, C07D 233/24, C07D 217/06, C07D 213/40, C07D 263/04, C07D 317/20, A61K 31/16**

(30) Priority: **06.03.89 GB 8905094**
**21.12.89 GB 8928970**

(43) Date of publication of application:
**12.09.90 Bulletin 90/37**

(84) Designated Contracting States:
**AT BE CH DE DK ES FR GB GR IT LI LU NL SE**

(71) Applicant: **F. HOFFMANN-LA ROCHE AG**
**Postfach 3255**
**CH-4002 Basel(CH)**

(72) Inventor: **Branca, Quirico, Dr.**
**2 Lenzgasse**
**CH-4056 Basle(CH)**
Inventor: **Edenhofer, Albrecht, Dr.**
**35 Rudolf Wackernagelstrasse**
**CH-4125 Riehen(CH)**
Inventor: **Martin, Joseph Armstrong**
**10 The Chownes, West Common**
**Harpenden, Hertfordshire(GB)**
Inventor: **Merrett, John Herbert**
**23 Bush Spring**
**Baldock, Hertfordshire(GB)**
Inventor: **Neidhart, Werner, Dr.**
**14 Oltmannstrasse**
**D-7800 Freiburg im Breisgau(DE)**

(74) Representative: **Lederer, Franz, Dr. et al**
**Lederer, Keller & Riederer, Patentanwälte,**
**Lucile-Grahn-Strasse 22**
**D-8000 München 80(DE)**

(54) **Amino acid derivatives.**

(57) Compounds of the formula

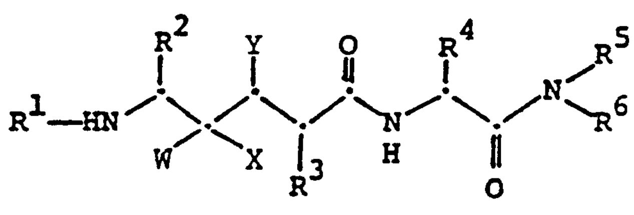

I

wherein $R^1$ represents alkoxycarbonyl, aralkoxycarbonyl, alkanoyl, aralkanoyl, aroyl, cycloalkylcarbonyl, heterocyclylcarbonyl, heterocyclyl-alkanoyl, 6-(dibenzylcarbamoyl)-4-oxohexanoyl or an acyl group of an α-amino acid in which the amino group is substituted by alkoxycaronyl, aralkoxycarbonyl, diaralkylcarbamoyl, diaralkylalkanoyl or aralkanoyl; $R^2$ represents alkyl, cycloalkylalkyl or aralkyl; $R^3$ represents hydrogen or alkyl; $R^4$ represents alkyl; and one of $R^5$ and $R^6$ represents hydrogen and the other represents hydrogen, alkyl, aryl, aralkyl, 1-alkoxycarbonyl-2-phenylethyl, 1-alkoxycarbonyl-2-(imidazol-4-yl)ethyl, 2-(imidazol-1-yl)-ethyl, indanyl, heterocyclyl-alkyl, carboxyalkyl, alkoxycarbonylalkyl, aryloxycarbonylalkyl, aralkoxycarbonylalkyl or a group of the formula -A-N($R^a$)($R^b$) in which A represents alkylene and $R^a$ and $R^b$ each

EP 0 386 611 A2

represent alkyl or $R^a$ and $R^b$ together represent a pentamethylene group in which one methylene group can be replaced by NH, N-alkyl, N-alkanoyl, N-aralkoxy carbonyl, O, S, SO or $SO_2$; or $R^5$ and $R^6$ together with the nitrogen atom to which they are attached represent a 1,2,3,4-tetrahydroisoquinoline ring; one of W and X represents hydrogen and the other represents hydroxy or amino or W and X together represent hydroxyimino and Y represents hydrogen or, where one of W and X represents hydrogen and the other represents hydroxy, Y can also represent hydroxy,
and pharmaceutically acceptable acid addition salts thereof can be used as medicaments for the treatment and prophylaxis of viral infections, particularly of infections caused by HIV and other retroid viruses. They can be manufactured according to generally known procedures.

## Amino Acid Derivatives

The present invention is concerned with amino acid derivatives.

The amino acid derivatives provided by the present invention are compounds of the general formula

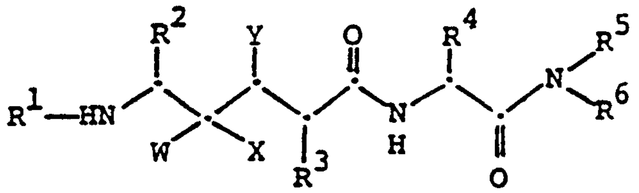

I

wherein $R^1$ represents alkoxycarbonyl, aralkoxycarbonyl, alkanoyl, aralkanoyl, aroyl, cycloalkylcarbonyl, heterocyclylcarbonyl, heterocyclyl-alkanoyl, 6-(dibenzylcarbamoyl)-4-oxohexanoyl or an acyl group of an α-amino acid in which the amino group is substituted by alkoxycarbonyl, aralkoxycarbonyl, diaralkylcarbamoyl, diaralkylalkanoyl or aralkanoyl; $R^2$ represents alkyl, cycloalkylalkyl or aralkyl; $R^3$ represents hydrogen or alkyl; $R^4$ represents alkyl; and one of $R^5$ and $R^6$ represents hydrogen and the other represents hydrogen, alkyl, aryl, aralkyl, 1-alkoxycarbonyl-2-phenylethyl, 1-alkoxycarbonyl-2-(imidazol-4-yl)ethyl, 2-(imidazol-1-yl)ethyl, indanyl, heterocyclyl-alkyl, carboxyalkyl, alkoxycarbonylalkyl, aryloxycarbonylalkyl, aralkoxycarbonylalkyl or a group of the formula $-A-N(R^a)(R^b)$ in which A represents alkylene and $R^a$ and $R^b$ each represent alkyl or $R^a$ and $R^b$ together represent a pentamethylene group in which one methylene group can be replaced by NH, N-alkyl, N-alkanoyl, N-aralkoxycarbonyl, O, S, SO or $SO_2$; or $R^5$ and $R^6$ together with the nitrogen atom to which they are attached represent a 1,2,3,4-tetrahydroisoquinoline ring; one of W and X represents hydrogen and the other represents hydroxy or amino or W and X together represent hydroxyimino and Y represents hydrogen or, where one of W and X represents hydrogen and the other represents hydroxy, Y can also represent hydroxy,
and pharmaceutically acceptable acid addition salts thereof.

The compounds of formula I and their pharmaceutically acceptable acid addition salts are novel and possess valuable pharmacological properties. In particular, they inhibit proteases of viral origin and can be used in the prophylaxis or treatment of viral infections, particularly of infections caused by HIV and other retroid viruses.

Objects of the present invention are the compounds of formula I and their aforementioned salts per se and for use as therapeutically active substances, a process for the manufacture of said compounds and salts, intermediates used in said process, medicaments containing said compounds and salts, the use of said compounds and salts in the control or prevention of illnesses, especially in the treatment or prophylaxis of viral infections, and the use of said compounds and salts for the manufacture of medicaments for the treatment or prophylaxis of viral infections.

As used in this Specification, the term "alkyl", alone or in combination, means a straight-chain or branched-chain alkyl group containing a maximum of 8, preferably a maximum of 4, carbon atoms such as methyl, ethyl, n-propyl, isopropyl, n-butyl, isobutyl, sec.butyl, tert.butyl, n-pentyl, 2,2-dimethylpropyl, 3-methylbutyl and the like. The term "alkylene" means a straight-chain or branched-chain alkylene group containing a maximum of 8, preferably a maximum of 4, carbon atoms such as methylene, ethylene, 1,3-propylene, 2-methylpropylene etc. The term "alkoxy", alone or in combination, means an alkyl ether group in which the term "alkyl" has the significance given earlier, such as methoxy, ethoxy, n-propoxy, isopropoxy, n-butoxy, isobutoxy, sec.butoxy, tert.butoxy and the like. The cycloalkyl part of a cycloalkylcarbonyl or cycloalkylalkyl group is a cycloalkyl group containing 3-8, preferably 3-6, carbon atoms such as cyclopropyl, cyclobutyl, cyclopentyl, cyclohexyl and the like. The term "aryl" means a phenyl or naphthyl group which optionally carries one or more substituents selected from alkyl, hydroxy, alkoxy and halogen, such as phenyl, p-tolyl, 4-methoxyphenyl, 4-chlorophenyl, 4-hydroxyphenyl, 2-naphthyl etc. The term "aralkyl" means an alkyl group as defined earlier in which one hydrogen atom is replaced by an aryl group as defined earlier, such as benzyl, 2-phenylethyl and the like. Dibenzylcarbamoyl is an example of a diaralkylcarbamoyl group. The term "aralkoxycarbonyl" means a group of the formula -C(O)-O-aralkyl in which the term "aralkyl" has the significance given earlier, such as benzyloxycarbonyl etc. The term "alkanoyl", alone or in combination, means an acyl group derived from an alkanecarboxylic acid, such as

acetyl, propionyl, butyryl, valeryl, tert.butylacetyl, 4-methylvaleryl etc. Dibenzylacetyl is an example of a diaralkylalkanoyl group. The term "aralkanoyl" means an acyl group derived from an aryl-substituted alkanecarboxylic acid such as phenylacetyl, 3-phenylpropionyl, 2,3,4-trimethoxyhydrocinnamoyl, 4-phenylbutyryl, (2-naphthyl)acetyl etc. The term "aroyl" means an acyl group derived from an aromatic carboxylic acid such as benzoyl, 1-naphthoyl, 2-naphthoyl etc. The term "heterocyclyl" means a saturated, partially unsaturated or aromatic monocyclic, bicyclic or tricyclic heterocycle which contains one or more hetero atoms selected from nitrogen, oxygen and sulphur, which is optionally substituted on one or more, preferably one or two, carbon atoms by alkyl, alkoxy and/or halogen and which is attached via a carbon atom. Examples of such heterocyclyl groups are pyrrolidinyl, piperidinyl, piperazinyl, morpholinyl, thiomorpholinyl, pyrrolyl, imidazolyl, pyrazolyl, pyridyl, pyrazinyl, pyrimidinyl, furyl, thienyl, triazolyl, oxazolyl, thiazolyl, indolyl, quinolyl, isoquinolyl, 1,2,3,4-tetrahydroisoquinolyl, quinoxalinyl, β-carbolinyl and the like. An acyl group of an α-amino acid in which the amino group is substituted in the manner defined earlier can be derived from a natural α-amino acid such as glycine, phenylalanine, asparagine, leucine, isoleucine, glutamine, histidine and the like or from a non-natural α-amino acid such as cyanoalanine, norleucine, norvaline and the like, with examples of such acyl groups being N-dibenzylacetylglycyl, N-dibenzylcarbamoylphenylalanyl, N-benzyloxycarbonylasparaginyl, N-benzyloxycarbonylleucyl, N-benzyloxycarbonylisoleucyl, N-benzyloxycarbonylglutaminyl, N-dibenzylcarbamoylhistidyl, N-(2,3,4-trimethoxyhydrocinnamoyl)histidyl, N-benzyloxycarbonyl-β-cyanoalanyl and the like. When $R^a$ and $R^b$ together represent a pentamethylene group in which one methylene group can be replaced in the manner defined earlier, the group -N($R^a$)($R^b$) can be, for example, piperidino, piperazino, N-methylpiperazino, N-acetylpiperazino, N-benzyloxycarbonylpiperazino, morpholino, thiomorpholino, thiomorpholino 4-oxide, thiomorpholine 4,4-dioxide etc. The term "halogen" means fluorine, chlorine, bromine and iodine.

The compounds of formula I form pharmaceutically acceptable acid addition salts with inorganic acids, for example hydrohalic acids such as hydrochloric acid or hydrobromic acid, sulphuric acid, nitric acid, phosphoric acid etc, and with organic acids, for example acetic acid, citric acid, maleic acid, fumaric acid, tartaric acid, methanesulphonic acid, p-toluenesulphonic acid etc.

The compounds of formula I contain at least three asymmetric carbon atoms and are therefore present in the form of optically pure diastereoisomers, mixtures of diastereoisomers, diastereoisomeric racemates or mixtures of diastereoisomeric racemates. The present invention includes within its scope all of these forms. Mixtures of diastereoisomers, diastereoisomeric racemates or mixtures of diastereoisomeric racemates can be separated according to conventional methods; for example, by column chromatography, thin-layer chromatrography, high pressure liquid chromatography etc.

One particular group of compounds of formula I comprises those in which $R^1$ represents alkoxycarbonyl, aralkoxycarbonyl, alkanoyl, aralkanoyl, aroyl, cycloalkylcarbonyl, heterocyclylcarbonyl, heterocyclylalkanoyl, 6-(dibenzylcarbamoyl)-4-oxohexanoyl or an acyl group of a natural α-amino acid in which the amino group is substituted by aralkoxycarbonyl, diaralkylcarbamoyl, diaralkylalkanoyl or aralkanoyl; one of $R^5$ and $R^6$ represents hydrogen and the other represents hydrogen, alkyl, aryl, aralkyl, 1-alkoxycarbonyl-2-phenylethyl, 1-alkoxycarbonyl-2-(imidazol-4-yl)ethyl, 2-(imidazol-1-yl)ethyl, indanyl, heterocyclyl-alkyl or a group of the formula -A-N($R^a$)($R^b$) in which A represents alkylene and $R^a$ and $R^b$ each represent alkyl or $R^a$ and $R^b$ together represent a pentamethylene group in which one methylene group can be replaced by NH, N-alkyl, N-alkanoyl, O, S, SO or $SO_2$; or $R^5$ and $R^6$ together with the nitrogen atom to which they are attached represent a 1,2,3,4-tetrahydroisoquinoline ring; one of W and X represents hydrogen and the other represents hydroxy and Y represents hydrogen; and $R^2$, $R^3$ and $R^4$ have the significance given earlier, and wherein the term "aryl" used alone or in combination means a phenyl or naphthyl group which optionally carries one or more substituents selected from alkyl, alkoxy and halogen.

In formula I hereinbefore $R^1$ preferably represents alkoxycarbonyl, aralkoxycarbonyl, alkanoyl or aroyl or an acyl group of an α-amino acid in which the amino group is substituted in the manner defined earlier, especially tert.butoxycarbonyl, benzyloxycarbonyl, acetyl, tert.butylacetyl, 4-methylvaleryl, p-toluoyl, N-benzyloxycarbonylasparaginyl or N-benzyloxycarbonyl-β-cyanoalanyl. $R^2$ preferably represents isobutyl, cyclohexylmethyl or benzyl. $R^3$ preferably represents alkyl, especially methyl or isopropyl. Preferably, $R^4$ represents isobutyl or sec.butyl. Preferably, one of $R^5$ and $R^6$ represents hydrogen and the other represents hydrogen, alkyl, aralkyl, 1-alkoxycarbonyl-2-phenylethyl, 1-alkoxycarbonyl-2-(imidazol-4-yl)ethyl, 2-(imidazol-1-yl)ethyl, indanyl, heterocyclyl-alkyl or a group of the formula -A-N($R^a$)($R^b$), especially hydrogen, isobutyl, benzyl, 2-phenylethyl, 2-(4-hydroxyphenyl)ethyl, 1-methoxycarbonyl-2-phenylethyl, 1-methoxycarbonyl-2-(imidazol-4-yl)ethyl, 2-(imidazol-1-yl)ethyl, 2-indanyl, 2-(2-pyridyl)ethyl, 2-(dimethylamino)ethyl, 2-morpholinoethyl or 2-[4-(benzyloxycarbonyl)-1-piperazinyl]ethyl, or $R^5$ and $R^6$ together with the nitrogen atom to which they are attached represent a 1,2,3,4-tetrahydroisoquinoline ring. Preferably, one of W and X represents hydrogen and the other represents hydroxy and Y represents

hydrogen or hydroxy.

From the foregoing it will be evident that particularly preferred compounds of formula I are those in which $R^1$ represents tert.butoxycarbonyl, benzyloxycarbonyl, acetyl, tert.butylacetyl, 4-methylvaleryl, p-toluoyl, N-benzyloxycarbonylasparaginyl or N-benzyloxycarbonyl-β-cyanoalanyl, $R^2$ represents isobutyl, cyclohexylmethyl or benzyl, $R^3$ represents methyl or isopropyl, $R^4$ represents isobutyl or sec.butyl and one of $R^5$ and $R^6$ represents hydrogen and the other represents hydrogen or isobutyl, benzyl, 2-phenylethyl, 2-(4-hydroxyphenyl)ethyl, 1-methoxycarbonyl-2-phenylethyl, 1-methoxycarbonyl-2-(imidazol-4-yl)ethyl, 2-(imidazol-1-yl)ethyl, 2-indanyl, 2-(2-pyridyl)ethyl, 2-(dimethylamino)ethyl, 2-morpholinoethyl or 2-[4-(benzyloxycarbonyl)-1-piperazinyl]ethyl, or $R^5$ and $R^6$ together with the carbon atom to which they are attached represent a 1,2,3,4-tetrahydroisoquinoline group; and one of W and X represents hydrogen and the other represents hydroxy and Y represents hydrogen or hydroxy.

The most preferred compounds of formula I are:

$N^2$-[5(S)-(tert.Butoxyformamido)-6-cyclohexyl-4(S)-hydroxy-2(S)-isopropylhexanoyl]-$N^1$-phenethyl-L-isoleucinamide,

N-[N-[5(S)-(tert.butoxyformamido)-6-cyclohexyl-4(S)-hydroxy-2(S)-isopropylhexanoyl]-L-isoleucyl]-L-phenylalanine methyl ester,

$N^2$-[5(S)-(tert.butoxyformamido)-6-cyclohexyl-4(S)-hydroxy-2(S)-isopropylhexanoyl]-$N^1$-benzyl-L-isoleucinamide,

$N^2$-[5(S)-(tert.butoxyformamido)-6-cyclohexyl-4(S)-hydroxy-2(S)-isopropylhexanoyl]-$N^1$-[2-(2-pyridyl)ethyl]-L-isoleucinamide,

$N^2$-[5(S)-(tert.butoxyformamido)-6-cyclohexyl-4(S)-hydroxy-2(S)-isopropylhexanoyl]-$N^1$-(2-indanyl)-L-isoleucinamide,

$N^2$-[5(S)-(tert.butoxyformamido)-6-cyclohexyl-4(S)-hydroxy-2(S)-isopropylhexanoyl]-$N^1$-(2-morpholinoethyl)-L-isoleucinamide,

$N^2$-[5(S)-(tert.butoxyformamido)-4(S)-hydroxy-2(S)-isopropyl-6-phenylhexanoyl]-$N^1$-phenethyl-L-isoleucinamide,

$N^2$-[5(S)-benzyloxyformamido-6-cyclohexyl-4(S)-hydroxy-2(S)-isopropylhexanoyl]-$N^1$-phenethyl-L-isoleucinamide,

$N^2$-[5(S)-[[N-(benzyloxycarbonyl)-L-asparaginyl]amino]-6-cyclohexyl-4(S)-hydroxy-2(S)-isopropylhexanoyl]-$N^1$-phenethyl-L-isoleucinamide,

$N^2$-[5(S)-(tert.butylacetamido)-6-cyclohexyl-4(S)-hydroxy-2(S)-isopropylhexanoyl]-$N^1$-phenethyl-L-isoleucinamide,

$N^2$-[5(S)-[[N-(benzyloxycarbonyl)-β-cyano-L-alanyl]amino]-6-cyclohexyl-4(S)-hydroxy-2(S)-isopropylhexanoyl]-$N^1$-phenethyl-L-isoleucinamide,

$N^1$-[2-[4-(benzyloxycarbonyl)-1-piperazinyl]ethyl]-$N^2$-[5(S)-(tert.butoxyformamido)-6-cyclohexyl-4(S)-hydroxy-2(S)-isopropylhexanoyl]-L-isoleucinamide,

$N^2$-[5(S)-(tert.butoxyformamido)-6-cyclohexyl-4(S)-hydroxy-2(S)-isopropylhexanoyl]-$N^1$-[2-(4-hydroxyphenyl)-ethyl]-L-isoleucinamide and

$N^1$-[2-[4-(benzyloxycarbonyl)-1-piperazinyl]ethyl]-$N^2$-[5(S)-(tert.butoxyformamido)-6-cyclohexyl-3(R),4(R)-dihydroxy-2(R)-isopropylhexanoyl]-L-isoleucinamide.

According to the process provided by the present invention, the compounds of formula I and their pharmaceutically acceptable acid addition salts are manufactured by

(a) for the manufacture of a compound of formula I in which $R^1$ represents alkoxycarbonyl or aralkoxycarbonyl, one of W and X represents hydrogen and the other represents hydroxy and Y represents hydrogen or hydroxy, treating a compound of the general formula

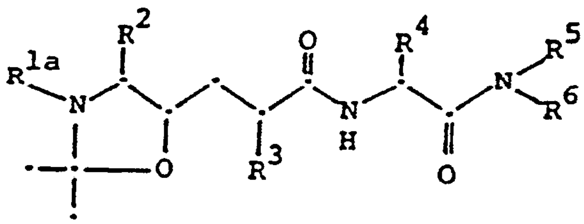

II

or

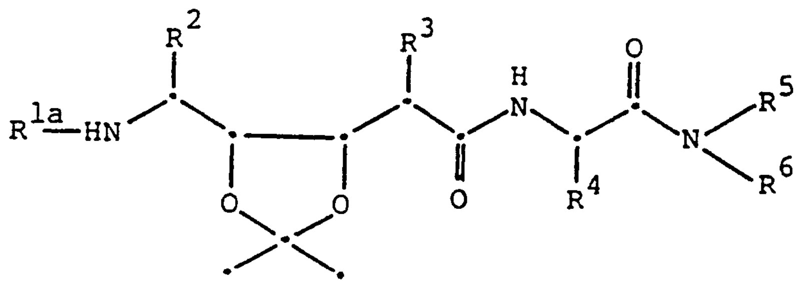

III

wherein $R^{1a}$ represents alkoxycarbonyl or aralkoxycarbonyl and $R^2$, $R^3$, $R^4$, $R^5$ and $R^6$ have the significance given earlier,
with an acid, or

(b) for the manufacture of a compound of formula I in which one of W and X represents hydrogen and the other represents hydroxy and Y represents hydrogen or hydroxy, reacting a compound of the general formula

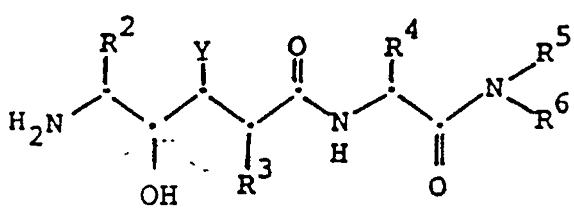

IV

wherein $R^2$, $R^3$, $R^4$, $R^5$, $R^6$ and Y have the significance given earlier,
with an acylating agent which introduces a group $R^1$ as defined earlier, or

(c) for the manufacture of a compound of formula I in which one of W and X represents hydrogen and the other represents hydroxy and Y represents hydrogen, cleaving off the trialkylsilyl protecting group from a compound of the general formula

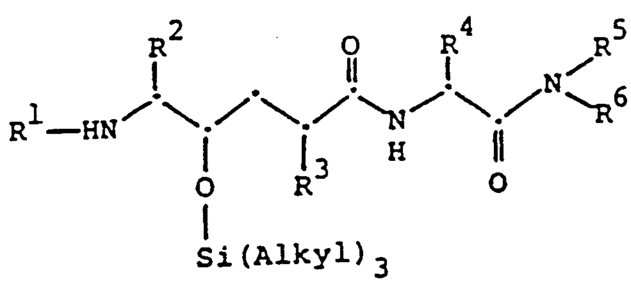

V

wherein $R^1$, $R^2$, $R^3$, $R^4$, $R^5$ and $R^6$ have the significance given earlier,

or

(d) for the manufacture of a compound of formula I in which $R^1$ represents alkanoyl, one of W and X represents hydrogen and the other represents hydroxy and Y represents hydrogen, treating a compound of the general formula

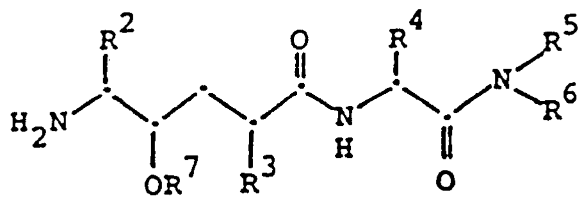

VI

wherein $R^2$, $R^3$, $R^4$, $R^5$ and $R^6$ have the significance given earlier and $R^7$ represents alkanoyl, with a base, or

(e) for the manufacture of a compound of formula I in which W and X together represent hydroxyimino and Y represents hydrogen, reacting a compound of the general formula

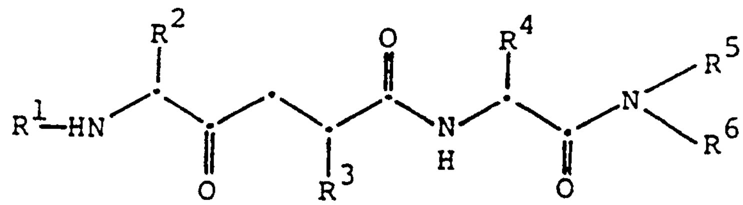

VII

wherein $R^1$, $R^2$, $R^3$, $R^4$, $R^5$ and $R^6$ have the significance given earlier, with hydroxylamine, or

(f) for the manufacture of a compound of formula I in which one of W and X represents hydrogen and the other represents amino and Y represents hydrogen, reducing a compound of formula I in which W and X together represent hydroxyimino and Y represents hydrogen, or

(g) for the manufacture of a compound of formula I in which $R^1$ represents N-(diaralkylcarbamoyl or aralkanoyl)-histidyl, one of $R^5$ and $R^6$ represents hydrogen and the other represents hydrogen, alkyl, aryl, aralkyl, 1-alkoxycarbonyl-2-phenylethyl, 1-alkoxycarbonyl-2-(imidazol-4-yl)ethyl, 2-(imidazol-1-yl)ethyl, indanyl, heterocyclyl-alkyl, carboxyalkyl, alkoxycarbonylalkyl, aryloxycarbonylalkyl or a group or the formula -A-N($R^a$)($R^b$) in which A represents alkylene and $R^a$ and $R^b$ each represent alkyl or $R^a$ and $R^b$ together represents a pentamethylene group in which one methylene group can be replaced by NH, N-alkyl, N-alkanoyl, O, S, SO or $SO_2$ or $R^5$ and $R^6$ together with the nitrogen atom to which they are attached represents a 1,2,3,4-tetrahydroisoquinoline ring, one of W and X represents hydrogen and the other represents hydroxy and Y represents hydrogen or hydroxy, hydrogenolyzing a compound of the general formula

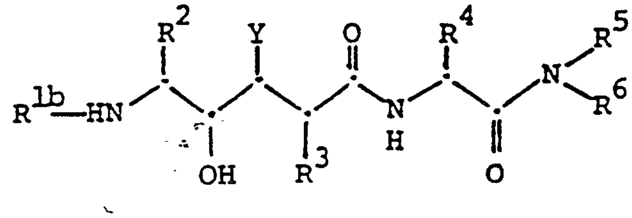

VIII

wherein $R^{1b}$ represents 3-(aralkoxymethyl)-N-(diaralkylcarbamoyl or aralkanoyl)-histidyl and $R^2$, $R^3$, $R^4$, $R^5$, $R^6$ and Y have the significance given earlier,

or

(h) for the manufacture of a compound of formula I in which $R^1$ represents alkoxycarbonyl, alkanoyl, aralkanoyl, aroyl, cycloalkylcarbonyl, heterocyclylcarbonyl, heterocyclyl-alkanoyl, 6-(dibenzylcarbamoyl)-4-oxohexanoyl or an acyl group of an α-amino acid in which the amino group is substituted by alkoxycarbonyl, diaralkylcarbamoyl, diaralkylalkanoyl or aralkanoyl and one of $R^5$ and $R^6$ represents hydrogen and the other represents carboxyalkyl or a group of the formula -A-N($R^a$)($R^b$) in which $R^a$ and $R^b$ together represent a pentamethylene group in which one methylene group is replaced by NH, hydrogenolyzing a corresponding compound of formula I in which one of $R^5$ and $R^6$ represents hydrogen and the other represents aralkoxycarbonylalkyl or a group of the formula -A-N($R^a$)($R^b$) in which $R^a$ and $R^b$ together represents a pentamethylene group in which one methylene group is replaced by N-aralkoxycarbonyl, and

(i) if desired, converting a compound of formula I obtained into a pharmaceutically acceptable acid addition salt.

In accordance with embodiment (a) of the process, the treatment of a compound of formula II with an acid yields a compound of formula I in which $R^1$ represents alkoxycarbonyl or aralkoxycarbonyl, one of W and X represents hydrogen and the other represents hydroxy and Y represents hydrogen, whereas the treatment of a compound of formula III with an acid yields a compound of formula I in which $R^1$ represents alkoxycarbonyl or aralkoxycarbonyl, one of W and X represents hydrogen and the other represents hydroxy and Y represents hydroxy. The type of acid which is used in this embodiment depends essentially on the nature of the substituent $R^{1a}$ present in the starting material of formula II or III. When $R^{1a}$ represents alkoxycarbonyl (e.g. tert.butoxycarbonyl), the treatment is preferably carried out using a strong organic acid, especially an organic sulphonic acid such as an alkanesulphonic acid (e.g. methanesulphonic acid etc) or an aromatic sulphonic acid (e.g. benzenesulphonic acid, p-toluenesulphonic acid, mesitylenesulphonic acid etc) and in the presence of an organic solvent which is inert under the reaction conditions, such as an alkanol (e.g. methanol, ethanol etc). In place of an organic sulphonic acid there can, however, also be used, for example, a halogenated alkanecarboxylic acid such as trifluoroacetic acid etc. When $R^{1a}$ represents aralkoxycarbonyl (e.g. benzyloxycarbonyl), the treatment is preferably carried out using hydrogen halide, for example hydrogen halide in an alkanol such as hydrogen chloride in methanol, and in the presence of an organic solvent which is inert under the reaction conditions, such as a halogenated aliphatic hydrocarbon (e.g. dichloromethane etc). The treatment of a compound of formula II or III with an acid is conveniently carried out at a temperature between about 0° C and about 30° C, preferably at about room temperature.

The reaction of a compound of formula IV with an acylating agent in accordance with embodiment (b) of the process cen be carried out in a manner known per se using as the acylating agent a corresponding acid or a reactive derivative thereof. Suitable reactive derivatives are the acid halides (e.g. acid chlorides), acid anhydrides, mixed anhydrides, activated esters etc. The reaction is conveniently carried out in an organic solvent which is inert under the reaction conditions, for example a halogenated hydrocarbon such as dichloromethane, dimethylformamide, acetonitrile, ethyl acetate etc. When the acylating agent is an α-amino acid, the reaction is expediently carried out in the presence of a condensation agent such as ethyldiisopropylamine, dicyclohexylcarbodiimide or benzotriazol-1-yloxy-tris(dimethylamino)-phosphonium hexafluorophosphate and ethyldiisopropylamine, and the like. The reaction is conveniently carried out at a temeprature between about 0° C and room temperature, preferably at about room temperature.

The cleavage of the trialkylsilyl protecting group from a compound of formula V in accordance with embodiment (c) of the process is expediently carried out using triethylamine trihydrofluoride, optionally in an organic solvent which is inert under the reaction conditions, such as a cyclic ether (e.g. tetrahydrofuran etc), and at about room temperature. The cleavage can, however, also be carried out using other reagents such as tetra-n-butylammonium fluoride and the like.

The treatment of a compound of formula VI with a base in accordance with embodiment (d) of the process results in the migration of the alkanoyl group $R^7$ from the oxygen atom to the adjacent amino group. Suitable bases which can be used in this embodiment are alkali metal carbonates (e.g. sodium carbonate, potassium carbonate etc) and alkali metal hydrogen carbonates (e.g. sodium hydrogen carbonate, potassium hydrogen carbonate etc). This treatment is conveniently carried out in an organic solvent which is inert under the reaction conditions, such as a halogenated hydrocarbon (e.g. dichloromethane etc). Conveniently, this treatment is carried out at about room temperature to about 40° C, preferably at about room temperature.

The reaction of a compound of formula VII with hydroxylamine in accordance with embodiment (e) of the process can be carried out in a manner known per se. Conveniently, the reaction is carried out using a hydroxylamine salt, preferably hydroxylamine hydrochloride, in the presence of a tertiary organic base such as dimethylaminopyridine or the like and in an inert organic solvent such as pyridine or the like. This reaction is expediently carried out at a temperature between about 0° C and room temperature, preferably at room temperature.

The reduction of a compound of formula I in which W and X together represent hydroxyimino and Y represents hydrogen in accordance with embodiment (f) of the process can also be carried out in a manner known per se. For example, the reduction can be carried out using hydrogen in the presence of a suitable catalyst and in an organic solvent which is inert under the reaction conditions, such as an alcohol (e.g. methanol, ethanol etc), expediently at about room temperature and under atmospheric pressure. Preferably, the reduction is carried out in alkanolic ammonia (e.g. methanolic ammonia). In a preferred aspect of this embodiment, a Raney nickel catalyst is used, whereby any hydrogenolytically cleavable group present in the compound of formula I is retained. Where R in the compound of formula I represents other than aralkoxycarbonyl, a palladium catalyst (e.g. palladium-on-carbon) can e used in place of a Raney nickel catalyst, whereby a hydrogenolytically cleavable group present as or on one of $R^5$ and $R^6$ is cleaved.

The hydrogenolysis of a compound of formula VIII in accordance with embodiment (g) of the process can be carried out in a manner known per se. Suitably, the hydrogenolysis is carried out using a palladium catalyst such as palladium-on-carbon. Conveniently, the hydrogenolysis is carried out in an acidic medium, for example an aqueous-alkanolic mineral acid such as methanolic hydrochloric acid or an aqueous alkanecarboxylic acid such as aqueous acetic acid. The hydrogenolysis is conveniently carried out at about room temperature and under atmospheric pressure.

The hydrogenolysis in accordance with embodiment (h) of the process can also be carried out in a manner known per se. For example, the hydrogenolysis can be carried out using a palladium catalyst such as palladium-on-carbon in an organic solvent which is inert under the reaction conditions, such as an alkanol (e.g. ethanol etc) or an alkanoic acid ester (e.g. ethyl acetate etc). Conveniently, the hydrogenolysis is carried out at about room temperature and under atmospheric pressure.

The conversion of a compound of formula I into a pharmaceutically acceptable acid addition salt in accordance with embodiment (i) of the process can be carried out by treating such a compound in a conventional manner with an inorganic acid, for example a hydrohalic acid such as hydrochloric acid or hydrobromic acid, sulphuric acid, nitric acid, phosphoric acid etc, or with an organic acid such as acetic acid, citric acid, maleic acid, fumaric acid, tartaric acid, methanesulphonic acid, p-toluenesulphonic acid etc.

The compounds of formula II hereinbefore, which are used as starting materials in embodiment (a) of the process, are novel and also form an object of the present invention. They can be prepared, for example, by firstly reacting a compound of the general formula

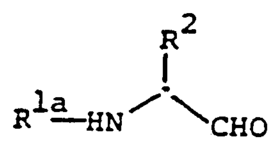

IX

wherein $R^{1a}$ and $R^2$ have the significance given earlier,
with a compound of the general formula

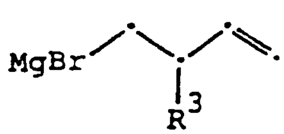

X

wherein $R^3$ has the significance given earlier,
in a Grignard reaction to give a compound of the general formula

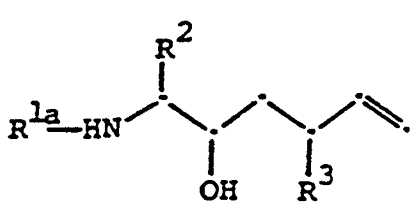

XI

wherein $R^{1a}$, $R^2$ and $R^3$ have the significance given earlier.

The reaction of a compound of formula IX with a compound of formula X is carried out according to methods known per se; for example, in an organic solvent which is inert under the reaction conditions, such as an ether (e.g. diethyl ether), and at a temperature between about 0° C and about 40° C, preferably at about room temperature.

A compound of formula XI is then converted into a compound of the general formula

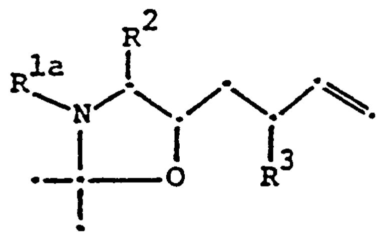

XII

by treatment with 2,2-dimethoxypropane in the presence of a strong organic acid such as p-toluenesulphonic acid, conveniently at about room temperature.

Subsequently, a compound of formula XII is then oxidized to give a compound of the general formula

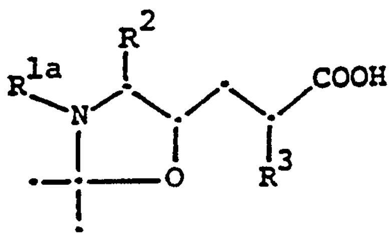

XIII

wherein $R^{1a}$, $R^2$ and $R^3$ have the significance given earlier.

The oxidation of a compound of formula XII is expediently carried out using an alkali metal permanganate such as potassium permanganate at about room temperature. Conveniently, the oxidation is carried out in a solvent system comprising a mixture of water, an alkanecarboxylic acid such as glacial acetic acid and an inert organic solvent which is not miscible therewith (e.g. an aromatic hydrocarbon such as benzene, toluene etc) and in the presence of a phase-transfer catalyst.

The final step for the preparation of the compounds of formula II comprises reacting a compound of formula XIII with a compound of the general formula

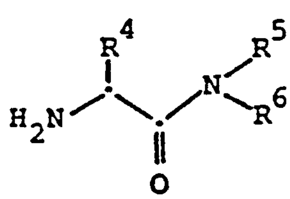

XIV

wherein $R^4$, $R^5$ and $R^6$ have the significance given earlier.

The reaction of a compound of formula XIII with a compound of formula XIV is carried out according to methods known per se. Thus, the reaction is conveniently carried out in the presence of a condensation agent such as dicyclohexylcarbodiimide and 1-hydroxybenzotriazole or benzotriazol-1-yloxy-tris-(dimethylamino)-phosphonium hexafluorophosphate and ethyldiisopropylamine and the like in an organic solvent which is inert under the reaction conditions, such as a halogenated aliphatic hydrocarbon (e.g. dichloromethane etc), dimethylformamide, acetonitrile, tetrahydrofuran etc. Suitably, the reaction is carried out at about 0° C to about 40° C, preferably at about room temperature.

The compounds of formula III hereinbefore, which are also used as starting materials in embodiment (a) of the process, are novel and also form an object of the present invention. They can be prepared, for

example, by reacting a compound of the general formula

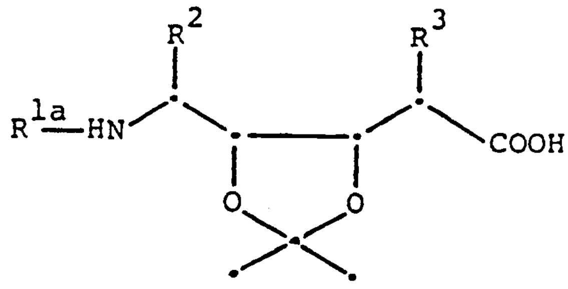

XV

wherein $R^{1a}$, $R^2$ and $R^3$ have the significance given earlier, with a compound of formula XIV hereinbefore.

The reaction of a compound of formula XV with a compound of formula XIV can be carried out in a manner analogous to that described earlier in connection with the reaction of a compound of formula XIII with a compound of formula XIV.

The compounds of formula IV, which are used as starting materials in embodiment (b) of the process, are novel and also form an object of the present invention. They can be prepared, for example, by treating a compound of formula I hereinbefore in which $R^1$ represents tert.butoxycarbonyl, one of W and X represents hydrogen and the other represents hydroxy and Y represents hydrogen or hydroxy with hydrogen halide in a manner know per se, conveniently in an organic solvent which is inert under the reaction conditions, such as an alkanol (e.g. methanol) and using a solution of a hydrogen halide in the same solvent (e.g. hydrogen chloride in an alkanol such as hydrogen chloride in methanol). Suitably, this treatment is carried out at about room temperature. Alternatively, the compounds of formula IV can be prepared by hydrogenolyzing a compound of formula I in which $R^1$ represents benzyloxycarbonyl, one of W and X represents hydrogen and the other represents hydroxy and Y represents hydrogen or hydroxy in an analogous manner to that described earlier in connection with embodiment (g) of the process.

The acylating agents which are used in embodiment (b) of the process, insofar as they are not know compounds, can be prepared in an analogous manner to the known compounds or as described in the Examples hereinafter or in analogy thereto.

The compounds of formula V hereinbefore, which are used as starting materials in embodiment (c) of the process, are novel and also form an object of the present invention. They can be prepared, for example, by reacting a compound of formula I hereinbefore in which $R^1$ represents tert.butoxycarbonyl, one of W and X represents hydrogen and the other represents hydroxy and Y represents hydrogen with an excess of an agent which introduces a trialkylsilyl group, whereby a trialkylsilyl group is introduced at the hydroxy group and the tert.butoxycarbonyl group is replaced by a trialkylsilyloxycarbonyl group. This reaction is carried out in a known manner. Suitable agents which introduce a trialkylsilyl group are trialkylsilyl trifluoromethanesulphonates such as tert.butyldimethylsilyl trifluoromethanesulphonate etc. The reaction is conveniently carried out in an organic solvent which is inert under the reaction conditions, such as halogenated aliphatic hydrocarbon (e.g. dichloromethane etc), and in the presence of an organic base such as pyridine, 2,6-lutidine etc. Conveniently, the reaction is carried out at about room temperature to about 40 °C, preferably at about room temperature. The trialkylsilyloxycarbonyl group is then cleaved off with the formation of an amino group, while the trialkylsilyloxy group is retained. This cleavage is carried out using, for example, tetra-n-butylammonium fluoride. Finally, the amine obtained is reacted with an acylating agent which yields a group $R^1$ in an analogous manner to that described earlier in connection with embodiment (b) of the process.

The compounds of formula VI, which are used as starting materials in embodiment (d) of the process, are novel and form a further object of the present invention. They can be prepared by reacting a compound of formula I in which $R^1$ represents tert.butoxycarbonyl, one of W and X represents hydrogen and the other represents hydroxy and Y represents hydrogen with an alkanoylating agent and treating the reaction product with hydrogen halide. The reaction with an alkanoylating agent can be carried out accoridng to methods known per se. Especially suitable alkanoylating agents are the corresponding acid halides (e.g. acid chlorides) or acid anhydrides. For example, the reaction can be carried out in a solvent which is inert under the reaction conditions, such as a halogenated aliphatic hydrocarbon (e.g. dichloromethane etc). Conveniently, the reaction is carried out in the presence of an organic base such as triethylamine, pyridine, 4-dimethylaminopyridine etc. The reaction is conveniently carried out at a temperature between about 0 °C

and about 40° C, especially at about room temperature. The treatment of the alkanoylation product with hydrogen halide, which results in the cleavage of the tert.butoxycarbonyl group, is carried out in the manner known per se. Suitably, the treatment is carried out using hydrogen halide, especially hydrogen chloride, in an organic solvent which is inert under the reaction conditions, such as ethyl acetate, at about 0° C to about 40° C, especially at about room temperature.

The compounds of formula VII, which are used as starting materials in embodiment (e) of the process, are novel and also form an object of the present invention. They can be prepared, for example, by oxidizing a compound of formula I in which one of W and X represents hydrogen and the other represents hydroxy and Y represents hydrogen. This oxidation can be carried out according to known methods; for example, using a sulphur trioxide/pyridine complex in dimethyl sulphoxide, oxalyl chloride in dimethyl sulphoxide or trifluoroacetic anhydride in dimethyl sulphoxide, in each case in the presence of an organic base such as a trialkylamine (e.g. triethylamine etc). Conveniently, the reaction is carried out at a temperature between about 0° C and room temperature, preferably at room temperature.

The compounds of formula VIII, which are used as starting materials in embodiment (g) of the process, are novel and are also an object of the present invention. They can be prepared, for example, by reacting a compound of formula IV hereinbefore with a 3-(aralkoxymethyl)-N-(diaralkylcarbamoyl or aralkanoyl)-histidine in a manner analogous to that described earlier in connection with embodiment (b) of the process.

The compounds of formulae (IX, X, XIV and XV hereinbefore, which are used in the preparation of the starting materials of formulae II and III, are known compounds or analogues of known compounds which can be prepared in a similar manner to the known compounds. Moreover, the Examples hereinafter contain details concerning the preparation of certain compounds of formula XIV.

The 3-(aralkoxymethyl)-N-(diaralkylcarbamoyl or aralkanoyl)-histidines, which are used in the preparation of the starting materials of formula VIII, can be prepared as described in the Examples hereinafter or in analogy thereto.

As mentioned earlier, the compounds of formula I and their pharmaceutically acceptable acid addition salts inhibit proteases of viral origin and are useful in the treatment or prophylaxis of viral infections, particularly of infections caused by HIV and other retroid viruses.

The in vitro inhibition of HIV protease by the compounds provided by the present invention can be demonstrated by means of the following test:

HIV protease was expressed in E. coli and partially purified from soluble extracts of the bacterium by ammonium sulphate fractionation (0-30%). Protease activity was assayed using as the substrate the protected hexapeptide succinyl-Ser-Leu-Asn-Tyr-Pro-Ile isobutylamide ($S^1$) or the protected heptapeptide succinyl-Val-Ser-Gln-Asn-Phe-Pro-Ile isobutylamide ($S^2$). Cleavage of the substrate was quantified by measuring the production of H-Pro-Ile isobutylamide by the spectrophotometric assay of N-terminal proline.

1.25 mM of substrate $S^1$ or 0.68 mM of substrate $S^2$ were dissolved in 125 mM of citrate buffer (pH 5.5) containing 0.125 mg/ml of Tween 20. 10 µl of a solution of various concentrations of the test compound (dissolved in methanol or dimethyl sulphoxide and diluted with water containing 0.1% Tween 20) and 10 µl of protease were added to 80 µl of the respective buffered substrate. Digestion was carried out at 37° C for a fixed period of time and was terminated by the addition of 1 ml of colour reagent [30 µg/ml of isatin and 1.5 mg/ml of 2-(4-chlorobenzoyl)benzoic acid in 10% acetone in ethanol (vol./vol.)]. The solution was heated in a boiling water bath for 15 minutes and then the pigmented residues were re-dissolved in 1 ml of 1% pyrogallol in 33% water in acetone (wt./vol./vol.). The optical density of the solution was measured spectrophotometrically at 599 nm. The formation of H-Pro-Ile isobutylamide in the presence of the test compound was compared with controls and the concentration of test compound required to give 50% inhibition ($IC_{50}$) was determined by means of a graph plotted from the various concentrations of test compound used.

In the above test the determination of the $IC_{50}$ values for potent inhibitors is limited by mutual depletion. In this respect, substrate $S^2$ is more sensitive than substrate $S^1$ and this allows reduced protease concentrations to be used, which results in lower $IC_{50}$ values. This will be seen from the results presented in the Table hereinafter where in turn the 2nd run with substrate $S^2$ was carried out using a lower protease concentration than in the 1st run which results again in lower $IC_{50}$ values.

The in vitro antiviral activity of the compounds provided by the invention can be demonstrated in the assay described below:

This assay uses HTLV-III (strain RF) grown in C8166 cells (a human $CD4^+$ T lymphoblastoid line) using RPM1 1640 medium with bicarbonate buffer, antibiotics and 10% foetal bovine serum.

A suspension of cells is infected with ten times the $TCD_{50}$ of virus and adsorption allowed to proceed for 90 minutes at 37° C. The cells are washed three times with medium. The test is carried out in 6 ml tissue culture tubes, each tube containing 2 x $10^5$ infected cells in 1.5 ml of medium. Test compounds are

dissolved in either aqueous medium or dimethyl sulphoxide, according to solubility, and a 15 µl solution of the compound added. The cultures are incubated at 37°C for 72 hours in a humidified atmosphere containing 5% carbon dioxide in air. The cultures are then centrifuged and an aliquot of the supernatant solubilized with Nonidet P40 and subjected to an antigen capture assay which uses a primary antiserum with particular reactivity against the viral protein 24 and a horseradish peroxidase detection system. Colour generation is measured spectrophotometrically and plotted against the concentration of test compound. The concentration that produces 50% protection is determined ($IC_{50}$).

The results obtained in the foregoing test using representative compounds of formula I as the test compound are compiled in the following Table.

Table

| Compound of formula I | $IC_{50}$ (nM) | | | |
|---|---|---|---|---|
| | Inhibition of HIV protease | | | Activity against HIV |
| | $S^1$ | $S^2$ | | |
| | | 1st run | 2nd run | |
| A | 12 | 4.5 | 0.62 | 100 |
| B | 20 | 3.4 | 0.48 | NT |
| C | 15 | 3.3 | NT | 7 |
| D | NT | 2.5 | 0.52 | 20 |
| E | NT | 4.3 | 2.20 | 70 |
| F | NT | 4.0 | 1.90 | 100 |
| G | 32 | 4.1 | 0.89 | 100 |
| H | 22 | 3.0 | 0.91 | 60 |
| I | 80 | 2.5 | 0.39 | 9 |
| J | NT | 7.7 | NT | 100 |
| K | NT | NT | 1.6 | 4 |
| L | NT | NT | 2.7 | 5 |
| M | NT | NT | 3.7 | 10 |
| N | NT | NT | 4.1 | <100 |
| NT = Not tested | | | | |

Compound A = $N^2$-[5(S)-(tert.Butoxyformamido)-6-cyclohexyl-4(S)-hydroxy-2(S)-isopropylhexanoyl]-$N^1$-phenethyl-L-isoleucinamide.

Compound B = N-[N-[5(S)-(tert.Butoxyformamido)-6-cyclohexyl-4(S)-hydroxy-2(S)-isopropylhexanoyl]-L-isoleucyl]-L-phenylalanine methyl ester.

Compound C = $N^2$-[5(S)-(tert.Butoxyformamido)-6-cyclohexyl-4(S)-hydroxy-2(S)-isopropylhexanoyl]-$N^1$-benzyl-L-isoleucinamide.

Compound D = $N^2$-[5(S)-(tert.Butoxyformamido)-6-cyclohexyl-4(S)-hydroxy-2(S)-isopropylhexanoyl]-$N^1$-[2-(2-pyridyl)ethyl]-L-isoleucinamide.

Compound E = $N^2$-[5(S)-(tert.Butoxyformamido)-6-cyclohexyl-4(S)-hydroxy-2(S)-isopropylhexanoyl]-$N^1$-(2-indanyl)-L-isoleucinamide.

Compound F = $N^2$-[5(S)-(tert.Butoxyformamido)-6-cyclohexyl-4(S)-hydroxy-2(S)-isopropylhexanoyl]-$N^1$-(2-morpholinoethyl)-L-isoleucinamide.

Compound G = $N^2$-[5(S)-(tert.Butoxyformamido)-4(S)-hydroxy-2(S)-isopropyl-6-phenylhexanoyl]-$N^1$-phenethyl-L-isoleucinamide.

Compound H = $N^2$-[5(S)-Benzyloxyformamido-6-cyclohexyl-4(S)-hydroxy-2(S)-isopropylhexanoyl]-$N^1$-phenethyl-L-isoleucinamide.

Compound I = $N^2$-[5(S)-[[N-(Benzyloxycarbonyl)-L-asparaginyl]amino]-6-cyclohexyl-4(S)-hydroxy-2-(S)-isopropylhexanoyl]-$N^1$-phenethyl-L-isoleucinamide.

Compound J = $N^2$-[5(S)-(tert.Butylacetamido)-6-cyclohexyl-4(S)-hydroxy-2(S)-isopropylhexanoyl]-$N^1$-phenethyl-L-isoleucinamide.

Compound K = $N^2$-[5(S)-[[N-(Benzyloxycarbonyl)-β-cyano-L-alanyl]amino]-6-cyclohexyl-4(S)-hydroxy-2(S)-isopropylhexanoyl]-$N^1$-phenethyl-L-isoleucinamide.

Compound L = $N^1$-[2-[4-(Benzyloxycarbonyl)-1-piperazinyl]ethyl]-$N^2$-[5(S)-(tert.butoxyformamido)-6-cyclohexyl-4(S)-hydroxy-2(S)-isopropylhexanoyl]-L-isoleucineamide.

Compound M = $N^2$-[5(S)-(tert.Butoxyformamido)-6-cyclohexyl-4(S)-hydroxy-2(S)-isopropylhexanoyl]-$N^1$-[2-(4-hydroxyphenyl)ethyl]-L-isoleucinamide.

Compound N = $N^1$-[2-[4-(Benzyloxycarbonyl)-1-piperazinyl]ethyl]-$N^2$-[5(S)-(tert.butoxyformamido)-6-cyclohexyl-3(R),4(R)-dihydroxy-2(R)-isopropylhexanoyl]-L-isoleucinamide.

The compounds of formula I and their pharmaceutically acceptable acid addition salts can be used as medicaments (e.g. in the form of pharmaceutical preparations). The pharmaceutical preparations can be administered enterally such as orally (e.g. in the form of tablets, coated tablets, dragees, hard and soft gelatine capsules, solutions, emulsions or suspensions), nasally (e.g. in the form of nasal sprays) or rectally (e.g. in the form of suppositories). However, the administration can also be effected parenterally such as intramuscularly or intravenously (e.g. in the form of injection solutions).

For the manufacture of tablets, coated tablets, dragees and hard gelatine capsules the compounds of formula I and their pharmaceutically acceptable acid addition salts can be processed with pharmaceutically inert, inorganic or organic excipients. Lactose, maize starch or derivatives thereof, talc, stearic acid or its salts etc can be used, for example, as such excipients for tablets, dragees and hard gelatine capsules. Suitable excipients for soft gelatine capsules are, for example, vegetable oils, waxes, fats, semi-solid and liquid polyols etc. Suitable excipients for the manufacture of solutions and syrups are, for example, water, polyols, saccharose, invert sugar, glucose etc. Suitable excipients for injection solutions are, for example, water, alcohols, polyols, glycerol, vegetable oils etc. Suitable excipients for suppositories are, for example, natural or hardened oils, waxes, fats, semi-liquid or liquid polyols etc.

Moreover, the pharmaceutical preparations can contain preserving agents, solubilizers, viscosity-increasing substances, stabilizing agents, wetting agents, emulsifying agents, sweetening agents, colouring agents, flavouring agents, salts for varying the osmotic pressure, buffers, coating agents or antioxidants. They can also contain still other therapeutically valuable substances.

In accordance with the invention the compounds of general formula I and their pharmaceutically acceptable acid addition salts can be used in the treatment or prophylaxis of viral infections, particularly of infections caused by HIV and other retroid viruses. The dosage can vary within wide limits and will, of course, be fitted to the individual requirements in each particular case. In general, in the case of oral administration there should suffice a daily dosage of about 3 mg to about 3 g, preferably about 10 mg to about 1 g, divided in preferably 1-3 unit doses, which can, for example, be of the same amount. It will, however, be appreciated that the upper limit given above can be exceeded when this is found to be indicated.

The following Examples illustrate the present invention:

## Example 1

A solution of 0.64 g of $N^2$-[3-[3-(tert.butoxycarbonyl)-4(S)-(cyclohexylmethyl)-2,2-dimethyl-5(S)-oxazolidinyl]-2(S)-isopropylpropionyl]-$N^1$-phenethyl-L-isoleucinamide and 11 mg of p-toluenesulphonic acid in 11 ml of methanol was stirred at room temperature overnight. The resulting suspension was evaporated and the residue was dissolved in 50 ml of dichloromethane. The solution was washed in succession with 10 ml of water, 10 ml of saturated sodium hydrogen carbonate solution and 10m ml of water and then dried over sodium sulphate. Evaporation of the solution yielded 0.6 g of product which was recrystallized from acetonitrile to give 0.42 g of analytically pure $N^2$-[5(S)-(tert.butoxyformamido)-6-cyclohexyl-4(S)-hydroxy-2-(S)-isopropylhexanoyl]-$N^1$-phenethyl-L-isoleucinamide as a white solid of melting point 193-196 °C.

The $N^2$-[3-[3-(tert.butoxycarbonyl)-4(S)-(cyclohexylmethyl)-2,2-dimethyl-5(S)-oxazolidinyl]-2(S)-isopropylpropionyl]-$N^1$-phenethyl-L-isoleucinamide used as the starting material was prepared as follows:

(i) A solution of 106 g of 4-brom-3-isopropyl-1-butene in 400 ml of anhydrous diethyl ether was added during 45 minutes to a suspension of 14.4 g of magnesium turnings in 120 ml of anhydrous diethyl ether under an argon atmosphere in such a manner that the mixture was maintained under gentle reflux. After completion of the addition the mixture was heated under reflux for a further 1 hour. The mixture was cooled to -60 °C and a solution of 51 g of N-tert.butoxycarbonyl-L-cyclohexylalaninal in 400 ml of anhydrous diethyl ether was added dropwise during 30 minutes. The mixture was stirred at -60 °C for 1 hour and then at room temperature overnight. The mixture was cooled to 5 °C in an ice bath and 150 ml of saturated ammonium

chloride solution were added slowly so that the temperature was maintained at below 20 °C. There were then added a further 800 ml of ammonium chloride solution followed by 1 l of diethyl ether. The phases were separated and the aqueous phase was extracted twice with 500 ml of diethyl ether each time. The combined ethereal phases were washed with 1 l of sodium chloride solution and 1 l of water and then dried over magnesium sulphate. Evaporation gave 81.7 g of a yellow oil which was mainly a mixture of two diastereoisomers. These were separated by repeated flash chromatography on silica gel using 5% ethyl acetate in dichloromethane for the elution. Evaporation of fractions containing the faster-eluting component gave 27.5 g of 6(S)-(tert.butoxyformamido)-7-cyclohexyl-4(S)-hydroxy-3(S)-isopropyl-1-heptene as a pale yellow syrup; $^1$H NMR (300 MHz): δ ($CDCl_3$) 0.87 (6H,dd), 0.9-1.9 (16H,m), 1.45 (9H,s), 2.04 (1H,m), 3.53 (2H,m), 4.60 (1H,d), 5.50 (2H,m) and 5.56 (1H,m) ppm. Evaporation of fractions containing the slower-eluting diastereoisomer gave 13.0 g of 6(S)-(tert.butoxyformamido)-7-cyclohexyl-4(S)-hydroxy-3(R)-isopropyl-1-heptene as a pale yellow syrup; $^1$H NMR (300 MHz): δ ($CDCl_3$) 0.85 (6H,dd), 0.9-1.9 (16H,m), 1.46 (9H,s), 2.0 (1H,m), 3.68 (2H,m), 4.65 (1H,d), 5.05 (2H,m) and 5.65 (1H,m) ppm.

(ii) A solution of 10 g of 6(S)-(tert.butoxyformamido)-7-cyclohexyl-4(S)-hydroxy-3(S)-isopropyl-1-heptene in 120 ml of 2,2-dimethoxypropane was treated with 0.5 g of p-toluenesulphonic acid. The solution was stirred at room temperature under an argon atmosphere for 24 hours and then poured into a mixture of ice and 120 ml of 2M sodium hydrogen carbonate solution. The mixture obtained was extracted once with 160 ml of diethyl ether and twice with 100 ml of diethyl ether. The combined extracts were washed with 100 ml of water and then dried over magnesium sulphate, filtered and evaporated to give 11.4 g of 3-(tert.butoxycarbonyl)-4(S)-(cyclohexylmethyl)-2,2-dimethyl-5(S)-[3(S)-isopropyl-1-butenyl]oxazolidine as a syrup which was used in the next step without further purification.

(iii) A solution of 11.4 g of the compound obtained according to paragraph (ii), 1.1 g of Aliquat 336 and 28.5 ml of glacial acetic acid in 263 ml of benzene was added slowly during 20 minutes to a stirred solution of 29.7 g of potassium permanganate in 263 ml of water while cooling in an ice bath. The mixture was stirred at room temperature for 20 hours, then cooled to 5 °C in an ice bath and treated with 36 g of sodium metabisulphite in portions so that the temperature did not rise above 10 °C. The mixture was stirred for 10 minutes and then treated with 40 ml of 10% citric acid solution. The mixture was transferred into a separating funnel and extracted twice with 200 ml of diethyl ether. The combined ethereal extracts were washed with 200 ml of sodium chloride solution and then dried over magnesium sulphate. Evaporation of the filtered solution gave 13.6 g of a syrup which was purified by flash chromatography on silica gel using 10% diethyl ether in dichloromethane for the elution. Fractions which contained pure product according to thin-layer chromatography were combined and evaporated to give 5.7 g of 3-(tert.butoxycarbonyl)-4(S)-(cyclohexylmethyl)-2,2-dimethyl-α(S)-isopropyl-5(S)-oxazolidinepropionic acid as a colourless gum; $^1$H NMR (250 MHz): δ ($CDCl_3$) 0.8-1.9 (28H,m), 1.50 (9H,s), 2.6 (1H,m), 3.65 (1H,b) and 3.89 (1H,m) ppm.

(iv) A solution of 0.82 g of 3-(tert.butoxycarbonyl)-4(S)-(cyclohexylmethyl)-2,2-dimethyl-α(S)-isopropyl-5-(S)-oxazolidinepropionic acid in 8 ml of dry dimethylformamide was cooled to -20 °C in an ice-salt bath. There were then added in sequence 0.51 g of N$^1$-phenethyl-L-isoleucinamide, 0.35 g of 1=hydroxybenzotriazole and 0.49 g of dicyclohexylcarbodiimide. The mixture was allowed to warm slowly to room temperature and was then stirred at this temperature overnight. The separated dicyclohexylurea was filtered off and the filtrate was evaporated. The residue was dissolved in 40 ml of dichloromethane and the solution was washed in succession with 10 ml of water, 10 ml of saturated sodium hydrogen carbonate solution and 10 ml of water. The solution was dried over magnesium sulphate, then filtered and evaporated. The residue was purified by flash chromatography on silica gel using 20% ethyl acetate in n-hexane for the elution. There was obtained 0.86 g of N$^2$-[3-[3-(tert.butoxycarbonyl)-4(S)-(cyclohexylmethyl)-2,2-dimethyl-5-(S)-oxazolidinyl]-2(S)-isopropylpropionyl]-N$^1$-phenethyl-L-isoleucinamide as a white solid; MS: m/e 628 $[M+H]^+$.

The N$^1$-phenethyl-L-isoleucinamide used in paragraph (iv) above was prepared as follows:

(a) A solution of 20 g of N-benzyloxycarbonyl-L-isoleucine in 400 ml of dry tetrahydrofuran was stirred and cooled at -10 °C while 10.2 g of N-ethylmorpholine were added followed after 2 minutes by 8.63 g of isobutyl chloroformate. The mixture was stirred at -10 °C for 3 minutes and then treated with 9.08 g of phenethylamine. After stirring at room temperature overnight the tetrahydrofuran was removed by evaporation under reduced pressure and the residue was partitioned between dichloromethane and water. The dichloromethane phase was washed in succession with 10% citric acid solution, water, saturated sodium hydrogen carbonate solution and water, then dried over sodium sulphate, filtered and evaporated to give a pale yellow solid residue. This residue was triturated with diethyl ether and filtered off to give 19.1 g of N$^2$-benzyloxycarbonyl-N$^1$-phenethyl-L-isoleucinamide as a white solid of melting point 161-164 °C.

(b) A solution of 19.1 g of N$^2$-benzyloxycarbonyl-N$^1$-phenethyl-L-isoleucinamide in 200 ml of ethanol was treated with 3 g of 10% palladium-on-carbon catalyst and the mixture was hydrogenated at room

temperature and under atmospheric pressure until the uptake of hydrogen had finished. The catalyst was removed by filtration and the filtrate was evaporated to give a colourless oil which was dissolved in ethyl acetate. Addition of n-hexane brought about crystallization of N[1]-phenethyl-L-isoleucinamide as a soft white solid (8.6 g) of melting point 69-74 °C.

## Example 2

In a manner analogous to that described in the first paragraph of Example 1, from 0.57 g of N-[N-[3-[3-(tert.butoxycarbonyl)-4(S)-(cyclohexylmethyl)-2,2-dimethyl-5(S)-oxazolindinyl]-2(S)-isopropylpropionyl]-L-isoleucyl]-L-phenylalanine methyl ester there was obtained 0.25 g of N-[N-[5(S)-(tert.butoxyformamido)-6-cyclohexyl-4(S)-hydroxy-2(S)-isopropylhexanoyl]-L-isoleucyl]-L-phenylalanine methyl ester of melting point 185 °C.

The N-[N-[3-[3-(tert.butoxycarbonyl)-4(S)-(cyclohexylmethyl)-2,2-dimethyl-5(S)-oxazolindinyl]-2(S)-isopropylpropionyl]-L-isoleucyl]-L-phenylalanine methyl ester used as the starting material was prepared as follows:

A solution of 0.64 g of 3-(tert.butoxycarbonyl)-4(S)-(cyclohexylmethyl)-2,2-dimethyl-α(S)-isopropyl-5(S)-oxazolidinepropionic acid in 2 ml of dimethylformamide was reacted with 0.46 g of L-isoleucyl-L-phenylalanine methyl ester in the presence of 0.23 g of 1-hydroxybenzotriazole and 0.39 g of dicyclohexylcarbodiimide according to the procedure described in Example 1 (iv) to give 0.57 g of N-[N-[3-[3-(tert.butoxycarbonyl)-4(S)-(cyclohexylmethyl)-2,2-dimethyl-5(S)-oxazolidinyl]-2(S)-isopropylpropionyl]-L-isoleucyl]-L-phenylalanine methyl ester as a white solid; MS: m/e 686 $[M+H]^+$.

## Example 3

In a manner analogous to that described in the first paragraph of Example 1, from 0.11 g of N-[N-[3-[3-(tert.butoxycarbonyl)-4(S)-(cyclohexylmethyl)-2,2-dimethyl-5(S)-oxazolidinyl]-2(R)-isopropylpropionyl]-L-isoleucyl]-L-phenylalanine methyl ester there was obtained 0.09 g of N-[N-[5(S)-(tert.butoxyformamido)-6-cyclohexyl-4(S)-hydroxy-2(R)-isopropylhexanoyl]-L-isoleucyl]-L-phenylalanine methyl ester. Recrystallization from toluene gave analytically pure product of melting point 192 °C.

The N-[N-[3-[3-(tert.butoxycarbonyl)-4(S)-(cyclohexylmethyl)-2,2-dimethyl-5(S)-oxazolidinyl]-2(R)-isopropylpropionyl]-L-isoleucyl]-L-phenylalanine methyl ester used as the starting material was prepared as follows:

In a manner analogous to that described in Example 1 (iv), from 0.14 g of 3-(tert.butoxycarbonyl)-4(S)-(cyclohexylmethyl)-2,2-dimethyl-α(R)-isopropyl-5(S)-oxazolidinepropionic acid there was obtained 0.11 g of N-[N-[3-[3-(tert.butoxycarbonyl)-4(S)-(cyclohexylmethyl)-2,2-dimethyl-5(S)-oxazolidinyl]-2(R)-isopropylpropionyl]-L-isoleucyl]-L-phenylalanine methyl ester; $^1H$ NMR (250 MHz): δ ($CDCl_3$) 0.8-2.3 (38H, m), 1.43 (9H, s), 3.09 (2H, d), 3.64 (1H, m), 3.70 (3H, s), 3.80 (1H, m), 4.30 (1H, m), 4.80 (1H, m), 6.20 (1H, d), 6.35 (1H, d) and 7.0-7.34 (5H, m) ppm.

## Example 4

In a manner analogous to that described in the first paragraph of Example 1, from 0.18 g of $N^2$-[3-[3-(tert.butoxycarbonyl)-4(S)-(cyclohexylmethyl)-2,2-dimethyl-5(S)-oxazolidinyl]-2(S)-isopropylpropionyl]- $N^1$-isobutyl-L-isoleucinamide there was obtained 0.126 g of $N^2$-[5(S)-(tert.butoxyformamido)-6-cyclohexyl-4(S)-hydroxy-2(S)-isopropylhexanoyl]-$N^1$-isobutyl-L-isoleucinamide. Recrystallization from acetonitrile gave analytically pure product of melting point 183-186 °C.

The $N^2$-[3-[3-(tert.butoxycarbonyl)-4(S)-(cyclohexylmethyl)-2,2-dimethyl-5(S)-oxazolidinyl]-2(S)-isopropylpropionyl]-$N^1$-isobutyl-L-isoleucinamide used as the starting material was prepared as follows:

A solution of 0.20 g of 3-(tert.butoxycarbonyl)-4(S)-(cyclohexylmethyl)-2,2-dimethyl-α(S)-isopropyl-5(S)-oxazolidinepropionic acid in 2 ml of dimethylformamide was reacted with 0.10 g of $N^1$-isobutyl-L-isoleucinamide in the presence of 0.088 g of 1-hydroxybenzotriazole and 0.124 g of dicyclohexylcarbodiimide according to the procedure described in Example 1 (iv) to give 0.19 g of $N^2$-[3-[3-

(tert.butoxycarbonyl)-4(S)-(cyclohexylmethyl)-2,2-dimethyl-5(S)-oxazolidinyl]-2(S)-isopropylpropionyl]-N¹-isobutyl-L-isoleucinamide; ¹H NMR (250 MHz): δ ($CDCl_3$) 0.76-2.4 (45H, m), 1.49 (9H, s), 3.04 (2H, m), 3.59 (1H, b), 3.71 (1H, d), 4.31 (1H, t), 6.40 (1H, d) and 6.58 (1H, b) ppm.

The N¹-isobutyl-L-isoleucinamide referred to in the preceding paragraph was prepared as follows:

(a) According to the procedure described in Example 1(a), from 10 g of N-benzyloxycarbonyl-L-isoleucine and 2.75 g of isobutylamine there were obtained 9.1 g of N²-benzyloxycarbonyl-N¹-isobutyl-L-isoleucinamide of melting point 152-154° C.

(b) According to the procedure described in Example 1(b), from 9.1 g of N²-benzyloxycarbonyl-N¹-isobutyl-L- isoleucinamide there were obtained 4.4 g of N¹-isobutyl-L-isoleucinamide as a gum which was used without purification.

## Example 5

In a manner analogous to that described in the first paragraph of Example 1, from 67 mg of N²-[3-[3-(tert.butoxycarbonyl)-4(S)-(cyclohexylmethyl)-2,2-dimethyl-5(S)-oxazolidinyl]-2(S)-isopropylpropionyl]-N¹-benzyl-L-isoleucinamide there were obtained 47 mg of N²-[5(S)-(tert.butoxyformamido)-6-cyclohexyl-4(S)-hydroxy-2(S)-isopropylhexanoyl]-N¹-benzyl-L-isoleucinamide. Analytically pure product was obtained by recrystallization from acetonitrile and melted at 194-195° C.

The N²-[3-[3-(tert.butoxycarbonyl)-4(S)-(cyclohexylmethyl)-2,2-dimethyl-5(S)-oxazolindinyl]-2(S)-isopropylpropionyl]-N¹-benzyl-L-isoleucinamide used as the starting material was prepared as follows:

A solution of 0.20 g of 3-(tert.butoxycarbonyl)-4(S)-(cyclohexylmethyl)-2,2-dimethyl-α(S)-isopropyl-5(S)-oxazolidinepropionic acid in 2 ml of dimethylformamide was reacted with 0.12 g of N¹-benzyl-L-isoleucinamide according to the procedure described in Example 1 (iv) to give 67 mg of N²-[3-[3-(tert.butoxycarbonyl)-4(S)-(cyclohexylmethyl)-2,2-dimethyl-5(S)-oxazolidinyl]-2(S)-isopropylpropionyl]-N¹-benzyl-L-isoleucinamide; ¹H NMR (250 MHz): δ ($CDCl_3$) 0.7-2.16 (38H, m), 1.48 (9H, s), 3.59 (1H, b), 3.71 (1H, d), 4.41 (2H, d), 4.46 (1H, t), 6.38 (1H, d), 7.0 (1H, b) and 7.24 (5H, m) ppm.

The N¹-benzyl-L-isoleucinamide referred to in the preceding paragraph was prepared as follows:

(a) According to the procedure described in Example 1(a), from 5.3 g of N-benzyloxycarbonyl-L-isoleucine and 2.14 g of benzylamine there were obtained 5.1 g of N¹-benzyl-N²-benzyloxycarbonyl-L-isoleucinamide of melting point 161-163° C.

(b) According to the procedure described in Example 1(b), from 3.0 g of N¹-benzyl-N²-benzyloxycarbonyl-L-isoleucinamide there were obtained 1.5 g of N¹-benzyl-L-isoleucinamide as an oil which was used without purification.

## Example 6

In a manner analogous to that described in the first paragraph of Example 1, from 0.19 g of N²-[3-[3-(tert.butoxycarbonyl)-4(S)-(cyclohexylmethyl)-2,2-dimethyl-5(S)-oxazolidinyl]-2(S)-isopropylpropionyl]-N¹-tert.butyl-L-isoleucinamide there was otbained 0.15 g of N²-[5(S)-(tert.butoxyformamido)-6-cyclohexyl-4-(S)-hydroxy-2(S)-isopropylhexanoyl]-N¹-tert.butyl-L-isoleucinamide. Recrystallization from acetonitrile gave analytically pure product of melting points 142-146° C.

The N²-[3-[3-(tert.butoxycarbonyl)-4(S)-(cyclohexylmethyl)-2,2-dimethyl-5(S)-oxazolidinyl]-2(S)-isopropylpropionyl]-N¹-tert.butyl-L-isoleucinamide used as the starting material was prepared as follows:

A solution of 0.20 g of 3-(tert.butoxycarbonyl)-4(S)-(cyclohexylmethyl)-2,2-dimethyl-α(S)-isopropyl-5(S)-oxazolidinepropionic acid in 2 ml of dimethylformamide was reacted with 0.09 g of N¹-tert.butyl-L-isoleucinamide according to the procedure described in Example 1 (iv) to give 0.19 g of N²-[3-[3-(tert.butoxycarbonyl)-4(S)-(cyclohexylmethyl)-2,2-dimethyl-5(S)-oxazolidinyl]- 2(S)-isopropylpropionyl]-N¹-tert.butyl-L-isoleucinamide; ¹H NMR (250 MHz): δ ($CDCl_3$) 0.78-2.25 (38H, m), 1.31 (9H, s), 1.48 (9H, s), 3.59 (1H, b), 3.70 (1H, d), 4.09 (1H, t), 5.88 (1H, s) and 6.26 (1H, d) ppm.

The N¹-tert.butyl-L-isoleucinamide referred to in the preceding paragraph was prepared as follows:

(a) According to the procedure described in Example 1(a), from 5.3 g of N-benzyloxycarbonyl-L-isoleucine and 1.46 g of tert.butylamine there were otbained 3.4 g of N-benzyloxycarbonyl-N¹-tert.butyl-L-isoleucinamide of melting point 80-86° C.

(b) According to the procedure described in Example 1(b), from 3.2 g of N²-benzyloxycarbonyl-N¹-tert.butyl-L-isoleucinamide there were otbained 1.8 g of N[1]-tert.butyl-L-isoleucinamide as an oil which was used without purification.

## Example 7

In a manner analogous to that described in the first paragraph of Example 1, from 0.14 g of N[2]-[3-[3-(tert.butoxycarbonyl)-4(S)-(cyclohexylmethyl)-2,2-dimethyl-5(S)-oxazolidinyl]-2(S)-isopropylpropionyl]-L-isoleucinamide there was otbained 0.12 g of N[2]-[5(S)-(tert.butoxyformamido)-6-cyclohexyl-4(S)-hydroxy-2-(S)-isopropylhexanoyl]-L-isoleucinamide as a white solid. Recrystallation form ethyl acetate gave analytically pure product of melting point 222° C.

The N[2]-[3-[3-(tert.butoxycarbonyl)-4(S)-(cyclohexylmethyl)-2,2-dimethyl-5(S)-oxazolidinyl]-2(S)-isopropylpropionyl]-L-isoleucinamide used as the starting material was prepared as follows:

A solution of 0.30 g of 3-(tert.butoxycarbonyl)-4(S)-(cyclohexylmethyl)-2,2-dimethyl-α(S)-isopropyl-5(S)-oxazolidinepropionic acid in 5 ml of dimethylformamide was reacted with 0.095 g of L-isoleucinamide according to the procedure described in Example 1 (iv) to give 0.14 g of N[2]-[3-[3-(tert.butoxycarbonyl)-4(S)-(cyclohexylmethyl)-2,2-dimethyl-5(S)-oxazolidinyl]-2(S)-isopropylpropionyl]-L-isoleucinamide; $^1H$ NMR (250 MHz): δ ($CDCl_3$ 0.6-2.1 (38H, m), 1.29 (9H, s), 3.29 (1H, b), 3.54 (1H, d), 4.12 (1H, d), 4.24 (1H, t), 5.67 (1H, s), 6.20 (1H, d) and 6.29 (1H, s) ppm.

## Example 8

In a manner analogous to that described in the first paragraph of Example 1, from 0.35 g of N[2]-[3-[3-(tert.butoxycarbonyl)-4(S)-(cyclohexylmethyl)-2,2-dimethyl-5(S)-oxazolidinyl]-2(S)-isopropylpropionyl]-N[1]-phenyl-L-isoleucinamide there was obtained 0.18 g of N[2]-[5(S)-(tert.butoxyformamido)-6-cyclohexyl-4(S)-hydroxy-2(S)-isopropylhexanoyl]-N[1]-phenyl-L-isoleucinamide. Recrystallization from ethyl acetate gave analytically pure product of melting point 188° C.

The N[2]-[3-[3-(tert.butoxycarbonyl)-4(S)-(cyclohexylmethyl)-2,2-dimethyl-5(S)-oxazolidinyl]-2(S)-isopropylpropionyl]-N[1]-phenyl-L-isoleucinamide used as the starting material was prepared as follows:

A solution of 0.41 g of 3-(tert.butoxycarbonyl)-4(S)-(cyclohexylmethyl)-2,2-dimethyl-α(S)-isopropyl-5(S)-oxazolidinepropionic acid in 5 ml of dimethylformamide was reacted with 0.21 g of N[1]-phenyl-L-isoleucinamide according to the procedure described in Example 1 (iv) to give 0.35 g of N[2]-[3-[3-(tert.butoxycarbonyl)-4(S)-(cyclohexylmethyl)-2,2-dimethyl-5(S)-oxazolidinyl]-2(S)- isopropylpropionyl]-N[1]-phenyl-L-isoleucinamide; MS: m/e 600 $[M+H]^+$.

The N[1]-phenyl-L-isoleucinamide referred to in the preceding paragraph was prepared as follows:

(a) According to the procedure described in Example 1(a), from 4.4 g of N-benzyloxycarbonyl-L-isoleucine and 1.54 g of aniline there were obtained 3.2 g of N[2]-benzyloxycarbonyl-N[1]-phenyl-L-isoleucinamide as a white solid; MS: m/e 341 $[M+H]^+$.

(b) According to the procedure described in Example 1(b), from 0.34 g of N[2]-benzyloxycarbonyl-N[1]-phenyl-L-isoleucinamide there was obtained 0.20 g of N[1]-phenyl-L-isoleucinamide which was used without purification.

## Example 9

In a manner analogous to that described in the first paragraph of Example 1, from 1.01 g of N[2]-[3-[3-(tert.butoxycarbonyl)-4(S)-(cyclohexylmethyl)-2,2-dimethyl-5(S)-oxazolidinyl]-2(S)-isopropylpropionyl]-N[1]-[2-(2-pyridyl)ethyl]-L-isoleucinamide there was obtained 0.68 g of N[2]-[5(S)-(tert.butoxyformamido)-6-cycl ohexyl-4(S)-hydroxy-2(S)-isopropylhexanoyl]-N[1]-[2-(2-pyridyl)ethyl]-L-isoleucinamide. Analytically pure material was obtained by crystallization from acetonitrile and melted at 172-174° C.

The N[2]-[3-[3-(tert.butoxycarbonyl)-4(S)-(cyclohexylmethyl)-2,2-dimethyl-5(S)-oxazolidinyl]-2(S)-isopropylpropionyl]-N[1]-[2-(2-pyridyl)ethyl]-L-isoleucinamide used as the starting material was prepared as follows:

A solution of 0.9 g of 3-(tert.butoxycarbonyl)-4(S)-(cyclohexylmethyl)-2,2-dimethyl-α(S)-isopropyl-5(S)-oxazolidinepropionic acid in 9 ml of dimethylformamide was reacted with 0.57 g of $N^1$-[2-(2-pyridyl)ethyl]-L-isoleucinamide according to the procedure described in Example 1 (iv) to give 1.01 g of $N^2$-[3-[3-(tert.butoxycarbonyl)-4(S)-(cyclohexylmethyl)-2,2-dimethyl-5(S)-oxazolidinyl]-2(S)-isopropylpropionyl]-$N^1$-[2-(2-pyridyl)ethyl]-L-isoleucinamide: MS: m/e 629 $[M+H]^+$.

The $N^1$-[2-(2-pyridyl)ethyl]-L-isoleucinamide referred to in the preceding paragraph was prepared as follows:

(a) A solution of 1.0 g of N-benzyloxycarbonyl-L-isoleucine succinimide ester in 20 ml of dry tetrahydrofuran was treated dropwise with 0.34 g of 2-(2-aminoethyl)pyridine and the mixture was stirred at room temperature overnight. The solution was evaporated and the residue was partitioned between 50 ml of dichloromethane and 50 ml of water. The dichloromethane phase was washed with 25 ml of saturated sodium hydrogen carbonate solution and 25 ml of water, then dried over sodium sulphate, filtered and evaporated to give 1.0 g of $N^2$-benzyloxycarbonyl-$N^1$-[2-(2-pyridyl)ethyl]-L-isoleucinamide as a white solid. Recrystallization from toluene gave colourless needles of melting point 156-159 °C.

(b) A suspension of 0.8 g of $N^2$-benzyloxycarbonyl-$N^1$-[2-(2-pyridyl)ethyl]-L-isoleucinamide in 15 ml of ethanol was treated with 40 mg of 10% palladium-on-carbon catalyst and the mixture was hydrogenated at room temperature and under atmospheric pressure until the uptake of hydrogen had finished. The catalyst was removed by filtration and the filtrate was evaporated to give 0.51 g of $N^1$-[2-(2-pyridyl)ethyl]-L-isoleucinamide as a solid of melting point 41-46 °C.

## Example 10

In a manner analogous to that described in the first paragraph of Example 1, from 0.24 g of $N^2$-[3-[3-(tert.butoxycarbonyl)-4(S)-(cyclohexylmethyl)-2,2-dimethyl-5(S)-oxazolidinyl]-2(S)-isopropylpropionyl]-$N^1$-(2-indanyl)-L-isoleucinamide there was obtained 0.20 g of $N^2$-[5(S)-(tert.butoxyformamido)-6-cyclohexyl-4(S)-hydroxy-2(S)-isopropylhexanoyl]-$N^1$-(2-indanyl)-L-isoleucinamide. Analytically pure product was obtained by recrystallization from acetonitrile and melted at 219-221 °C.

The $N^2$-[3-[3-(tert.butoxycarbonyl)-4(S)-(cyclohexylmethyl)-2,2-dimethyl-5(S)-oxazolidinyl]-2(S)-isopropylpropionyl]-$N^1$-(2-indanyl)-L-isoleucinamide used as the starting material was prepared as follows:

A solution of 0.2 g of 3-(tert.butoxycarbonyl)-4(S)-(cyclohexylmethyl)-2,2-dimethyl-α(S)-isopropyl-5(S)-oxazolidinepropionic acid in 2 ml of dimethylformamide was reacted with 0.135 g of $N^1$-(2-indanyl)-L-isoleucinamide according to the procedure described in Example 1 to give 0.25 g of $N^2$-[3-[3-tert.butoxycarbonyl)-4(S)-(cyclohexylmethyl)-2,2-dimethyl-5(S)-oxazolidinyl]-2(S)-isopropylpropionyl]-$N^1$-(2-indanyl)-L-isoleucinamide: $^1$H NMR (250 MHz): δ ($CDCl_3$) 0.8-1.0 (13H, m), 1.05-2.15 (25H, m), 1.49 (9H, s), 2.80 (2H, dt), 3.31 (2H, dt), 3.6 (1H, b), 3.71 (1H, d), 4.20 (1H, t), 4.73 (1H, m), 6.17 (1H, d), 6.26 (1H, d) and 7.19 (4H, m) ppm.

The $N^1$-(2-indanyl)-L-isoleucinamide referred to in the preceding paragraph was prepared as follows:

(a) A mixture of 1.0 g of N-benzyloxycarbonyl-L-isoleucine succinimide ester and 0.47 g of 2-aminoindan hydrochloride in 20 ml of dry tetrahydrofuran was treated with 0.35 g of N-ethylmorpholine and the resulting mixture was stirred and heated at 60 °C for 10 hours. Tetrahydrofuran was removed by evaporation under reduced pressure and the residue was partitioned between 50 ml of dichloromethane and 50 ml of water. The dichloromethane phase was washed in succession with 20 ml of 2M hydrochloric acid, 10 ml of water and 10 ml of saturated sodium hydrogen carbonate solution, then dried over sodium sulphate, filtered and evaporated to give 1.04 g of $N^2$-benzyloxycarbonyl-$N^1$-(2-indanyl)-L-isoleucinamide which was recrystallized from ethyl acetate and then had a melting point of 173-180 °C.

(b) A suspension of 0.6 g of $N^2$-benzyloxycarbonyl-$N^1$-(2-indanyl)-L-isoleucinamide in 11 ml of ethanol was treated with 30 mg of 10% palladium-on-carbon catalyst and the mixture was hydrogenated at room temperature and under atmospheric pressure until the uptake of hydrogen had finished. The catalyst was removed by filtration and the fitrate was evaporated to give 0.42 g of $N^1$-(2-indanyl)-L-isoleucinamide as a solid of melting point 72-76 °C.

## Example 11

In a manner analogous to that described in the first paragraph of Example 1, from 0.25 g of $N^2$-[3-[3-(tert.butoxycarbonyl)-4(S)-(cyclohexylmethyl)-2,2-dimethyl-5(S)-oxazolidinyl]-2(S)-isopropylpropionyl]-$N^1$-(2-

morpholinoethyl)-L-isoleucinamide there was obtained 0.20 g of $N^2$-[5(S)-(tert.butoxyformamido)-6-cyclohexyl-4(S)-hydroxy-2(S)-isopropylhexanoyl]-$N^1$-(2-morpholinoethyl)-L-isoleucinamide. Analytically pure product was obtained by recrystallization from acetonitrile and melted at 173° C.

The $N^2$-[3-[3-(tert.butoxycarbonyl)-4(S)-(cyclohexylmethyl)-2,2-dimethyl-5(S)-oxazolidinyl]-2(S)-isopropylpropionyl]-$N^1$-(2-morpholinoethyl)-L-isoleucinamide used as the starting material was prepared as follows:

A solution of 0.2 g of 3-(tert.butoxycarbonyl)-4(S)-(cyclohexylmethyl)-2,2-dimethyl-α(S)-isopropyl-5(S)-oxazolidinepropionic acid in 3 ml of dimethylformamide was reacted with 0.12 g of $N^1$-(2-morpholinoethyl)-L-isoleucinamide according to the procedure described in Example 1 (iv) to give 0.25 g of $N^2$-[3-[3-(tert.butoxycarbonyl)-4(S)-(cyclohexylmethyl)-2,2-dimethyl-5(S)-oxazolidinyl]-2(S)-isopropylpropionyl]-$N^1$-(2-morpholinoethyl)-L-isoleucinamide; $^1$H NMR (250 MHz): δ ($CDCl_3$) 0.68-1.9 (37H, m), 1.40 (9H, s), 2.01 (1H, m), 2.33 (6H, m), 3.25 (3H, m), 3.59 (6H, m), 4.19 (1H, t), 6.20 (1H, d) and 6.34 (1H, t) ppm.

The $N^1$-(2-morpholinoethyl)-L-isoleucinamide referred to in the preceding paragraph was prepared as follows:

(a) A solution of 1.0 g of N-benzyloxycarbonyl-L-isoleucine succinimide ester in 20 ml of dry tetrahydrofuran was reacted with 0.39 g of 4-(2-aminoethyl)morpholine according to the procedure described in Example 9(a) to give 0.82 g of $N^2$-benzyloxycarbonyl-$N^1$-(2-morpholinoethyl)-L-isoleucinamide as a white solid; $^1$H NMR (250 MHz): δ ($CDCl_3$) 0.96 (6H, m), 1.18 (1H, m), 1.50 (1H, m), 1.90 (1H, m), 2.62 (6H, bs), 3.45 (2H, q), 3.30 (4H, bs), 4.04 (1H, q), 5.12 (2H, s), 5.48 (1H, d), 6.79 (1H, bs) and 7.39 (5H, m) ppm.

(b) According to the procedure described in Example 9(b), from 0.8 g of $N^2$-benzyloxycarbonyl-$N^1$-(2-morpholinoethyl)-L-isoleucinamide there was obtained 0.49 g of $N^1$-(2-morpholinoethyl)-L-isoleucinamide in the form of a gum which was used without purification.

## Example 12

In a manner analogous to that described in the first paragraph of Example 1, from 0.24 g of N-[N-[3-[3-(tert.butoxycarbonyl)-4(S)-(cyclohexylmethyl)-2,2-dimethyl-5(S)-oxazolidinyl]-2(S)-isopropylpropionyl]-L-isoleucyl]-1,2,3,4-tetrahydroisoquinoline there was obtained 0.18 g of 2-[N-[5(S)-(tert.butoxyformamido)-6-cyclohexyl-4(S)-hydroxy-2(S)-isopropylhexanoyl]-L-isoleucyl]-1,2,3,4-tetrahydroisoquinoline. Analytically pure material was obtained as a white foam after flash chromatography on silica gel using 5% methanol in dichloromethane for the elution and melted at 75-78° C.

The N-[N-[3-[3-(tert.butoxycarbonyl)-4(S)-(cyclohexylmethyl)-2,2-dimethyl-5(S)-oxazolidinyl]-2(S)-isopropylpropionyl]-L-isoleucyl]-1,2,3,4-tetrahydroisoquinoline used as the starting material was prepared as follows:

A solution of 0.20 g of 3-(tert.butoxycarbonyl)-4(S)-(cyclohexylmethyl)-2,2-dimethyl-α(S)-isopropyl-5(S)-oxazolidinepropionic acid in 2 ml of dimethylformamide was reacted with 0.135 g of 2-(L-isoleucyl)-1,2,3,4-tetrahydroisoquinoline according to the procedure described in Example 1 (iv) to give 0.25 g of N-[N-[3-[3-(tert.butoxycarbonyl)-4(S)-(cyclohexylmethyl)-2,2-dimethyl-5(S)-oxazolidinyl]-2(S)-isopropylpropionyl]-L-isoleucyl]-1,2,3,4-tetrahydroisoquinoline; $^1$H NMR (250 MHz): δ ($CDCl_3$) 0.72-1.94 (37H, m), 1.50 (9H, s), 2.12 (1H, m), 2.87 (1H, t), 2.94) (1H, dt), 3.62 (1H, b), 3.79 (1H, d), 3.86 (2H, m), 4.74 (2H, m), 5.0 (1H, dt), 6.39 (1H, d) and 7.18 (4H, m) ppm.

The 2-(L-isoleucyl)-1,2,3,4-tetrahydroisoquinoline referred to in the preceding paragraph was prepared as follows:

(a) A solution of 1.0 g of N-benzyloxycarbonyl-L-isoleucine succinimide ester in 20 ml of dry tetrahydrofuran was reacted with 0.37 g of 1,2,3,4-tetrahydroisoquinoline according to the procedure described in Example 9(a) to give 0.54 g of 2-(N-benzyloxycarbonyl-L-isoleucyl)-1,2,3,4-tetrahydroisoquinoline as a colourless syrup; $^1$H NMR (250 MHz): δ ($CDCl_3$) 0.90 (6H, m), 1.11 (1H, m), 1.52 (1H, m), 1.76 (1H, m), 2.88 (2H, m), 3.79 (2H, m), 4.64 (1H, q), 4.72 (2H, q), 5.1 (2H, s), 5.61 (1H, d), 7.19 (4H, m) and 7.31 (5H, m) ppm.

(b) According to the procedure described in Example 9(b), from 0.47 g of 2-(N-benzyloxycarbonyl-L-isoleucyl)-1,2,3,4-tetrahydroisoquinoline there was obtained 0.30 g of 2-(L-isoleucyl)-1,2,3,4-tetrahydroisoquinoline which was used without purification.

## Example 13

In a manner analogous to that described in the first paragraph of Example 1, from 0.18 g of $N^2$-[3-[3-(tert.butoxycarbonyl)-4(S)-(cyclohexylmethyl)-2,2-dimethyl-5(S)-oxazolidinyl]-2(S)-isopropylpropionyl]-$N^1$-[2-(dimethylamino)ethyl]-L-isoleucinamide there was obtained 0.14 g of $N^2$-[5(S)-(tert.butoxyformamido)-6-cyclohexyl-4(S)-hydroxy-2(S)-isopropylhexanoyl]-$N^1$-[2-(dimethylamino)ethyl]-L-isoleucinamide. Recrystallization from acetonitrile gave analytically pure product of melting point 152-155° C.

The $N^2$-[3-[3-(tert.butoxycarbonyl)-4(S)-(cyclohexylmethyl)-2,2-dimethyl-5(S)-oxazolidinyl]-2(S)-isopropylpropionyl]-$N^1$-[2-(dimethylamino)ethyl]-L-isoleucinamide used as the starting material was prepared as follows:

A solution of 0.20 g of 3-(tert.butoxycarbonyl)-4(S)-(cyclohexylmethyl)-2,2-dimethyl-α(S)-isopropyl-5(S)-oxazolidinepropionic acid in 2 ml of dimethylformamide was reacted with 0.10g of $N^1$-[2-(dimethylamino)-ethyl]-L-isoleucinamide according to the procedure described in Example 1 (iv) to give 0.18 g of $N^2$-[3-[3-(tert.butoxycarbonyl)-4(S)-(cyclohexylmethyl)-2,2-dimethyl-5(S)-oxazolidinyl]-2(S)-isopropylpropionyl]-$N^1$-[2-(dimethylamino)ethyl]-L-isoleucinamide; MS: m/e 595 $[M+H]^+$.

The $N^1$-[2-(dimethylamino)ethyl]-L-isoleucinamide referred to in the preceding paragraph was prepared as follows:

(a) A solution of 0.5 g of N-benzyloxycarbonyl-L-isoleucine succinimide ester in 10 ml of dry tetrahydrofuran was reacted with 0.13 g of N,N-dimethylethylenediamine according to the procedure described in Example 9(a) to give 0.36 g of $N^2$-benzyloxycarbonyl-$N^1$-[2-(dimethylamino)ethyl]-L-isoleucinamide; $^1H$ NMR (250 MHz): δ ($CDCl_3$) 0.94 (6H, m), 1.15 (1H, m), 1.50 (1H, m), 1.9 (1H, m), 2.30 (6H, s), 2.47 (2H, t), 3.28 (1H, bs), 3.37 (2H, q), 4.03 (1H, q), 5.10 (2H, s), 5.56 (1H, d), 6.70 (1H, bs) and 7.35 (5H, m) ppm.

(b) According to the procedure described in Example 9(b), from 0.36 g of $N^2$-benzyloxycarbonyl-$N^1$-[2-(dimethylamino)ethyl]-L-isoleucinamide there was obtained 0.19 g of $N^1$-[2-(dimethylamino)ethyl]-L-isoleucinamide as a gum which was used without purification.

## Example 14

In a manner analogous to that described in the first paragraph of Example 1, from 0.21 g of $N^2$-[3-[3-(tert.butoxycarbonyl)-4(S)-(cyclohexylmethyl)-2,2-dimethyl-5(S)-oxazolidinyl]-2(S)-isopropylpropionyl]-$N^1$-benzyl-L-leucinamide there was obtained 0.15 g of $N^2$-[5(S)-(tert.butoxyformamido)-6-cyclohexyl-4(S)-hydroxy-2(S)-isopropylhexanoyl]-$N^1$-benzyl-L-leucinamide. Analytically pure product was obtained by recrystallization from acetonitrile and melted at 161-166° C.

The $N^2$-[3-[3-(tert.butoxycarbonyl)-4(S)-(cyclohexylmethyl)-2,2-dimethyl-5(S)-oxazolidinyl]-2(S)-isopropylpropionyl]-$N^1$-benzyl-L-leucinamide used as the starting material was prepared as follows:

A solution of 0.20 g of 3-(tert.butoxycarbonyl)-4(S)-(cyclohexylmethyl)-2,2-dimethyl-α(S)-isopropyl-5(S)-oxazolidinepropionic acid in 2 ml of dimethylformamide was reacted with 0.12 g of $N^1$-benzyl-L-leucinamide acording to the procedure described in Example 1(iv) to give 0.22 g of $N^2$-[3-[3-(tert.butoxycarbonyl)-4(S)-(cyclohexylmethyl)-2,2-dimethyl-5(S)-oxazolidinyl]-2(S)-isopropylpropionyl]-$N^1$-benzyl-L-leucinamide; MS: m/e 614 $[M+H]^+$.

The $N^1$-benzyl-L-leucinamide referred to in the preceding paragraph was prepared as follows:

(a) According to the procedure described in Example 1(A), from 5.3 g of N-benzyloxycarbonyl-L-leucine there were obtained 4.3 g of $N^1$-benzyl-$N^2$-benzyloxycarbonyl-L-leucinamide of melting point 110-112° C.

(b) According to the procedure described in Example 1(b), from 2.0 g of N-benzyloxycarbonyl-L-leucine there were obtained 1.2 g of $N^1$-benzyl-L-leucinamide of melting point 44-46° C.

## Example 15

In a manner analogous to that described in the first paragraph of Example 1, from 0.12 g of $N^2$-[3-[3-(tert.butoxycarbonyl)-4(S)-(cyclohexylmethyl)-2,2-dimethyl-5(S)-oxazolidinyl]propionyl]-$N^1$-phenethyl-L-isoleucinamide there were obtained 55 mg of material which was a mixture of two diastereoisomers in the ratio of 7:3. Recrystallization from ethyl acetate/n-hexane gave analytically pure $N^2$-[5(S)-(tert.butoxyformamido)-6-cyclohexyl-4(S)-hydroxyhexanoyl]-$N^1$-phenethyl-L-isoleucinamide of melting point

146° C.

The $N^2$-[3-[3-(tert.butoxycarbonyl)-4(S)-(cyclohexylmethyl)-2,2-dimethyl-5(S)-oxazolidinyl]propionyl-$N^1$-phenethyl-L-isoleucinamide used as the starting material was prepared as follows:

(i) 3-(Tert.butoxycarbonyl)-4(S)-(cyclohexylmethyl)-2,2-dimethyl-5(R,S)-oxazolidinepropionic acid was prepared from N-(tert.butoxycarbonyl)-L-cyclohexylalaninal and 4-bromo-1-butene in a manner analogous to that described in Example 1 (i)-(iii). The acid was obtained in the form of a gum; $^1$H NMR: δ ($CDCl_3$) 0.8-2.0 (21H,m), 1.5 (9H,s), 2.51 (2H,m), 3.7 (1H,b) and 3.9 (1H,dt) ppm.

(ii) A solution of 0.2 g of 3-(tert.butoxycarbonyl)-4(S)-(cyclohexylmethyl)-2,2-dimethyl-5(R,S)-oxazolidinepropionic acid in 2 ml of dimethylformamide was reacted with $N^1$-phenethyl-L-isoleucinamide according to the procedure described in Example 1 (iv) to give 0.12 g of $N^2$-[3-[3-(tert.butoxycarbonyl)-4(S)-(cyclohexylmethyl)-2,2-dimethyl-5(S)-oxazolidinyl]propionyl]-$N^1$-phenethyl-L-isoleucinamide; MS: m/e 586 $[M+H]^+$.

## Example 16

In a manner analogous to that described in the first paragraph of Example 1, from 1.02 g of $N^2$-[3-[3-(tert.butoxycarbonyl)-4(S)-(cyclohexylmethyl)-2,2-dimethyl-5(S)-oxazolidinyl]-2(R,S)-methylpropionyl]-$N^1$-phenethyl-L-isoleucinamide there was obtained, after chromatography, 0.61 g of $N^2$-[5(S)-(tert.butoxyformamido)-6-cyclohexyl-4(S)-hydroxy-2(R,S)-methylhexanoyl]-$N^1$-phenethyl-L-isoleucinamide of melting point 139-141° C.

The $N^2$-[3-[3-(tert.butoxycarbonyl)-4(S)-(cyclohexylmethyl)-2,2-dimethyl-5(S)-oxazolidinyl]-2(R,S)-methylpropionyl]-$N^1$-phenethyl-L-isoleucinamide used as the starting material was prepared as follows:

(i) 3-(Tert.butoxycarbonyl)-4(S)-(cyclohexylmethyl)-2,2-dimethyl-α(R,S)-methyl-5(S)-oxazolidinepropionic acid was prepared from N-(tert.butoxycarbonyl)-L-cyclohexylalaninal and 4-brom-3-methyl-1-butene according to the procedure described in Example 1 (i)-(iii) the acid was obtained in the form of a gum as a mixture of diastereoisomers which could not be separated; $^1$H NMR (250 MHz): δ ($CDCl_3$) 0.7-1.9 (25H, m), 1.40 and 1.49 (9H, ds) and 3.9-4.2 (2H, bm) ppm.

(ii) A solution of 1.7 g of 3-(tert.butoxycarbonyl)-4(S)-(cyclohexylmethyl)-2,2-dimethyl-α(R,S)-methyl-5-(S)-oxazolidinepropionic acid in 15 ml of dimethylformamide was reacted with 1.04 g of $N^1$-phenethyl-L-isoleucinamide according to the procedure described in Example 1 (iv) to give 1.02 g of $N^2$-[3-[3-(tert.butoxycarbonyl)-4(S)-(cyclohexylmethyl)-2,2-dimethyl-5(S)-oxazolidinyl]-2(R,S)-methylpropionyl]-$N^1$-phenethyl-L-isoleucinamide; MS: m/e 600 $[M+H]^+$.

## Example 17

In a manner analogous to that described in the first paragraph of Example 1, from 0.19 g of $N^2$-3-[[4(S)-benzyl-3-(tert.butoxycarbonyl)-2,2-dimethyl-5(S)-oxazolidinyl]-2(S)-isopropylpropionyl]-$N^1$-phenethyl-L-isoleucinamide there were obtained 88 mg of $N^2$-[5(S)-(tert.butoxyformamido)-4(S)-hydroxy-2(S)-isopropyl-6-phenylhexanoyl]-$N^1$-phenethyl-L-isoleucinamide. Analytically pure product was obtained by recrystallization from ethanol and melted at 207-208° C.

The$N^2$-[3-[4(S)-benzyl-3-(tert.butoxycarbonyl)-2,2-dimethyl-5(S)-oxazolidinyl]-2(S)-isopropylpropionyl]-$N^1$-phenethyl-L-isoleucinamide used as the starting material was prepared as follows:

(i) 4(S)-Benzyl-3-(tert.butoxycarbonyl)-2,2-dimethyl-α(S)-isopropyl-5(S)-oxazolidinepropionic acid was obtained in the form of a gum, MS: m/e 406 $[M+H]^+$, from N-(tert.butoxycarbonyl)-L-phenylalaninal and 4-bromo-3-isopropyl-1-butene according to the procedure described in Example 1 (i)-(iii).

(ii) A solution of 0.23 g of 4(S)-benzyl-3-(tert.butoxycarbonyl)-2,2-dimethyl-α(S)-isopropyl-5(S)-oxazolidinepropionic acid in 2,5 ml of dimethylformamide was reacted with 0.14 g of $N^1$-phenethyl-L-isoleucinamide according to the procedure described in Example 1 (iv) to give 0.20 g of $N^2$-[3-[4(S)-benzyl-3-(tert.butoxycarbonyl)-2,2-dimethyl-5(S)-oxazolidinyl]-2(S)-isopropylpropionyl]-$N^1$-phenethyl-L-isoleucinamide; MS: m/e 622 $[M+H]^+$.

## Example 18

A solution of 0.15 g of N-[N-[3-[3-(tert.butoxycarbonyl)-4(S)-(cyclohexylmethyl)-2,2-dimethyl-5(S)-oxazolidinyl]-2(S)-isopropylpropionyl]-L-isoleucyl]-L-phenylalanine methyl ester in 0.6 ml of methanol was treated with 0.3 ml of a 3M solution of hydrogen chloride in methanol. The solution was stirred at room temperature for 5 hours and then evaporated to dryness. The residue was suspended in 0.5 ml of ethyl acetate and the suspension was stirred and cooled in an ice bath while a solution of 79 mg of sodium hydrogen carbonate in 1 ml of water was added followed dropwise by a solution of 39 mg of benzyl chloroformate in 0.5 ml of ethyl acetate. The mixture was stirred at room temperature overnight. After separation of the phases the ethyl acetate phase was dried over sodium sulphate, filtered and evaporated. The crude product was purified by flash chromatography on silica gel using 1% methanol in dichloromethane for the elution. There were obtained 65 mg of N-[N-[5(S)-(benzyloxyformamido)-6-cyclohexyl-4-(S)-hydroxy-2(S)-isopropylhexanoyl]-L-isoleucyl]-L-phenylalanine methyl ester. Analytically pure product was obtained by recrystallization from ethyl acetate and melted at 205° C.

## Example 19

A solution of 60 mg of N-[N-[3-[3-(tert.butoxycarbonyl)-4(S)-(cyclohexylmethyl)-2,2-dimethyl-5(S)-oxazolidinyl]-2(S)-isopropylpropionyl]-L-isoleucyl]-L-phenylalanine methyl ester in 1 ml of a 1M solution of hydrogen chloride in methanol was stirred at room temperature for 5 hours and then evaporated to dryness. The residue was dissolved in 1 ml of dry dimethylformamide and the solution was stirred and cooled in an ice bath while 11 mg of N-ethylmorpholine were added followed by 48 mg of the succinimide ester of N-benzyloxycarbonyl-L-asparagine. The mixture was allowed to warm to room temperature and was then stirred overnight. Dimethylformamide was removed by evaporation and the residue was partitioned between dichloromethane and water. The dichloromethane phase was separated, dried over sodium sulphate, filtered and evaporated. The residue was triturated with ethyl acetate and filtered to give 45 mg of N-[N-[5(S)-[[N-(benzyloxycarbonyl)-L-asparaginyl]amino]-6-cyclohexyl-4(S)-hydroxy-2(S)-isopropylhexanoyl]-L-isoleucyl]-L-phenylalanine methyl ester as a white solid of melting point 246° C.

## Example 20

A solution of 0.15 g of N-[N-[3-[3-(tert.butoxycarbonyl)-4(S)-(cyclohexylmethyl)-2,2-dimethyl-5(S)-oxazolidinyl]-2(S)-isopropylpropionyl]-L-isoleucyl]-L-phenylalanine methyl ester in 0.6 ml of methanol was treated with 0.3 ml of a 3M solution of hydrogen chloride in methanol. The solution was stirred at room temperature for 5 hours and then evaporated to dryness. The residue was suspended in 0.5 ml of ethyl acetate and the suspension was stirred and cooled in an ice/salt bath while a solution of 79 mg of sodium hydrogen carbonate in 1 ml of water was added followed by 23 mg of acetic anhydride. The mixture was stirred at room temperature overnight, 10 ml of water and 15 ml of ethyl acetate were then added and the phases were separated. The ethyl acetate phase was dried over sodium sulphate, filtered and evaporated. The residue was purified by flash chromatography on silica gel using 2% methanol in dichloromethane for the elution. There were obtained 18 mg of N-[N-(5(S)-acetamido-6-cyclohexyl-4(S)-hydroxy-2(S)-isopropylhexanoyl]-L-isoleucyl]-L-phenylalanine methyl ester; MS: m/e 588 $[M+H]^+$.

## Example 21

0.12 g of $N^2$-[5(S)-benzyloxyformamido-4(S)-(tert.butyldimethylsilyloxy)-6-cyclohexyl-2(S)-isopropyl hexanoyl]-$N^1$-phenethyl-L-isoleucinamide was dissolved in 1.2 ml of triethylamine trihydrofluoride and the mixture was stirred at room temperature overnight. A thick white precipitate separated. The mixture was diluted with 12 ml of dichloromethane and 12 ml of saturated sodium hydrogen carbonate solution. The phases were separated and the dichloromethane phase was washed in succession with in each case 5 ml of saturated sodium hydrogen carbonate solution, water, 10% citric acid solution, water, saturated sodium hydrogen carbonate solution and water, then dried over sodium sulphate, filtered and evaporated to give 0.10 g of $N^2$-[5(S)-benzyloxyformamido-6-cyclohexyl-4(S)-hydroxy-2(S)-isopropylhexanoyl]-$N^1$-phenethyl-L-isoleucinamide as a white solid. Analytically pure product was obtained by recrystallization from acetonitrile

and melted at 218-219° C.

The $N^2$-[5(S)-benzyloxyformamido-4(S)-(tert.butyldimethylsilyloxy)-6-cyclohexyl-2(S)-isopropylhexanoyl]-$N^1$-phenethyl-L-isoleucinamide used as the starting material was prepared as follows:

(i) A solution of 0.15 g of $N^2$-[5(S)-(tert.butoxyformamido)-6-cyclohexyl-4(S)-hydroxy-2(S)-isopropylhexanoyl]-$N^1$-phenethyl-L-isoleucinamide in 1.5 ml of dry dichloromethane was stirred under an argon atmosphere and cooled in an ice bath while 0.1 ml of 2,6-lutidine was added followed dropwise by 0.18 ml of tert.butyldimethylsilyl trifluoromethanesulphonate. The solution was stirred at room temperature for 30 minutes and there were then added in succession 10 ml of saturated ammonium chloride solution and 10 ml of dichloromethane. The phases were separated and the aqueous phase was extracted with 5 ml of dichloromethane. The combined organic extracts were dried over sodium sulphate, filtered ad evaporated to give a gum. This gum was dissolved in 3 ml of dry tetrahydrofuran and the solution was treated with 0.75 ml of a 1M solution of tetrabutylammonium fluoride in tetrahydrofuran. The solution was stirred under argon for 1 hour and then 0.75 ml of water was added. The solution was concentrated and the residue was dissolved in 15 ml of dichloromethane. The solution was washed twice with 5 ml of water each time, then dried over sodium sulphate and evaporated to give crude $N^2$-[5(S)-amino-4(S)-(tert.butyldimethylsilyloxy)-6-cyclohexyl-2(S)-isopropylhexanoyl]-$N^1$-phenethyl-L-isoleucinamide as a gum.

(ii) The crude amine obtained according to paragraph (i) above was dissolved in 2 ml of dry dichloromethane and the solution was stirred under an argon atmosphere and cooled in an ice bath while 44 µl of 2,6-lutidine were added followed by 43 µl of benzyl chloroformate. The solution was allowed to come to room temperature and was then stirred overnight. The mixture was diluted with 5 ml of dichloromethane, then washed in succession with in each case 2 ml of water, 10% citric acid solution, water and saturated sodium bicarbonate solution. The solution was dried over sodium sulphate, filtered and evaporated. The residual syrup was purified by flash chromatography on silica gel using 20% ethyl acetate in n-hexane for the elution. There was obtained 0.13 g of $N^2$-[5(S)-benzyloxyformamido-4(S)-(tert.butyldimethylsilyloxy)-6-cyclohexyl-2(S)-isopropylhexanoyl]-$N^1$-phenethyl-L-isoleucinamide as a gum; MS: m/e 736 $[M+H]^+$.

## Example 22

A solution of 0.23 g of $N^2$-[5(S)-[[N-(benzyloxycarbonyl)-L-asparaginyl]amino]-4(S)-(tert.butyldimethylsilyloxy)-6-cyclohexyl-2(S)-isopropylhexanoyl]-$N^1$-phenethyl-L-isoleucinamide in 2.3 ml of triethylamine trihydrofluoride was stirred at room temperature for 3 hours. The resulting mixture was diluted with 20 ml of dichloromethane and treated with 20 ml of saturated sodium hydrogen carbonate solution. The precipitated white solid was filtered off, washed with water and diethyl ether and dried to give 0.13 g of $N^2$-[5(S)-[[N-(benzyloxycarbonyl)-L-asparaginyl]amino]-6-cyclohexyl-4(S)-hydroxy-2(S)-isopropylhexanoyl]-$N^1$-phenethyl-L-isoleucinamide. Analytically pure product was obtained by recrystallization from a mixture of dimethylformamide and ethyl acetate and melted at 272-275° C.

The $N^2$-[5(S)-[[N-(benzyloxycarbonyl)-L-asparaginyl]amino]-4(S)-(tert.butyldimethylsilyloxy)-6-cyclohexyl-2(S)-isopropylhexanoyl]-$N^1$-phenethyl-L-isoleucinamide used as the starting material was prepared as follows:

Crude $N^2$-[5(S)-amino-4(S)-(tert.butyldimethylsilyloxy)-6-cyclohexyl-2(S)-isopropylhexanoyl]-$N^1$-phenethyl-L-isoleucinamide was prepared from 0.25 g of $N^2$-[5(S)-(tert.butoxyformamido)-6-cyclohexyl-4(S)-hydroxy-2(S)-isopropylhexanoyl]-$N^1$-phenethyl-L-isoleucinamide according to the procedure described in Example 21 (i). This crude material was dissolved in 2.5 ml of dry dimethylformamide, the solution was cooled to -20° C in an ice/salt bath and then treated with 0.197 g of the succinimide ester of N-benzyloxycarbonyl-L-asparagine. The mixture was stirred under an argon atmosphere and allowed to warm to room temperature. It was then stirred at room temperature overnight. Dimethylformamide was removed by evaporation and the residue was partitioned between 10 ml of dichloromethane and 10 ml of water. The aqueous phase was extracted with 10 ml of dichloromethane and the combined organic phases were washed in succession with in each case 5 ml of 10% citric acid solution, water, saturated sodium hydrogen carbonate solution and water and then dried over sodium sulphate, filtered and evaporated. The residue was purified by flash chromatography on silica gel using 2% methanol in dichloromethane for the elution. There was obtained 0.23 g of $N^2$-[5(S)-[[N-(benzyloxycarbonyl)-L-asparaginyl]amino]-4(S)-(tert.butyldimethylsilyloxy)-6-cyclohexyl-2(S)-isopropylhexanoyl]-$N^1$-phenethyl-L-isoleucinamide; MS: m/e 850 $[M+H]^+$.

## Example 23

A solution of 0.22 g of $N^2$-[4(S)-acetoxy-5(S)-(tert.butoxyformamido)-6-cyclohexyl-2(S)-isopropylhexanoyl]-$N^1$-phenethyl-L-isoleucinamide in 4.6 ml of a 4M solution of hydrogen chloride in ethyl acetate was stirred at room temperature for 2 hours. The solution was evaporated to dryness and the residue was dissolved in 12 ml of dichloromethane. 5 ml of saturated sodium hydrogen carbonate solution were added and the mixture was stirred vigorously at room temperature overnight, a white precipitate separating. The mixture was filtered and the solid was washed with water and diethyl ether to give, after drying, 0.15 g of $N^2$-[5(S)-acetamido-6-cyclohexyl-4(S)-hydroxy-2(S)-isopropylhexanoyl]-$N^1$-phenethyl-L-isoleucinamide. Recrystallization from methanol gave analytically pure product of melting point 236-237 °C.

The $N^2$-[4(S)-acetoxy-5(S)-(tert.butoxyformamido)-6-cyclohexyl-2(S)-isopropylhexanoyl]-$N^1$-phenethyl-L-isoleucinamide used as the starting material was prepared as follows:

A solution of 0.25 g of $N^2$-[5(S)-(tert.butoxyformamido)-6-cyclohexyl-4(S)-hydroxy-2(S)-isopropylhexanoyl]-$N^1$-phenethyl-L-isoleucinamide and 5 mg of 4-dimethylaminopyridine in 1.5 ml of dry pyridine was treated with 66 mg of acetic anhydride and the mixture was stirred at room temperature overnight. Pyridine was removed by evaporation under reduced pressure and the residue was dissolved in 20 ml of dichloromethane. The solution was washed with 10 ml of 10% citric acid solution, 10 ml of water and 10 ml of saturated sodium hydrogen carbonate solution, dried over sodium sulphate, filtered and evaporated. The crude product was purified by flash chromatography on silica gel using 30% ethyl acetate in n-hexane for the elution. There was obtained 0.23 g of $N^2$-[4(S)-acetoxy-5(S)-(tert.butoxyformamido)-6-cyclohexyl-2(S)-isopropylhexanoyl]-$N^1$-phenethyl-L-isoleucinamide as a white foam.

## Example 24

A solution of 0.15 g of $N^2$-[5(S)-(tert.butoxyformamido)-6-cyclohexyl-4(S)-hydroxy-2(S)-isopropylhexanoyl]-$N^1$-phenethyl-L-isoleucinamide and 50 mg of 4-dimethylaminopyridine in 1 ml of dry acetonitrile was stirred at room temperature while 54 µl of tert.butylacetyl chloride were added dropwise. The mixture was stirred at room temperature overnight and then evaporated to dryness. The residue was worked-up and treated as described in Example 23 to give 67 mg of $N^2$-[5(S)-(tert.butylacetamido)-6-cyclohexyl-4(S)-hydroxy-2(S)-isopropylhexanoyl]-$N^1$-phenethyl-L-isoleucinamide as a white solid. Recrystallization from methanol gave analytically pure product of melting point 242-243 °C.

## Example 25

A solution of 0.2 g of $N^2$-[5(S)-(tert.butoxyformamido)-6-cyclohexyl-4(S)-hydroxy-2(S)-isopropylhexanoyl]-$N^1$-phenethyl-L-isoleucinamide and 68 mg of 4-dimethylaminopyridine in 1.5 ml of dry acetonitrile was stirred at room temperature and treated with 72 mg of isovaleryl chloride. After stirring at room temperature overnight the solution was evaporated to dryness. The residue was worked-up and treated as described in Example 23 to give 69 mg of $N^2$-[6-cyclohexyl-4(S)-hydroxy-2(S)-isopropyl-5(S)-(4-methylvaleramido)hexanoyl]-$N^1$-phenethyl-L-isoleucinamide. Analytically pure product was obtained by recrystallizaton from ethyl acetate and melted at 232 °C.

## Example 26

A solution of 0.13 g of $N^2$-[4(S)-(tert.butyldimethylsilyloxy)-6-cyclohexyl-2(S)-isopropyl-5(S)-(4-toluidino)-hexanoyl]-$N^1$-phenethyl-L-isoleucinamide in 0.2 ml of tetrahydrofuran was treated with 1 ml of triethylamine trihydrofluoride. The mixture was stirred at room temperature under an argon atmosphere overnight, then diluted with 10 ml of chloroform and poured into 15 ml of saturated sodium hydrogen carbonate solution. The aqueous layer was separated and extracted with 5 ml of chloroform. The combined organic phases were washed in succession with in each case 5 ml of saturated sodium hydrogen carbonate solution, water, 10% citric acid solution and water, dried over sodium sulphate, filtered and evaporated to give 0.1 g of $N^2$-

[6-cyclohexyl-4(S)-hydroxy-2(S)-isopropyl-5(S)-(4-toluidino)hexanoyl]-N[1]-phenethyl-L-isoleucinamide as an amorphous solid. Recrystallization from acetonitrile gave analytically pure material of melting point 237-238° C.

The $N^2$-[4(S)-(tert.butyldimethylsilyloxy)-6-cyclohexyl-2(S)-isopropyl-5(S)-(4-toluidino)hexanoyl]-$N^1$-phenethyl-L-isoleucinamide used as the starting material was prepared as follows:

A solution of crude $N^2$-[5(S)-amino-4(S)-(tert.butyldimethylsilyloxy)-6-cyclohexyl-2(S)-isopropylhexanoyl]-$N^1$-phenethyl-L-isoleucinamide was prepared from 0.15 g of $N^2$-[5(S)-(tert.butoxyformamido)-6-cyclohexyl-4-(S)-hydroxy-2(S)-isopropylhexanoyl]-$N^1$-phenethyl-L-isoleucinamide as described in Example 19 (i). This crude material was dissolved in 2 ml of dry dichloromethane and the solution was stirred under an argon atmosphere and cooled in ice while 44 µl of 2,6-lutidine were added followed dropwise by 39 µl of p-toluoyl chloride. The mixture was allowed to warm to room temperature and then stirred at room temperature overnight. The mixture was diluted with 20 ml of dichloromethane, then washed in succession with in each case 5 ml of water, 10% citric acid solution, water, saturated sodium hydrogen carbonate solution and water, dried over sodium sulphate, filtered and evaporated. The residue was purified by flash chromatography on silica gel using 20% ethyl acetate in n-hexane for the elution. There was obtained 0.13 g of $N^2$-[4-(S)-(tert.butyldimethylsilyloxy)-6-cyclohexyl-2(S)-isopropyl-5(S)-(4-toluidino)hexanoyl]-$N^1$-phenethyl-L-isoleucinamide.

## Example 27

73 mg of N-[N-[3-[3-(benzyloxycarbonyl)-4(S)-isobutyl-2,2-dimethyl-5(S)-oxazolidinyl]-2(S)-isopropylpropionyl]-L-isoleucyl]-L-phenylalanine methyl ester were dissolved in 5 ml of dichloromethane and treated with two drops of a solution of freshly prepared hydrogen chloride in methanol. The solid residue obtained after evaporation of the solvent was purified by crystallization from ethyl acetate/n-hexane. There were obtained 41 mg of N-[N-[5(S)-(benzyloxyformamido)-4(S)-hydroxy-2(S)-isopropyl-7-methyloctanoyl]-L-isoleucyl]-L-phenylalanine methyl ester of melting point 203-204° C.

The N-[N-[3-[3-(benzyloxycarbonyl)-4(S)-isobutyl-2,2-dimethyl-5(S)-oxazolidinyl]-2(S)-isopropylpropionyl]-L-isoleucyl]-L-phenylalanine methyl ester used as the starting material was prepared as follows:

1.75 g of triethylamine and 6.54 g of benzotriazol-1-yloxy-tris(dimethylamino)-phosphonium hexafluorphosphate were added in succession to a solution, stirred at room temperature, of 1.4 g of 3-(benzyloxycarbonyl)-4(S)-isobutyl-α(S)-isopropyl-2,2-dimethyl-5(S)-oxazolidinepropionic acid and 2.43 g of N-(L-isoleucyl)-L-phenylalanine methyl ester hydrochloride in 100 ml of acetonitrile. The mixture was stirred at room temperature overnight, the precipitate was subsequently filtered off and the solvent was removed on a rotary evaporator. The residue was extracted three times with 300 ml of ethyl acetate each time, the organic phases were washed in succession with 150 ml of 2M sodium hydrogen carbonate solution, 150 ml of saturated ammonium chloride solution, 150 ml of 2M sodium hydrogen carbonate solution and 150 ml of saturated sodium chloride solution, dried over magnesium sulphate and evaporated. The crude product (7.8 g) was chromatographed on 450 g of silica gel using toluene/ethyl acetate 9:1 and then 4:1 for the elution, whereby there were obtained 1.25 g of a white powder. Recrystallization of this powder form methylene chloride/ n-hexane gave N-[N-[3-[3-(benzyloxycarbonyul)-4(S)-isobutyl-2,2-dimethyl-5(S)-oxazolidinyl]-2(S)-isopropylpropionyl]-L-isoleucyl]-L-phenylalanine methyl ester which melted at about 160° C.

## Example 28

In a manner analogous to that described in the first paragraph of Example 27, using $N^2$-[3-[3-(benzyloxycarbonyl)-4(S)-isobutyl-2,2-dimethyl-5(S)-oxazolidinyl]-2- isopropylpropionyl]-$N^1$-phenethyl-L-isoleucinamide there was obtained $N^2$-[5(S)-(benzyloxyformamido)-4(S)-hydroxy-2(S)-isopropyl-7-methyloctanoyl]-$N^1$-phenethyl-L-isoleucinamide of melting point 218-219° C (from ethyl acetate).

The $N^2$-[3-[3-(benzyloxycarbonyl)-4(S)-isobutyl-2,2-dimethyl-5(S)-oxazolidinyl]-2-isopropylpropionyl]-$N^1$-phenethyl-L-isoleucinamide, melting point 156-157° C (from n-hexane), used as the starting material can be prepared by coupling 3-(benzyloxycarbonyl)-4(S)-isobutyl-α(S)-isopropyl-2,2-dimethyl-5(S)-oxazolidinepropionic acid with $N^1$-phenethyl-L-isoleucinamide according to the procedure described in the last paragraph of Example 27.

The $N^1$-phenethyl-L-isoleucinamide referred to in the preceding paragraph can be obtained by the procedure described in Example 1(a) or by coupling N-tert.butoxycarbonyl-L-isoleucine with phenethylamine in the presence of dicyclohexylcarbodiimide and subsequent cleavage of the tert.butoxycarbonyl protecting group using trifluoroacetic acid.

## Example 29

A suspension of 51 mg of $N^2$-[5(S)-amino-4(S)-hydroxy-2(S)-isopropyl-7-methyloctanyl]-$N^1$-phenethyl-L-isoleucinamide acetate in 3 ml of acetonitrile was treated with 16 mg of ethyldiisopropylamine (solution A).

Separately, a solution of 30 mg of N-(dibenzylacetyl)glycine, 44 mg of benzotriazol-1-yl-oxy-tris-(dimethylamino)-phosphonium hexafluorophosphate and 16 mg of ethyldiisopropylamine in 3 ml of acetone was prepared (solution B).

After the additon of solution B to solution A the mixture was stirred at room temperature for 3 hours, the solvent was removed by evaporation and the residue was suspended in ethyl acetate. After washing with 5% sodium hydrogen carbonate solution, water, 2M citric acid and water the product was purified by chromatography on silica gel using ethyl acetate for the elution. There were obtained 27 mg of $N^2$-[5(S)-[[N-(dibenzylacetyl)glycyl]amino]-4(S)-hydroxy-2(S)-isopropyl-7-methyloctanoyl]-$N^1$-phenethyl-L-isoleucinamide of melting point 214-215 °C after recrystallization from ethyl acetate/ n-hexane.

The $N^2$-[5(S)-amino-4(S)-hydroxy-2(S)-isopropyl-7-methyloctanoyl]-$N^1$-phenethyl-L-isoleucinamide acetate, melting point 156-158 °C (from diethyl ether/ n-hexane), used as the starting material was prepared by hydrogenolyzing $N^2$-[5(S)-(benzyloxyformamido)-4(S)-hydroxy-2(S)-isopropyl-7-methyloctanoyl]-$N^1$-phenethyl-L-isoleucinamide according to the procedure described in Example 34(ii) hereinafter, but using acetic acid/water (4:1) in place of methanolic hydrochloric acid.

The N-(dibenzylacetyl)glycine used as the starting material was prepared as follows:

A suspension of 3.7 g of dibenzylacetic acid and 2.36 g of glycine ethyl ester hydrochloride in 30 ml of dichloromethane was treated with 2.36 ml of triethylamine and 3.49 g of dicyclohexylcarbodiimide. After stirring at room temperature for 18 hours the mixture was filtered and the filtrate was evaporated. The residue was dissolved in ethyl acetate, the organic phase was washed with water, 5% sodium hydrogen carbonate solution, water, 2M citric acid solution and water, dried and purified by chromatography on silica gel using ethyl acetate for the elution. There were obtained 4.9 g of a yellow oil. 350 mg of this oil were dissolved in 2 ml of dioxan and treated with 2.1 ml of 1M sodium hydroxide solution. After stirring for 2 hours the pH was brought to 13 by the additon of 1 ml of 1M sodium hydroxide solution. After stirring at room temperature for a further 2 hours the solvent was removed by evaporation, the aqueous phase was extracted with ethyl acetate, acidified with 1M hydrochloric acid and again extracted with ethyl acetate. The N-(dibenzylacetyl)glycine, obtained by evaporation of the solvent, melted at 133 °C after recrystallization from ethyl acetate. The yield was 125 mg.

## Example 30

In a manner analogous to that described in the first paragraph of Example 27, from $N^1$-benzyl-$N^2$-[3-[3-(benzyloxycarbonyl)-4(S)-isobutyl-2,2-dimethyl-5(S)-oxazolidinyl]-2-isopropylpropionyl]-L-leucinamide there was obtained $N^1$-benzyl-$N^2$-[5(S)-(benzyloxyformamido)-4(S)-hydroxy-2(S)-isopropyl-7-methyloctanoyl]-L-leucinamide of melting point 185-186 °C (from ethyl acetate).

The $N^1$-benzyl-$N^2$-[3-[3-(benzyloxycarbonyl)-4(S)-isobutyl-2,2-dimethyl-5(S)-oxazolidinyl]-2-isopropylpropionyl]-L-leucinamide, melting point 63-65 °C, used as the starting material was prepared by coupling 3-(benzyloxycarbonyl)-4(S)-isobutyl-$\alpha$(S)-isopropyl-2,2-dimethyl-5(S)-oxazolidinepropionic acid with $N^1$-benzyl-L-leucinamide in a manner analogous to that described in the last paragraph of Example 27.

## Example 31

100 mg of $N^2$-[5(S)-amino-4(S)-hydroxy-2(S)-isopropyl-7-methyloctanoyl]-$N^1$-phenethyl-L-isoleucin amide were suspended in 5 ml of acetonitrile and treated with 45 µl of ethyldiisopropylamine. Subsequently, a solution of 86.6 mg of N-(dibenzylcarbamoyl)-L-phenylalanine and 98 mg of benzotriazol-1-yl-oxy-tris-(dimethylamino)-phosphonium hexafluorophosphate in 5 ml of acetonitrile was added dropwise thereto. The mixture was stirred at room temperature for 2 hours and worked-up in the usual manner. The desired product was crystallized from diethyl ether/dichloromethane and there were obtained 180 mg of $N^2$-[5(S)-[[N-(dibenzylcarbamoyl)-L-phenylalanyl]amino]-4(S)-hydroxy-2(S)-isopropyl-7-methyloctanoyl]-$N^1$-phenethyl-L-isoleucinamide as white crystals of melting point 165° C.

The N-(dibenzylcarbamoyl)-L-phenylalanine used as the starting material was prepared as follows:

(i) 1 g of dibenzylamine were dissolved in 50 ml of dichloromethane and the solution was treated at 0° C with 1.73 ml of ethyldiisopropylamine. Subsequently, the solution was treated with 2.6 ml of a 20% solution of phosgene in toluene and the mixture was stirred at 0-10° C for 3 hours. 1.1 g of L-phenylalanine methyl ester hydrochloride were then added thereto and the solution was stirred at 40° C for 12 hours. After the usual working-up, the product was purified by chromatography on silica gel using dichloromethane/diethyl ether (15:1) for the elution. There were obtained 900 mg of N-(dibenzylcarbamoyl)-L-phenylalanine methyl ester as white crystals; MS: m/e 402 $[M]^+$.

(ii) 900 mg of N-(dibenzylcarbamoyl)-L-phenylalanine methyl ester were dissolved in 20 ml of ethanol, 9 ml of 0.5M sodium hydroxide solution were added and the resulting solution was heated to 40° C for 1 hour. After the usual working-up there were otbained 800 mg of N-(dibenzylcarbamoyl)-L-phenylalanine as a white foam; MS: m/e 388 $[M]^+$.

## Example 32

123 mg of $N^2$-[5(S)-[[3-(benzyloxymethyl)-N-(2,3,4-trimethoxyhydrocinnamoyl)-L-histidyl]amino]-4(S)-hydroxy-2(S)-isopropyl-4-methyloctanoyl]-$N^1$-phenethyl-L-isoleucinamide were taken up in a mixture of 10 ml of acetic acid and 2.5 ml of water, treated with 80 mg of palladium-on-carbon and subsequently hydrogenated at room temperature for about 2 hours. The catalyst was filtered off and the filtrate was concentrated almost completely and then brought to pH 8 by the addition of aqueous potassium hydrogen carbonate solution. The desired product thereby crystallized out and was recrystallized from methanol/water. There were obtained 40 mg of $N^2$-[4(S)-hydroxy-2(S)-isopropyl-5(S)-[[N-(2,3,4-trimethoxyhydrocinnamoyl)-L-histidyl]amino]-7-methyloctanoyl]-$N^1$-phenethyl-L-isoleucinamide as white crystals; MS: m/e 807 $[M+H]^+$.

The $N^2$-[5(S)-[[3-(benzyloxymethyl)-N-(2,3,4-trimethoxyhydrocinnamoyl)-L-histidyl]amino]-4(S)-hydroxy-2(S)-isopropyl-4-methyloctanoyl]-$N^1$-phenethyl-L-isoleucinamide, MS: m/e 927 $[M+H]^+$, used as the starting material was prepared as follows:

(i) 2.4 g of 2,3,4-trimethoxyhydrocinnamic acid were dissolved in 80 ml of dichloromethane and a solution of 2.06 g of dicyclohexylcarbodiimide in 80 ml of dichloromethane was subsequently added dropwise while cooling with ice. The mixture was then stirred at room temperature for 2 hours. Subsequently, a solution of 2.89 g of 3-(benzyloxymethyl)-L-histidine methyl ester in dichloromethane was added dropwise at 0° C and the solution was stirred at room temperature for 12 hours. After the usual working-up the crude product was chromatographed on silica gel using dichloromethane/methanol (19:1) for the elution. There were obtained 3.1 g of 3-(benzyloxymethyl)-N-(2,3,4-trimethoxyhydrocinnamoyl)-L-histidine methyl ester in the form of a resin; MS: m/e 511 $[M]^+$.

(ii) Basic saponification of 3-(benzyloxymethyl)-N-(2,3,4-trimethoxyhydrocinnamoyl)-L-histidine methyl ester and subsequent condensation with $N^2$-[5(S)-amino-4(S)-hydroxy-2(S)-isopropyl-7-methyloctanoyl]-$N^1$-phenethyl-L-isoleucinamide in a manner analogous to that described in the first paragraph of Example 31 gave $N^2$-[5(S)-[[3-(benzyloxymethyl)-N-(2,3,4-trimethoxyhydrocinnamoyl)-L-histidyl]amino]-4(S)-hydroxy-2-(S)-isopropyl-4-methyloctanoyl]-$N^1$-phenethyl-L-isoleucinamide as a crystalline solid; MS: m/e 927 $[M+H]^+$.

## Example 33

In a manner analogous to that described in the first paragraph of Example 32, from $N^2$-[5(S)-[[N-(dibenzylcarbamoyl)-3-(benzyloxymethyl)-L-histidyl]amino]-4(S)-hydroxy-2(S)-isopropyl-7-methyloctanoyl]-$N^1$-phenethyl-L-isoleucinamide there was obtained $N^2$-[5(S)-[[N-(dibenzylcarbamoyl)-L-histidyl]amino]-4(S)-hydroxy-2(S)-isopropyl-7-methyloctanoyl]-$N^1$-phenethyl-L-isoleucinamide; MS: m/e 807 $[M+H]^+$.

The $N^2$-[5(S)-[[N-(dibenzylcarbamoyl)-3-(benzyloxymethyl)-L-histidyl]amino]-4(S)-hydroxy-2(S)-

isopropyl-7-methyloctanoyl]-N1-phenethyl-L-isoleucinamide used as the starting material was prepared as follows:

(i) In a manner analogous to that described in Example 31(i), using dibenzylamine, phosgene and 3-(benzyloxymethyl)-L-histidine methyl ester in place of L-phenylalanine methyl ester there was obtained N-(dibenzylcarbamoyl)-3-(benzyloxymethyl)-L-histidine methyl ester; MS: m/e 512 $[M]^+$.

(ii) Basic saponification of N-(dibenzylcarbamoyl)-3-(benzyloxymethyl)-L-histidine methyl ester and subsequent condensation with $N^2$-[5(S)-amino-4(S)-hydroxy-2(S)-isopropyl-7-methyloctanoyl]-$N^1$-phenethyl-L-isoleucinamide in a analogous manner to that described in the first paragraph of Example 31 yielded $N^2$-[5(S)-[[N-(dibenzylcarbamoyl)-3-(benzyloxymethyl)-L-histidyl]amino]-4(S)-hydroxy-2(S)-isopropyl-7-methyloctanoyl]-$N^1$-phenethyl-L-isoleucinamide as a white powder; MS: m/e 927 $[M+H]^+$.

## Example 34

In a manner analogous to that described in the first paragraph of Example 31, from N-[N-(5-amino-4(S)-hydroxy-2(S)-isopropyl-7-methyloctanoyl]-L-isoleucyl]-L-histidine methyl ester and N-(benzyloxycarbonyl)-L-glutamine there was obtained N-[N-[5(S)-[[N-(benzyloxycarbonyl)-L-glutaminyl]amino]-4(S)-hydroxy-2(S)-isopropyl-7-methyloctanoyl]-L-isoleucyl]-L-histidine methyl ester; MS: m/e 758 $[M+H]^+$.

The N-[N-(5-amino-4(S)-hydroxy-2(S)-isopropyl-7-methyloctanoyl)-L-isoleucyl]-L-histidine methyl ester used as the starting material was prepared as follows:

(i) In a manner analogous to that described in the second paragraph of Example 27, from 3-(benzyloxycarbonyl)-4(S)-isobutyl-α(S)-isopropyl-2,2-dimethyl-5(S)-oxazolidinepropionic acid and N-(L-isoleucyl)-L-histidine methyl ester dihydrochloride there was obtained N-[N-3-[3- (benzyloxycarbonyl)-4(S)-isobutyl-2,2-dimethyl-5(S)-oxazolidinyl]-2(S)-isopropylpropionyl]-L-isoleucyl]-L-histidine methyl ester; MS: 670 $[M+H]^+$.

(ii) 0.5 g of the above methyl ester in 100 ml of 0.5M methanolic hydrochloric acid was hydrogenated overnight in the presence of 100 mg of 5% palladium/carbon. After removing the catalyst by filtration over Dicalite and evaporation on a rotary evaporator the residue was chromatographed over 200 g of silica gel using dichloromethane/methanol/ammonia (80:10:1) for the elution and subsequently recrystallized form methanol, dichloromethane and diethyl ether. There was otbained N-[N-(5-amino-4(S)-hydroxy-2(S)-isopropyl-7-methyloctanoyl)-L-isoleucyl]-L-histidine methyl ester of melting point 137° C.

## Example 35

In a manner analogous to that described in the first paragraph of Example 31, from N-[N-(5-amino-4(S)-hydroxy-2(S)-isopropyl-7-methyloctanoyl)-L-isoleucyl]-L-histidine methyl ester and 6- (dibenzylcarbamoyl)-4-oxohexanoic acid there was obtained N-[N-[5(S)-[6-(dibenzylcarbamoyl)-4-oxohexanamido]-4(S)-hydroxy-2-(S)-isopropyl-7-methyloctanoyl]-L-isoleucyl]-L-histidine methyl ester of melting point 182° C (after recrystallization from methanol, dichloromethane and ether).

## Example 36

In a manner analogous to that described in the first paragraph of Example 22, from 0.20 g of $N^2$-5(S)-[-[N-(benzyloxycarbonyl)-ß-cyano-L-alanyl]amino]-4(S)-(tert.butyldimethylsilyloxy)-6-cyclohexyl-2(S)-isopropylhexanoyl]-$N^1$-phenethyl-L-isoleucinamide there was obtained 0.17 g of $N^2$-[5(S)-[[N-(benzyloxycarbonyl)-ß- cyano-L-alanyl]amino]-6-cyclohexyl-4(S)-hydroxy-2(S)-isopropylhexanoyl]-$N^1$-phenethyl-L-isoleucinamide. Analytically pure product was obtained by recrystallization from methanol and melted at 247-248° C; MS: m/e 718 $(M+H)^+$.

The $N^2$-[5(S)-[[N-(benzyloxycarbonyl)-ß-cyano-L-alanyl]amino]-4(S)-(tert.butyldimethylsilyloxy)-6-cyclohexyl-2(S)-isopropylhexanoyl]-$N^1$-phenethyl-L-isoleucinamide used as the starting material was prepared as follows:-

Crude $N^2$-[5(S)-amino-4(S)-(tert.butyldimethylsilyloxy)-6-cyclohexyl-2(S)-isopropylhexanoyl]-$N^1$-phenethyl-L-isoleucinamine was prepared from 0.2 g of $N^2$-[5(S)-(tert.butoxyformamido)-6-cyclohexyl-4(S)-hydroxy-2(S)-isopropylhexanoyl]-N$^1$-phenethyl-L-isoleucinamide according to the procedure described in Example 21(i). This crude material was dissolved in 4 ml of dry tetrahydrofuran, the solution was cooled to -20° C in an ice/salt bath and then treated with 0.09 g of N-(benzyloxycarbonyl)-ß-cyano-L-alanine, 0.06 g of 1-hydroxybenzotriazole and 0.08 g of dicyclohexylcarbodiimide. The mixture was allowed to warm to room temperature and then was stirred at room temperature for 18 hours. The mixture was filtered and the filtrate was evaporated. The residue was partitioned between 20 ml of dichloromethane and 20 ml of water. The dichloromethane solution was washed in succession with in each case 10 ml of 10% citric acid solution, water, saturated sodium hydrogen carbonate solution and water and then dried over sodium sulphate, filtered and evaporated. The residue was purified by flash chromatography on silica gel using ethyl acetate/ hexane (1:2) for the elution. There was obtained 0.20 g of N$^2$-[5(S)-[[N-(benzyloxycarbonyl)-ß-cyano-L-alanyl]amino-4(S)-(tert.butyldimethylsilyloxy)-6-cyclo hexyl-2(S)-isopropylhexanoyl]-N$^1$-phenethyl-L-isoleucinamide; MS: 832 $(M+H)^+$.

## Example 37

In a manner analogous to that described in the first paragraph of Example 22, form 0.16 g of N$^2$-[5(S)-[-[N-(benzyloxycarbonyl)-L-isoleucyl]amino]-4(S)-(tert.butyldimethylsilyloxy)-6-cyclohexyl-2(S)-isopropylhexanoyl]-N$^1$-phenethyl-L-isoleucinamide there was obtained 0.14 g of N$^2$-[5(S)-[[N-(benzyloxycarbonyl)-L-isoleucyl]amino]-6-cyclohexyl-4(S)-hydroxy-2(S)-isopropylhexanoyl-N$^1$-phenethyl-L-isoleucinamide. Analytically pure product was obtained by recrystallization from dimethylformamide and melted at 279-280° C; MS: m/e 735 $(M+H)^+$.

The N$^2$-[5(S)-[[N-(benzyloxycarbonyl)-L-isoleucyl]amino]-4(S)-(tert.butyldimethylsilyloxy)-6-cyclohexyl-2-(S)-isopropylhexanoyl]-N$^1$-phenethyl-L-isoleucinamide used as the starting material was prepared as follows:

Crude N$^2$-[5(S)-amino-4(S)-(tert.butyldimethylsilyloxy)-6-cyclohexyl-2(S)-isopropylhexanoyl]-N$^1$-phenethyl-L-isoleucinamide was prepared from 0.2 g of N$^2$-[5(S)-(tert.butoxyformamido)-6-cyclohexyl-4(S)-hydroxy-2(S)-isopropylhexanoyl]-N$^1$-phenethyl-L-isoleucinamide according to the procedure described in Example 21(i). This crude material was reacted with 0.12 g of N-(benzyloxycarbonyl)-L-isoleucine succinimide ester in a manner analogous to that described in the third paragraph of Example 22 to give 0.26 g of N$^2$-[5(S)-[[N-benzyloxycarbonyl)-L-isoleucyl]amino]-4(S)-(tert.butyldimethylsilyloxy)-6-cyclohexyl-2(S)-isopropylhexanoyl]-N$^1$-phenethyl-L-isoleucinamide; MS: 849 $(M+H)^+$.

## Example 38

In a manner analogous to that described in the first paragraph of Example 1, from 0.6 g of N$^1$-[2-[4-(benzyloxycarbonyl)-1-piperazinyl]ethyl]-N$^2$-[3-[3-(tert.butoxycarbonyl)-4(S)-(cyclohexylmethyl)-2,2-dimethyl-5(S)-oxazolidinyl]-2(S)-isopropylpropionyl]-L-isoleucinamide there was obtained 0.28 g of N$^1$-[2-[4-(benzyloxycarbonyl)-1-piperazinyl]ethyl]-N$^2$-[5(S)-(tert.butoxyformamido)-6-cyclohexyl-4(S)-hydroxy-2(S)-isopropylhexanoyl]-L-isoleucinamide. Analytically pure product was obtained by recrystallization from acetonitrile and melted at 166-169° C; MS: 730 $(M+H)^+$.

The N$^1$-[2-[4-(benzyloxycarbonyl)-1-piperazinyl]ethyl]-N$^2$-[3-[3-(tert.butoxycarbonyl)-4(S)-(cyclohexylmethyl)-2,2-dimethyl-5(S)-oxazolidinyl]-2(S)-isopropylpropionyl]-L-isoleucinamide used as the starting material was prepared as follows:

A solution of 1.0 g of 3-(tert.butoxycarbonyl)-4(S)-(cyclohexylmethyl)-2,2-dimethyl-α(S)-isopropyl-5(S)-oxazolidinepropionic acid in 10 ml of dimethylformamide was reacted with 1.0 g of N$^1$-[2-[4-(benzyloxycarbonyl)-1-piperazinyl]ethyl]-L-isoleucinamide in the presence of 0.43 g of 1-hydroxybenzotriazole and 0.60 g of dicyclohexylcarbodiimide according to the procedure described in Example 1(iv) to give, after flash chromatography using ethyl acetate/hexane (2:1) for the elution, 1.2 g of N$^1$-[2-[4-(benzyloxycarbonyl)-1-piperazinyl]ethyl]-N$^2$-[3-[3-(tert.butoxycarbonyl)-4(S)-(cyclohexylmethyl)-2,2-dimethyl-5(S)-oxazolidinyl]-2(S)-isopropylpropionyl]-L-isoleucinamide as a white foam; MS: 770 $(M+H)^+$.

The N$^1$-[2-[4-(benzyloxycarbonyl)-1-piperazinyl]ethyl]-L-isoleucinamide referred to in the preceding paragraph was prepared as follows:

(a) A solution of 4.1 g of N-(tert.butoxycarbonyl)-L-isoleucine succinimide ester in 80 ml of dry tetrahydrofuran was reacted with 3.3 g of 1-(2-aminoethyl)-4-(benzyloxycarbonyl)piperazine according to the procedure described in Example 9(a) to give 4.2 g of N$^1$-[2-[4-(benzyloxycarbonyl)-1-piperazinyl]ethyl]-N$^2$-

(tert.butoxycarbonyl)-L-isoleucinamide as a white solid of melting point 83-87° C; MS: 477 $(M+H)^+$.

(b) A solution of 1.4 g of $N^1$-[2-[4-(benzyloxycarbonyl)-1-piperazinyl]ethyl]-$N^2$-(tert.butoxycarbonyl)-L-isoleucinamide in 28 ml of a 4M solution of hydrogen chloride in ethyl acetate was stirred at room temperature for 2 hours. The solution was evaporated to dryness and the residue partitioned between 100 ml of dichloromethane and 50 ml of 1M sodium hydroxide solution. The dichloromethane solution was washed with 50 ml of water and then dried over sodium sulphate and filtered. The filtrate was evaporated to give 1.05 g of $N^1$-[2-[4-(benzyloxycarbonyl)-1-piperazinyl]ethyl]-L-isoleucinamide as a syrup; MS: 377 $(M+H)^+$.

## Example 39

A solution of 0.17 g of $N^1$-[2-[4-(benzyloxycarbonyl)-1-piperazinyl]ethyl]-$N^2$-[5(S)-(tert.butoxyformamido)-6-cyclohexyl-4(S)-hydroxy-2(S)-isopropylhexanoyl]-L-isoleucinamide in 2 ml of ethanol was hydrogenated at atmospheric temperature and pressure in the presence of 10 mg of 10% palladium on carbon catalyst. After 24 hours the catalyst was removed by filtration and the filtrate was evaporated to give 0.13 g of $N^2$-[5-(S)-(tert.butoxyformamido)-6-cyclohexyl-4(S)-hydroxy-2(S)-isopropylhexanoyl]-$N^1$-[2-(1-piperazinyl)ethyl]-L-isoleucinamide. Analytically pure material was obtained by recrystallization form acetonitrile and melted at 137-140° C; MS: 596 $(M+H)^+$.

## Example 40

In a manner analogous to that described in the first paragraph of Example 1, from 0.12 g of $N^2$-[3-[3-(tert.butoxycarbonyl)-4(S)-(cyclohexylmethyl)-2,2-dimethyl-5(S)-oxazolidinyl]-2(S)-isopropylpropionyl]-$N^1$-[2-(1-imidazolyl)ethyl]-L-isoleucinamide there was otbained 75 mg of $N^2$-[5(S)-(tert.butoxyformamido)-6-cyclohexyl-4(S)-hydroxy-2(S)-isopropylhexanoyl]-$N^1$-[2-(1-imidazolyl)ethyl]-L-isoleucinamide. Analytically pure product was obtained by recrystallization from acetonitrile and melted at 192° C; MS: 578 $(M+H)^+$.

The $N^2$-[3-[3-(tert.butoxycarbonyl)-4(S)-(cyclohexylmethyl)-2,2-dimethyl-5(S)-oxazolidinyl]-2(S)-isopropylpropionyl]-$N^1$-[2-(1-imidazolyl)ethyl]-L-isoleucinamide used as the starting material was prepared as follows:

A solution of 0.13 g of N-(tert.butoxycarbonyl)-4(S)-(cyclohexylmethyl)-2,2-dimethyl-α(S)-isopropyl-5(S)-oxazolidinepropionic acid in 3 ml of dimethylformamide was reacted with 0.07 g of $N^1$-[2-(1-imidazolyl)-ethyl]-L-isoleucinamide in the presence of 0.04 g of 1-hydroxybenzotriazole and 0.065 g of dicyclohexylcarbodiimide according to the procedure described in Example 1(iv) to give after flash chromatography using methanol/dichloromethane (1:19) for the elution 0.12 g of $N^2$-[3-[3-(tert.butoxycarbonyl)-4(S)-(cyclohexylmethyl)-2,2-dimethyl-5(S)-oxazolidinyl]-2(S)-isopropylpropionyl]-$N^1$-[2-(1-imidazolyl)ethyl]-L-isoleucinamide as a foam.

The $N^1$-[2-(1-imidazolyl)ethyl]-L-isoleucinamide referred to in the preceding paragraph was prepared as follows:

(a) A solution of 0.34 g of N-(benzyloxycarbonyl)-L-isoleucine succinimide ester in 3 ml of dry dimethylformamide was reacted with 0.11 g of 1-(2-aminoethyl)imidazole according to the procedure described in Example 9(a). There was obtained 0.13 g of $N^2$-(benzyloxycarbonyl)-$N^1$-[2-(1-imidazolyl)ethyl]-L-isoleucinamide.

(b) By a procedure analogous to that described in Example 9(b) from 0.13 g of $N^2$-(benzyloxycarbonyl)-$N^1$-[2-(1-imidazolyl)ethyl]-L-isoleucinamide there was obtained 71 mg of $N^1$-[2-(1-imidazolyl)ethyl]-L-isoleucinamide as a gum.

## Example 41

In a manner analogous to that described in the first paragraph of Example 1, from 0.25 g of $N^2$-[3-[3-(tert.butoxycarbonyl)-4(S)-(cyclohexylmethyl)-2,2-dimethyl-5(S)-oxazolidinyl]-2(S)-isopropylpropionyl]-$N^1$-[2-(4-thiomorpholino)ethyl]-L-isoleucinamide there was obtained 0.13 g of $N^2$-[5(S)-(tert.butoxyformamido)-6-cyclohexyl-4(S)-hydroxy-2(S)-isopropylhexanoyl]-$N^1$-[2-(4-thiomorpholino)ethyl]-L-isoleucinamide. Analytically pure product was obtained by recrystallization from acetonitrile and melted at 151-153 °C; MS: 613 $(M+H)^+$.

The $N^2$-[3-[3-(tert.butoxycarbonyl)-4(S)-(cyclohexylmethyl)-2,2-dimethyl-5(S)-oxazolidinyl]-2(S)-isopropylpropionyl]-$N^1$-[2-(4-thiomorpholino)ethyl]-L-isoleucinamide used as the starting material was prepared as follows:

A solution of 0.29 g of N-(tert.butoxycarbonyl)-4(S)-(cyclohexylmethyl)-2,2-dimethyl-α(S)-isopropyl-5(S)-oxazolidinepropionic acid in 3 ml of dimethylformamide was reacted with 0.18 g of $N^1$-[2-(4-thiomorpholino)-ethyl]-L-isoleucinamide in the presence of 0.09 g of 1-hydroxybenzo triazole and 0.14 g of dicyclohexylcarbodiimide according to the procedure described in Example 1(iv). There was obtained after flash chromatography using ethyl acetate/hexane (2:1) for the elution 0.25 g of $N^2$-[3-[3-(tert.butoxycarbonyl)-4(S)-(cyclohexylmethyl)-2,2-dimethyl-5(S)-oxazolidinyl]-2(S)-isopropylpropionyl]-$N^1$-[2-(4-thiomorpholino)ethyl]-L-isoleucinamide; MS: 653 $(M+H)^+$.

The $N^1$-[2-(4-thiomorpholino)ethyl]-L-isoleucinamide referred to in the preceding paragraph was prepared as follows:

(a) A solution of 0.5 g of N-(benzyloxycarbonyl)-L-isoleucine succinimide ester in 5 ml of dimethylformamide was reacted with 0.21 g of 4-(2-aminoethyl)thiomorpholine according to the procedure described in Example 9(a). There was obtained 0.36 g of $N^2$-(benzyloxycarbonyl)-$N^1$-[2-(4-thiomorpholino)ethyl]-L-isoleucinamide; MS: 394 $(M+H)^+$.

(b) A mixture of 0.36 g of $N^2$-(benzyloxycarbonyl)-$N^1$-[2-(4-thiomorpholino)ethyl]-L-isoleucinamide and 0.90 ml of a 32% (w/w) solution of hydrogen bromide in acetic acid was stirred at room temeprature for 1.5 hours. The resulting solution was diluted with 30 ml of anhydrous diethyl ether. After stirring for 0.5 hours the precipitated solid was filtered and washed with fresh ether, then dissolved in water. The solution was neutralized by additon of solid potassium carbonate and was then extracted three times with 10 ml of chloroform. The combined chloroform extracts were dried over sodium sulphate then filtered and the filtrate was evaporated to give 0.18 g of $N^1$-[2-(4-thiomorpholino)ethyl]-L-isoleucinamide as a gum.

## Example 42

In a manner analogous to that described in the first paragraph of Example 1, from 80 mg of $N^2$-[3-[3-tert.butoxycarbonyl)-4(S)-(cyclohexylmethyl)-2,2-dimethyl-5(S)-oxazolidinyl]-2(S)-isopropylpropionyl]-$N^2$-[2-(1-oxothiomorpholino)ethyl]-L-isoleucinamide there was obtained 41 mg of $N^2$-[5(S)-(tert.butoxyformamido)-6-cyclohexyl-4(S)-hydroxy-2(S)-isopropylhexanoyl]-$N^1$-[2-(1-oxothiomorpholino)ethyl]-L-isoleucinamide, MS: 629 $(M+H)^+$.

The $N^2$-[3-[3-tert.butoxycarbonyl)-4(S)-(cyclohexylmethyl)-2,2-dimethyl-5(S)-oxazolidinyl]-2(S)-isopropylpropionyl]-$N^1$-[2-(1-oxothiomorpholino)ethyl]-L-isoleucinamide used as the starting material was prepared as follows:

A solution of 0.14 g of N-(tert.butoxycarbonyl)-4(S)-(cyclohexylmethyl)-2,2-dimethyl-α(S)-isopropyl-5(S)-oxazolidinepropionic acid in 2 ml of dimethylformamide was reacted with 90 mg of $N^1$-[2-(1-oxothiomorpholino)ethyl]-L-]isoleucinamide in the presence of 45 mg of 1-hydroxybenzotriazole and 70 mg of dicyclohexylcarbodiimide according to the procedure described in Example 1(iv). There was obtained 83 mg of $N^2$-[3-[3-tert.butoxycarbonyl)-4(S)-(cyclohexylmethyl)-2,2-dimethyl-5(S)-oxazolidinyl]-2(S)-isopropylpropionyl]-$N^1$-[2-(1-oxothiomorpholino)ethyl]-L-isoleucinamide; MS: 669 $(M+H)^+$.

The $N^1$-[2-(1-oxothiomorpholino)ethyl]-L-isoleucinamide referred to in the preceding paragraph was prepared as follows:

(a) A mixture of 0.5 g of $N^2$-(tert.butoxycarbonyl)-$N^1$-[2-(4-thiomorpholino)ethyl]-L-isoleucinamide and 3.2 ml of 0.5M sodium metaperiodate solution was stirred at room temperature for 18 hours. The mixture was filtered and the filtrate extracted three times with 5 ml of chloroform. The combined extracts were dried over sodium sulphate then filtered and the filtrate was evaporated. The residue was purified by flash chromatography on silica gel using methanol/dichloromethane (3:97) for the elution. There was obtained 0.17 g of $N^2$-(tert.butoxycarbonyl)-$N^1$[2-(1-oxothiomorpholino)ethyl]-L-isoleucinamide; MS: 376 $(M+H)^+$.

(b) By a procedure analogous to that described in Example 38(b), form 0.2 g of $N^2$-(tert.butoxycarbonyl)-$N^1$-[2-(1-oxothiomorpholino)ethyl]-L-isoleucinamide there was obtained 92 mg of $N^1$-[2-(1-oxothiomorpholino)ethyl]-L-isoleucinamide as a gum.

## Example 43

In a manner analogous to that described in the first paragraph of Example 1, from 0.64 g of $N^1$-[2-(benzyloxycarbonyl)ethyl]-$N^2$-[3-[3-(tert.butoxycarbonyl)-4(S)-(cyclohexylmethyl)-2,2-dimethyl-5(S)-oxazolidinyl]-2(S)-isopropylpropionyl]-L-isoleucinamide there was obtained 0.30 g of $N^1$-[2-(benzyloxycarbonyl)ethyl]-$N^2$-5(S)-(tert.butoxyformamido)-6-cyclohexyl-4(S)-hydroxy-2(S)-isopropylhexanoyl]-L-isoleucinamide. Analytically pure product was obtained by recrystallization from ethyl acetate/hexane and melted at 136-140 °C; MS: 646 $(M+H)^+$.

The $N^1$-[2-(benzyloxycarbonyl)ethyl]-$N^2$-[3-[3-(tert.butoxycarbonyl)-4(S)-(cyclohexylmethyl)-2,2-dimethyl-5(S)-oxazolidinyl]-2(S)-isopropylpropionyl]-L-isoleucinamide used as the starting material was prepared as follows:

A solution of 0.49 g of N-(tert.butoxycarbonyl)-4(S)-(cyclohexylmethyl)-2,2-dimethyl-α(S)-isopropyl-5(S)-oxazolidinepropionic acid in 10 ml of dimethylformamide was reacted with 0.35 g of $N^1$-[2-(benzyloxycarbonyl)ethyl]-L-isoleucinamide in the presence of 0.16 g of 1-hydroxybenzotriazole and 0.25 g of dicyclohexylcarbodiimide according to the procedure described in Example 1(iv) to give after flash chromatography using methanol/dichloromethane (3:97) for the elution 0.64 g of $N^1$-[2-(benzyloxycarbonyl)-ethyl]-$N^2$-[3-[3-(tert.butoxycarbonyl)-4(S)-(cyclohexylmethyl)-2,2-dimethyl-5(S)-oxazolidinyl]-2(S)-isopropylpropionyl]-L-isoleucinamide as a gum.

The $N^1$-[2-(benzyloxycarbonyl)ethyl]-L-isoleucinamide referred to in the preceding paragraph was prepared as follows:

(a) A solution of 0.81 g of N-(tert.butoxycarbonyl)-L-isoleucine succinimide ester in 15 ml of dry tetrahydrofuran was reacted with 0.44 g of 2-(benzyloxycarbonyl)ethylamine by a procedure analogous to that described in Example 9(a). There was obtained, after flash chromatography using methanol/dichloromethane (1:49) for the elution, 0.53 g of $N^1$-[2-(benzyloxycarbonyl)ethyl]-$N^2$-(tert.butoxycarbonyl)-L-isoleucinamide; MS: 393 $(M+H)^+$.

(b) By a procedure analogous to that described in Example 38(b), from 0.53 g of $N^1$-[2-(benzyloxycarbonyl)ethyl]-$N^2$-(tert.butoxycarbonyl)-L-isoleucinamide there was obtained 0.37 g of $N^1$-[2-(benzyloxycarbonyl)ethyl]-L-isoleucinamide as a gum.

## Example 44

A solution of 0.12 g of $N^1$-[2-(benzyloxycarbonyl)ethyl]-$N^2$-[5(S)-(tert.butoxyformamido)-6-cyclohexyl-4-(S)-hydroxy-2(S)-isopropylhexanoyl]-L-isoleucinamide in 10 ml of ethyl acetate was hydrogenated at atmospheric temperature and pressure in the presence of 20 mg of 5% palladium on carbon catalyst for 24 hours. The catalyst was removed by filtration and was washed with dichloromethane. Evaporation of the filtrate gave 85 mg of $N^2$-[5(S)-(tert.butoxyformamido)-6-cyclohexyl-4(S)-hydroxy-2(S)-isopropylhexanoyl]-$N^1$-[2-carboxyethyl]-L-isoleucinamide. Analytically pure product was obtained by recrystallization from acetonitrile and melted at 164-166 °C; MS: 556 $(M+H)^+$.

## Example 45

In a manner analogous to that described in the first paragraph of Example 1, from 0.17 g of $N^2$-[3-[3-(tert.butoxycarbonyl)-4(S)-(cyclohexylmethyl)-2,2-dimethyl-5(S)-oxazolidinyl]-2(S)-isopropylpropionyl]-$N^1$-[2-(4-hydroxyphenyl)ethyl]-L-isoleucinamide there was otbained 73 mg of $N^2$-[5(S)-(tert.butoxyformamido)-6-cyclohexyl-4(S)-hydroxy-2(S)-isopropylhexanoyl]-$N^1$-[2-(4-hydroxyphenyl)ethyl]-L-isoleucinamide. Analytically pure product was obtained by recrystallization from acetonitrile and melted at 291-293 °C; MS: 604 $(M+H)^+$.

The $N^2$-[3-[3-(tert.butoxycarbonyl)-4(S)-(cyclohexylmethyl)-2,2-dimethyl-5(S)-oxazolidinyl]-2(S)-isopropylpropionyl]-$N^1$-[2-(4-hydroxyphenyl)ethyl]-L-isoleucinamide used as the starting material was prepared as follows:

A solution of 0.20 g of N-(tert.butoxycarbonyl)-4(S)-(cyclohexylmethyl)-2,2-dimethyl-α(S)-isopropyl-5(S)-oxazolidinepropionic acid in 4 ml of dimethylformamide was reacted with 0.12 g of $N^1$-[2-(4-hydroxyphenyl)-ethyl]-L-isoleucinamide in the presence of 0.065 g of 1-hydroxybenzotriazole and 0.10 g of dicyclohexylcarbodiimide according to the procedure described in Example I(iv) to give after flash chromatography using

methanol/dichloromethane (3:97) for the elution 0.17 g of $N^2$-[3-[3-tert.butoxycarbonyl)-4(S)-(cyclohexylmethyl)-2,2-dimethyl-5(S)-oxazolidinyl]-2(S)-isopropylpropionyl]-$N^1$-[2-(4-hydroxyphenyl)ethyl]-L-isoleucinamide; MS: 644 $(M+H)^+$.

The $N^1$-[2-(4-hydroxyphenyl)ethyl]-L-isoleucinamide referred to in the preceding paragraph was prepared as follows:

(a) A solution of 1.0 g of N-(tert.butoxycarbonyl)-L-isoleucine succinimide ester in 20 ml of dry tetrahydrofuran was reacted with 0.42 g of 2-(4-hydroxyphenyl)ethylamine by a procedure analogous to that described in Example 9(a). There was obtained, after flash chromatography using methanol/dichloromethane (3:97) for the elution, 0.67 g of $N^2$-(tert.butoxycarbonyl)-$N^1$-[2-(4-hydroxyphenyl)ethyl]-L-isoleucinamide as a foam.

(b) By a procedure analogous to that described in Example 38(b), from 0.2 g of $N^2$-(tert.butoxycarbonyl)-$N^1$-[2-(4-hydroxyphenyl)ethyl]-L-isoleucinamide there was obtained 0.12 g of $N^1$-[2-(4-hydroxyphenyl)ethyl)-L-isoleucinamide as a white solid; MS: 251 $(M+H)^+$.

## Example 46

A solution of 0.24 g of $N^2$-[5(S)-(tert.butoxyformamido)-6-cyclohexyl-2(S)-isopropyl-4-oxohexanoyl]-$N^1$-phenethyl-L-isoleucinamide in 5 ml of pyridine was stirred and cooled in a bath of ice while 50 mg of dimethylaminopyridine was added followed by 31 mg of hydroxylamine hydrochloride. The mixture was allowed to warm to room temperature and then was stirred for 18 hours. Pyridine was removed by evaporation under reduced pressure and the residue was partitioned between 10 ml of dichloromethane and 10 ml of water. The dichloromethane solution was washed in succession with in each case 5 ml of 5% citric acid solution, water, saturated sodium hydrogen carbonate solution and water then was dried over sodium sulphate and filtered. The filtrate was evaporated to give 0.24 g of crude $N^2$-[5(S)-(tert.butoxyformamido)-6-cyclohexyl-2(S)-isopropyl-4-hydroxyiminohexanoyl]-$N^1$-phenethyl-L-isoleucinamide as a mixture of syn and anti isomers. These could be separated by flash chromatography on silica gel using ethyl acetate/hexane (1:2) for the elution. Evaporation of appropriate fractions gave 0.11 g of the faster eluting isomer as a solid foam; MS: 601 $(M+H)^+$. Evaporation of later fractions gave 43 mg of the second isomer which was recrystallized form acetonitrile; MS: 601 $(M+H)^+$.

The $N^2$-[5(S)-(tert.butoxyformamido)-6-cyclohexyl-2(S)-isopropyl-4-oxohexanoyl]-$N^1$-phenethyl-L-isoleucinamide used as the starting material was prepared as follows:

A mixture of 0.3 g of $N^2$-[5(S)-(tert.butoxyformamido)-6-cyclohexyl-4(S)-hydroxy-2(S)-isopropylhexanoyl]-$N^1$-phenethyl-L-isoleucinamide, 1.71 ml of triethylamine and 1.2 ml of dimethyl sulphoxide was stirred under argon and cooled in ice while a solution of 0.81 g of sulphur trioxide/pyridine complex in 1.6 ml of dimethyl sulphoxide was added dropwise. The mixture was stirred at room temperature for 4 hours and then was poured onto 6 ml of ice/water. The mixture was extracted three times with 10 ml of ethyl acetate. The combined ethyl acetate extracts were washed twice with 10 ml of 10% citric acid solution and then with 5 ml of water and 5 ml of saturated sodium hydrogen carbonate solution before drying over sodium sulphate. The solution was filtered and the filtrate was evaporated. The residue was purified by flash chromatography on silica gel using ethyl acetate/hexane (1:3) for the elution. There was obtained 0.26 g of $N^2$-[5(S)-(tert.butoxyformamido)-6-cyclohexyl-2(S)-isopropyl-4-oxohexanoyl]-$N^1$-phenethyl-L-isoleucinamide as a white solid. Analytically pure material was obtained by recrystallization from acetonitrile and melted at 184-185°C; MS: 586 $(M+H)^+$.

## Example 47

A solution of 90 mg of $N^2$-[5(S)-(tert.butoxyformamido)-6-cyclohexyl-2(S)-isopropyl-4-hydroxyiminohexanoyl]-$N^1$-phenethyl-L-isoleucinamide in 2 ml of 7.5M methanolic ammonia solution was hydrogenated at room temperature and atmospheric pressure int he presence of a catalytic quantity of Raney nickel for 5 hours. The catalyst was removed by filtration and the filtrate was evaporated to give 75 mg of $N^2$-[4(R,S)-amino-5(S)-(tert.butoxyformamido)-6-cylohexyl-2(S)-isopropylhexanoyl]-$N^1$-phenethyl-L-isoleucinamide. The mixture of epimers was separated by flash chromatography on silica gel using methanol/dichloromethane (1:49) for the elution. Evaporation of appropriate fractions gave 35 mg of the faster eluting isomer which was recrystallized from acetonitrile and then melted at 169-170°C; MS: 587

$(M+H)^+$. Evaporation of later fractions gave 19 mg of the second isomer as a solid foam; MS: 587 $(M+H)^+$.

## Example 48

A solution of 0.40 g of N[2]-[5(R)-[1(S)-(tert.butoxyformamido)-2-cyclohexylethyl]-2,2-dimethyl-4(R)-dioxolanyl]-2(R)-isopropylacetyl]-N[1]-phenethyl-L-isoleucinamide in 8 ml of methanol was treated with 12 mg of p-toluenesulphonic acid. The solution was stirred at room temperature for 2 days and then evaporated. The residue was dissolved in 100 ml of dichloromethane and the solution was washed twice with 60 ml of saturated sodium hydrogen carbonate solution and with 60 ml of sodium chloride solution, then dried over sodium sulphate. The solution was filtered and the filtrate was evaporated. The residue was purified by flash chromatography on silica gel using ethyl acetate/hexane (2:3) for the elution. There was obtained 77 mg of N[2]-[5(S)-(tert.butoxyformamido)-6-cyclohexyl-3(R),4(R)-dihydroxy-2(R)-isopropylhexanoyl]-N[1]-phenethyl-L-isoleucinamide and 0.28 g of recovered starting material. The recovered starting material was redissolved in 6 ml of methanol and treated again with 8 mg of p-toluenesulphonic acid as described above to give after flash chromatography a further 21 mg of product. Combined crops of product were recrystal lized from methanol to give colourless needles which melted at 208-209° C; MS: 604 $(M+H)^+$.

The N[2]-[5(R)-[1(S)-(tert.butoxyformamido)-2-cyclohexylethyl]-2,2-dimethyl-4(R)-dioxolanyl]-2(R)-isopropylacetyl]-N[1]-phenethyl-L-isoleucinamide used as the starting material was prepared as follows:

A solution of 0.40 g of 2(R)-[5(R)-[1(S)-(tert.butoxyformamido)-3-cyclohexylethyl]-2,2-dimethyl-4(R)-dioxolanyl]-3-methylbutanoic acid [prepared as described in PCT/US87/00291 (WO 87/05302)] in 5 ml of dimethylformamide was reacted with 0.23 g of N[1]-phenethyl-L-isoleucinamide in the presence of 0.15 g of 1-hydroxybenzotriazole and 0.21 g of dicyclohexylcarbodiimide in a procedure analogous to that described in Example 1(iv) to give, after flash chromatography on silica gel using ethyl acetate/hexane (1:4) for the elution 0.41 g of N[2]-[5(R)-[1(S)-(tert.butoxyformamido)-2-cyclohexylethyl]-2,2-dimethyl-4(R)-dioxolanyl]-2(R)-isopropylacetyl]-N[1]-phenethyl-L-isoleucinamide as a colourless glass; MS: 644 $(M+H)^+$.

## Example 49

In a manner analogous to that described in the first paragraph of Example 48, from 0.13 g of N[1]-[2-[4-(benzyloxycarbonyl)-1-piperazinyl]ethyl]-N[2]-[5(R)-[1(S)-(tert.butoxyformamido)-2-cyclohexylethyl]-2,2-dimethyl-4(R)-dioxolanyl]-2(R)-isopropylacetyl]-L-isoleucinamide there was obtained, after flash chromatography on silica gel using methanol/dichloromethane (1:32) for the elution, 18 mg of N[1]-[2-[4-(benzyloxyoxycarbonyl)-1-piperazinyl]ethyl]-$N_2$-[5(S)-(tert.butoxyformamido)-6-cyclohexyl-3(R),4(R)-dihydroxy-2(R)-isopropylhexanoyl]-L-isoleucinamide; MS: 746 $(M+H)^+$.

The N[1]-[2-[4-(benyloxycarbonyl)-1-piperazinyl]ethyl]-N[2]-[5(R)-[1(S)-(tert.butoxyformamido)-2-cyclohexylethyl]-2,2-dimethyl-4(R)-dioxolanyl]-2(R)-isopropylacetyl]-L-isoleucinamide used as the starting material was prepared as follows:

A solution of 0.10 g of 2(R)-[5(R)-[1(S)-(tert.butoxyformamido)-3-cyclohexylethyl]-2,2-dimethyl-4(R)-dioxolanyl]-3-methylbutanoic acid and in 2 ml of dimethylformamide was reacted with 96 mg of N[1]-[2-[4-(benzyloxycarbonyl)-1-piperazinyl]ethyl]-L-isoleucinamide in the presence of 40 mg of 1-hydroxybenzotriazole and 55 mg of dicyclohexylcarbodiimide in a procedure analogous to that described in Example I-(iv) to give, after flash chromatography on silica gel using ethyl acetate/hexane (1:1) for the elution, 0.13 g of N[1]-[2-[4-(benzyloxycarbonyl)-1-piperazinyl]ethyl]-N[2]-[5(R)-[1(S)-(tert.butoxyformamido)-2-cyclohexylethyl]-2,2-dimethyl-4(R)-dioxolanyl]-2(R)-isopropylacetyl]-L-isoleucinamide; MS: 786 $(M+H)^+$.

The following Example illustrates the manufacture of a pharmaceutical preparation containing a compound of formula I or a pharmaceutically acceptable acid addition salt thereof as the active ingredient:

## Example A

An aqueous solution of the active ingredient is filtered sterile and mixed while warming with a sterile gelatine solution, which contains phenol as a preserving agent, using amounts such that 1.00 ml of the resulting solution contains 3.0 mg of active ingredient, 15.0 mg of gelatine, 4.7 mg of phenol and distilled

water ad 1.0 ml. The mxiture is filled into vials of 1.0 ml capacity under aseptic conditions.

## Claims

1. Compounds of the general formula

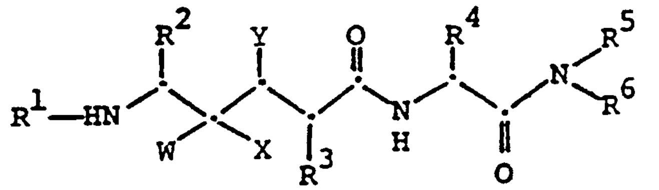

I

wherein $R^1$ represents alkoxycarbonyl, aralkoxycarbonyl, alkanoyl, aralkanoyl, aroyl, cycloalkylcarbonyl, heterocyclylcarbonyl, heterocyclyl-alkanoyl, 6-(dibenzylcarbamoyl)-4-oxohexanoyl or an acyl group of an α-amino acid in which the amino group is substituted by alkoxycarbonyl, aralkoxycarbonyl, diaralkylcarbamoyl, diaralkylalkanoyl or aralkanoyl; $R^2$ represents alkyl, cycloalkylalkyl or aralkyl; $R^3$ represents hydrogen or alkyl; $R^4$ represents alkyl; and one of $R^5$ and $R^6$ represents hydrogen and the other represents hydrogen, alkyl, aryl, aralkyl, 1-alkoxycarbonyl-2-phenylethyl, 1-alkoxycarbonyl-2-(imidazol-4-yl)ethyl, 2-(imidazol-1-yl)ethyl, indanyl, heterocyclyl-alkyl, carboxyalkyl, alkoxycarbonylalkyl, aryloxycarbonylalkyl, aralkoxycarbonylalkyl or a group of the formula -A-N($R^a$) ($R^b$) in which A represents alkylene and $R^a$ and $R^b$ each represent alkyl or $R^a$ and $R^b$ together represent a pentamethylene group in which one methylene group can be replaced by NH, N-alkyl, N-alkanoyl, N-aralkoxycarbonyl, O, S, SO or $SO_2$; or $R^5$ and $R^6$ together with the nitrogen atom to which they are attached represent a 1,2,3,4-tetrahydroisoquinoline ring; one of W and X represents hydrogen and the other represents hydroxy or amino or W and X together represent hydroxyimino and Y represents hydrogen or, where one of W and X represents hydrogen and the other represents hydroxy, Y can also represent hydroxy,
and pharmaceutically acceptable acid addition salts thereof.

2. Compounds according to claim 1, wherein $R^1$ represents alkoxycarbonyl, aralkoxycarbonyl, alkanoyl, aralkanoyl, aroyl, cycloalkylcarbonyl, heterocyclylcarbonyl, heterocyclyl-alkanoyl, 6-(dibenzylcarbamoyl)-4-oxohexanoyl or an acyl group of a natural α-amino acid in which the amino group is substituted by aralkoxycarbonyl, diaralkylcarbamoyl, diaralkylalkanoyl or aralkanoyl; one of $R^5$ and $R^6$ represents hydrogen and the other represents hydrogen, alkyl, aryl, aralkyl, 1-alkoxycarbonyl-2-phenylethyl, 1-alkoxycarbonyl-2-(imidazol-4-yl)ethyl, 2-(imidazol-1-yl)ethyl, indanyl, heterocyclyl-alkyl or a group of the formula -A-N($R^a$)($R^b$) in which A represents alkylene and $R^a$ and $R^b$ each represent alkyl or $R^a$ and $R^b$ together represent a pentamethylene group in which one methylene group can be replaced by NH, N-alkyl, N-alkanoyl, O, S, SO or $SO_2$; or $R^5$ and $R^6$ together with the nitrogen atom to which they are attached represent a 1,2,3,4-tetrahydroisoquinoline ring; one of W and X represents hydrogen and the other represents hydroxy and Y represents hydrogen; and wherein the term "aryl." used alone or in combination means a phenyl or naphthyl group which optionally carries one or more substituents selected from alkyl, alkoxy and halogen.

3. Compounds according to claim 1 or claim 2, wherein $R^1$ represents alkoxycarbonyl,. aralkoxycarbonyl, alkanoyl or aroyl or an acyl group of an α-amino acid in which the amino group is substituted by alkoxycarbonyl, aralkoxycarbonyl, diaralkylcarbamoyl, diaralkylalkanoyl or aralkyl.

4. Compounds according to claim 3, wherein $R^1$ represents tert.butoxycarbonyl, benzyloxycarbonyl, acetyl, tert.butylacetyl, 4-methylvaleryl, p-toluoyl, N-benzyloxycarbonylasparaginyl or N-benzyloxycarbonyl-ß-cyanoalanyl.

5. Compounds according to any one of claims 1 to 4, wherein $R^2$ represents isobutyl, cyclohexylmethyl or benzyl.

6. Compounds according to any one of claims 1 to 5, wherein $R^3$ represents alkyl.

7. Compounds according to claim 6, wherein $R^3$ represents methyl or isopropyl.

8. Compounds according to any one of claims 1 to 7, wherein $R^4$ represents isobutyl or sec.butyl.

9. Compounds according to any one of claims 1 to 8, wherein one of $R^5$ and $R^6$ represents hydrogen and the other represents hydrogen, alkyl, aralkyl, 1-alkoxycarbonyl-2-phenylethyl, 1-alkoxycarbonyl-2-(imidazol-4-yl)ethyl, 2-(imidazol-1-yl)ethyl, indanyl, heterocyclyl-alkyl or a group of the formula -A-N($R^a$)($R^b$)

in which A represents alkylen and $R^a$ and $R^b$ each represents alkyl or $R^a$ and $R^b$ together represent a pentamethylene group in which one methylene group can be replaced by NH, N-alkyl, N-alkanoyl, N-aralkoxycarbonyl, O, S, SO or $SO_2$; or $R^5$ and $R^6$ together represent a 1,2,3,4-tetrahydroisoquinoline ring.

10. Compounds according to claim 9, wherein one of $R^5$ and $R^6$ represents hydrogen and the other represents hydrogen, isobutyl, benzyl, 2-phenylethyl, 2-(4-hydroxyphenyl)ethyl, 1-methoxycarbonyl-2-phenylethyl, 1-methoxycarbonyl-2-(imidazol-4-yl)ethyl, 2-(imidazol-1-yl)ethyl, 2-indanyl, 2-(2-pyridyl)ethyl, 2-(dimethylamino)ethyl, 2-morpholinoethyl or 2-[4-(benzyloxycarbonyl)-1-piperazinyl]ethyl or $R^5$ and $R^6$ together with the nitrogen atom to which they are attached represent a 1,2,3,4-tetrahydroisoquinoline ring.

11. Compounds according to any one of claims 1 to 10, wherein one of W and X represents hydrogen and the other represents hydroxy and Y represents hydrogen or hydroxy.

12. Compounds according to any one of claims 1 to 11, wherein $R^1$ represents tert.butoxycarbonyl, benzyloxycarbonyl, acetyl, tert.butylacetyl, 4-methylvaleryl, p-toluoyl, N-benzyloxycarbonylasparaginyl or N-benzyloxycarbonyl-ß-cyanoalanyl, $R^2$ represents isobutyl, cyclohexylmethyl or benzyl, $R^3$ represents methyl or isopropyl, $R^4$ represents isobutyl or sec.butyl and one of $R^5$ and $R^6$ represents hydrogen and the other represents hydrogen, isobutyl, benzyl, 2-phenylethyl, 2-(4-hydroxyphenyl)ethyl, 1-methoxycarbonyl-2-phenylethyl, 1-methoxycarbonyl-2-(imidazol-4-yl)ethyl, 2-(imidazol-1-yl)ethyl, 2-indanyl, 2-(2-pyridyl)ethyl, 2-(dimethylamino)ethyl, 2-morpholinoethyl or 2-[4-(benzyloxycarbonyl)-1-piperazinyl]ethyl or $R^5$ and $R^6$ together with the nitrogen atom to which they are attached represent a 1,2,3,4-tetrahydroisoquinoline ring and one of W and X represents hydrogen and the other represents hydroxy and Y represents hdyrogen or hydroxy.

13. $N^2$-[5(S)-(tert.Butoxyformamido)-6-cyclohexyl-4(S)-hydroxy-2(S)-isopropylhexanoyl]-$N^1$-phenethyl-L-isoleucinamide.

14. N-[N-[5(S)-(tert.Butoxyformamido)-6-cyclohexyl-4(S)-hydroxy-2(S)-isopropylhexanoyl]-L-isoleucyl]-L-phenylalanine methyl ester.

15. $N^2$-[5(S)-(tert.Butoxyformamido)-6-cyclohexyl-4(S)-hydroxy-2(S)-isopropylhexanoyl]-$N^1$-benzyl-L-isoleucinamide.

16. $N^2$-[5(S)-(tert.Butoxyformamido)-6-cyclohexyl-4(S)-hydroxy-2(S)-isopropylhexanoyl]-$N^1$-[2-(2-pyridyl)-ethyl]-L-isoleucinamide.

17. $N^2$-[5(S)-(tert.Butoxyformamido)-6-cyclohexyl-4(S)-hydroxy-2(S)-isopropylhexanoyl]-$N^1$-(2-indanyl)-L-isoleucinamide.

18. $N^2$-[5(S)-(tert.Butoxyformamido)-6-cyclohexyl-4(S)-hydroxy-2(S)-isopropylhexanoyl]-$N^1$-(2-morpholinoethyl)-L-isoleucinamide.

19. $N^2$-[5(S)-(tert.Butoxyformamido)-4(S)-hydroxy-2(S)-isopropyl-6-phenylhexanoyl]-$N^1$-phenethyl-L-isoleucinamide.

20. $N^2$-[5(S)-Benzyloxyformamido)-6-cyclohexyl-4(S)-hydroxy-2(S)-isopropylhexanoyl]-$N^1$-phenethyl-L-isoleucinamide.

21. $N^2$-[5(S)-[[N-(Benzyloxycarbonyl)-L-asparaginyl]amino]-6-cyclohexyl-4(S)-hydroxy-2(S)-isopropylhexanoyl]-$N^1$-phenethyl-L-isoleucinamide.

22. $N^2$-[5(S)-(tert.Butylacetamido)-6-cyclohexyl-4(S)-hydroxy-2(S)-isopropylhexanoyl]-$N^1$-phenethyl-L-isoleucinamide.

23. $N^2$-[5(S)-[[N-(Benzyloxycarbonyl)-ß-cyano-L-alanyl]amino]-6-cyclohexyl-4(S)-hydroxy-2(S)-isopropylhexanoyl]-$N^1$-phenethyl-L-isoleucinamide.

24. $N^1$-[2-[4-(Benzyloxycarbonyl)-1-piperazinyl]ethyl]-$N^2$-[5(S)-(tert.butoxyformamido)-6-cyclohexyl-4(S)-hydroxy-2(S)-isopropylhexanoyl]-L-isoleucinamide.

25. $N^2$-[5(S)-(tert.Butoxyformamido)-6-cyclohexyl-4(S)-hydroxy-2(S)-isopropylhexanoyl]-$N^1$-[2-(4-hydroxyphenyl)ethyl]-L-isoleucinamide.

26. $N^1$-[2-[4-(Benzyloxycarbonyl)-1-piperazinyl]ethyl]-$N^2$-[5(S)-(tert.butoxyformamido)-6-cyclohexyl-3(R)-,4(R)-dihydroxy-2(R)-isopropylhexanoyl]-L-isoleucinamide.

27. Compounds of the general formula

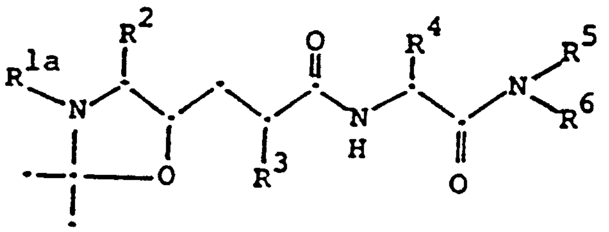

II

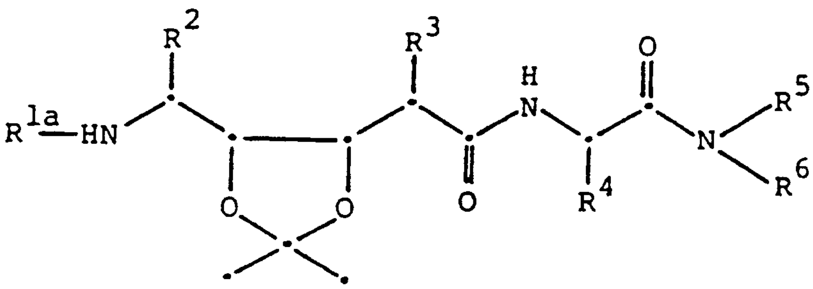

III

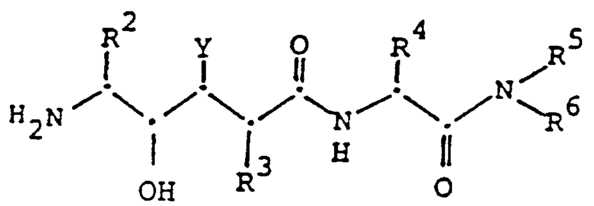

IV

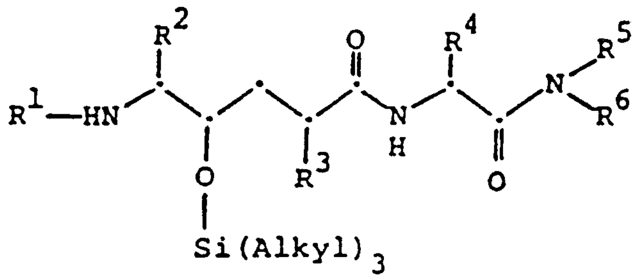

V

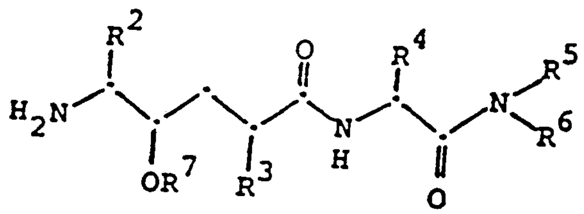

VI

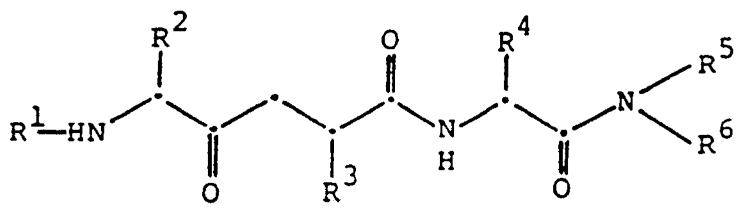

VII

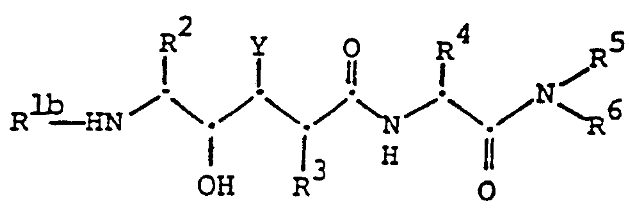

VIII

wherein $R^1$ represents alkoxycarbonyl, aralkoxycarbonyl, alkanoyl, aralkanoyl, aroyl, cycloalkylcar bonyl, heterocyclylcarbonyl, heterocyclyl-alkanoyl, 6-(dibenzylcarbamoyl)-4-oxohexanoyl or an acyl group of an α-amino acid in which the amino group is substituted by alkoxycarbonyl, aralkoxycarbonyl, diaralkylcarbamoyl, diaralkylalkanoyl or aralkanoyl; $R^{1a}$ represents alkoxycarbonyl or aralkoxycarbonyl, $R^{1b}$ represents 3-(aralkoxymethyl)-N-(diarylcarbamoyl or aralkanoyl)-histidyl; $R^2$ represents alkyl, cycloalkylalkyl or aralkyl; $R^3$ represents hydrogen or alkyl; $R^4$ represents alkyl; and one of $R^5$ and $R^6$ represents hydrogen and the other represents hydrogen, alkyl, aryl, aralkyl, 1-alkoxycarbonyl-2-phenylethyl, 1-alkoxycarbonyl-2-(imidazol-4-yl)ethyl, 2-(imidazol-1-yl)ethyl, indanyl, heterocyclyl-alkyl, carboxyalkyl, alkoxycarbonylalkyl, aryloxycarbonylalkyl, aralkoxycarbonylalkyl or a group of the formula -A-N($R^a$)($R^b$) in which A represents alkylene and $R^a$ and $R^b$ each represent alkyl or $R^a$ and $R^b$ together represent a pentamethylene group in which one methylene group can be replaced by NH, N-alkyl, N-alkanoyl, N-aralkoxycarbonyl, O, S, SO or $SO_2$; or $R^5$ and $R^6$ together with the nitrogen atom to which they are attached represent a 1,2,3,4-tetrahydroisoquinoline ring, $R^7$ represents alkanoyl and Y represents hydrogen or hydroxy.

28. A compound according to any one of claims 1 to 26 or a pharmaceutically acceptable acid addition salt thereof for use as a therapeutically active substance.

29. A compound according to any one of claims 1 to 26 or a pharmaceutically acceptable acid addition salt thereof for use as a therapeutically active substance in the treatment or prophylaxis of viral infections, particularly of retroviral infections and especially of HIV infections.

30. A process for the manufacture of a compound in accordance with in any one of claims 1 to 26, which process comprises

(a) for the manufacture of a compound of formula I in which $R^1$ represents alkoxycarbonyl or aralkoxycarbonyl, one of W and X represents hydrogen and the other represents hydroxy and Y represents hydrogen or hydroxy, treating a compound of the general formula

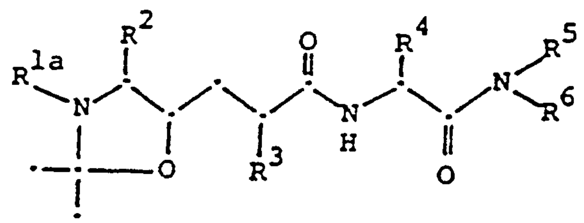

II

or

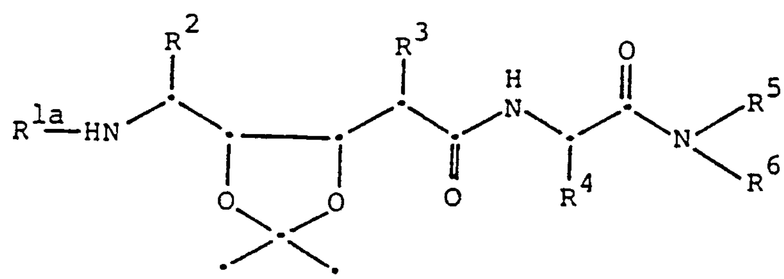

III

wherein $R^{1a}$ represents alkoxycarbonyl or aralkoxycarbonyl and $R^2$, $R^3$, $R^4$, $R^5$ and $R^6$ have the significance given in claim 1,
with an acid, or

(b) for the manufacture of a compound of formula I in which one of W and X represents hydrogen and the other represents hydroxy and Y represents hydrogen or hydroxy, reacting a compound of the general formula

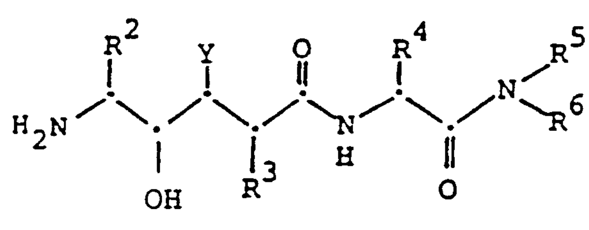

IV

wherein $R^2$, $R^3$, $R^4$, $R^5$, $R^6$ and Y have the significance given in claim 1,
with an acylating agent which introduces a group $R^1$ as defined earlier, or

(c) for the manufacture of a compound of formula I in which one of W and X represents hydrogen and the other represents hydroxy and Y represents hydrogen, cleaving off the trialkylsilyl protecting group form a compound of the general formula

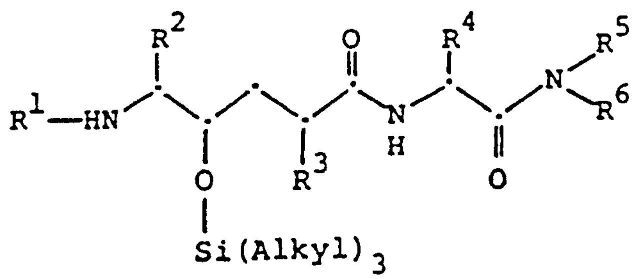

V

wherein $R^1$, $R^2$, $R^3$, $R^4$, $R^5$ and $R^6$ have the significance given in claim 1,
or

(d) for the manufacture of a compound of formula I in which $R^1$ represents alkanoyl, one of W and X represents hydrogen and the other represents hydroxy and Y represents hydrogen, treating a compound of the general formula

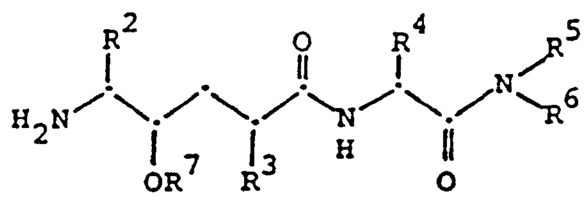

VI

wherein, $R^2$, $R^3$, $R^4$, $R^5$ and $R^6$ have the significance given in claim 1 and $R^7$ represents alkanoyl, with a base, or

(e) for the manufacture of a compound of formula I in which W and X together represent hydroxyimino and Y represents hydrogen, reacting a compound of the general formula

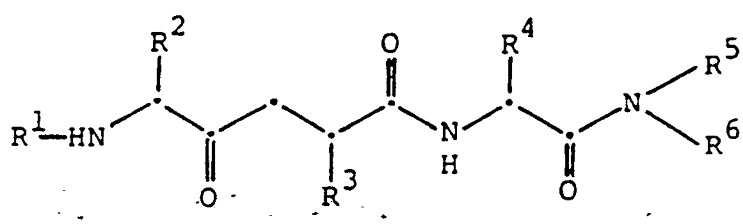

VII

wherein $R^1$, $R^2$, $R^3$, $R^4$, $R^5$ and $R^6$ have the significance given in claim 1,
with hydroxylamine, or

(f) for the manufacture of a compound of formula I in which one of W and X represents hydrogen and the other represents amino and Y represents hydrogen, reducing a compound of formula I in which W and X together represents hydroxyimino and Y represents hydrogen, or

(g) for the manufacture of a compound of formula I in which $R^1$ represents N-(diaralkylcarbamoyl or aralkanoyl)-histidyl, one of $R^5$ and $R^6$ represents hydrogen and the other represents hydrogen, alkyl, aryl, aralkyl, 1-alkoxycarbonyl-2-phenylethyl, 1-alkoxycarbonyl-2-(imidazol-4-yl)ethyl, 2-(imidazol-1-yl)ethyl, indanyl, heterocyclyl-alkyl, carboxyalkyl, alkoxycarbonylalkyl, aryloxycarbonylalkyl or a group of the formula $-A-N(R^a)(R^b)$ in which A represents alkylene and $R^a$ and $R^b$ each represent alkyl or $R^a$ and $R^b$ together represent a pentamethylene group in which one methylene group can be replaced by NH, N-alkyl, N-alkanoyl, O, S, SO or $SO_2$ or $R^5$ and $R^6$ together with the nitrogen atom to which they are attached represents a 1,2,3,4-tetrahydroisoquinoline ring, one of W and X represents hydrogen and the other

represents hydroxy and Y represents hydrogen or hydroxy, hydrogenolyzing a compound of the general formula

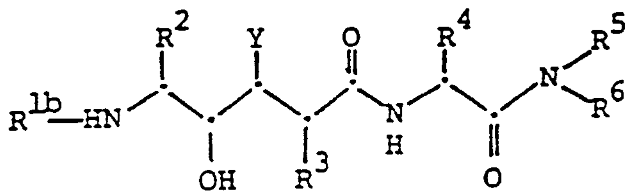

VIII

wherein $R^{1b}$ represents 3-(aralkoxymethyl)-N-(diaralkylcarbamoyl or aralkanoyl)-histidyl and $R^2$, $R^3$, $R^4$, $R^5$, $R^6$ and Y have the significance given in claim 1,

or

(h) for the manufacture of a compound of formula I in which $R^1$ represents alkoxycarbonyl, alkanoyl, aralkanoyl, aroyl, cycloalkylcarbonyl, heterocyclylcarbonyl, heterocyclyl-alkanoyl, 6-(dibenzylcarbamoyl)-4-oxohexanoyl or an acyl group of an $\alpha$-amino acid in which the amino group is substituted by alkoxycarbonyl, diaralkylcarbamoyl, diaralkylalkanoyl or aralkanoyl and one of $R^5$ and $R^6$ represents hydrogen and the other represents carboxyalkyl or a group of the formula -A-N($R^a$)($R^b$) in which $R^a$ and $R^b$ together represent a pentamethylene group in which one methylene group is replaced by NH, hydrogenolyzing a corresponding compound of formula I in which one of $R^5$ and $R^6$ represents hydrogen and the other represents aralkoxycarbonylalkyl or a group of the formula -A-N($R^a$)($R^b$) in which $R^a$ and $R^b$ together represent a pentamethylene group in which one methylene group is replaced by N-aralkoxycarbonyl, and

(i) if desired, converting a compound of formula I obtained into a pharmaceutically acceptable acid addition salt.

31. A process according to claim 30 wherein compound of formula I wherein $R^1$ represents alkoxycarbonyl, aralkoxycarbonyl, alkanoyl, aralkanoyl, aroyl, cycloalkylcarbonyl, heterocyclylcarbonyl, heterocyclyl-alkanoyl, 6-(dibenzylcarbamoyl)-4-oxohexanoyl or an acyl group of a natural $\alpha$-amino acid in which the amino group is substituted by aralkoxycarbonyl, diaralkylcarbamoyl, diaralkylalkanoyl or aralkanoyl; one of $R^5$ and $R^6$ represents hydrogen and the other represents hydrogen, alkyl, aryl, aralkyl, 1-alkoxycarbonyl-2-phenylethyl, 1-alkoxycarbonyl-2-(imidazol-4-yl)ethyl, 2-(imidazol-1-yl)ethyl, indanyl, heterocyclyl-alkyl or a group of the formula -A-N($R^a$)($R^b$) in which A represents alkylene and $R^a$ and $R^b$ each represent alkyl or $R^a$ and $R^b$ together represent a pentamethylene group in which one methylene group can be replaced by NH, N-alkyl, N-alkanoyl, O, S, SO or $SO_2$; or $R^5$ and $R^6$ together with the nitrogen atom to which they are attached represent a 1,2,3,4-tetrahydroisoquinoline ring; one of W and X represents hydrogen and the other represents hydroxy and Y represents hydrogen; and wherein the term "aryl" used alone or in combination means a phenyl or naphthyl group which optionally carries one or more substituents selected from alkyl, alkoxy and halogen; and their pharmaceutically acceptable acid addition salts are manufactured according to process embodiments (a), (b), (c), (d), (g) and/or (i).

32. A medicament containing a compound according to any one of claims 1 to 26 or a pharmaceutically acceptable acid addition salt thereof and a therapeutically inert excipient.

33. A medicament for the treatment or prophylaxis of viral infections, particularly of HIV infections, containing a compound according to any one of claims 1 to 26 or a pharmaceutically acceptable acid addition salt thereof and a therapeutically inert excipient.

34. The use of a compound according to any one of claims 1 to 26 or a pharmaceutically acceptable acid addition salt thereof for the manufacture of a medicament for the treatment or prophylaxis of viral infections, particularly of HIV infections.

Claims for the following Contracting State: ES

1. A process for the manufacture of compounds of the general formula

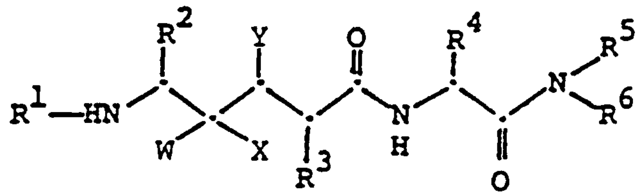

I

wherein $R^1$ represents alkoxycarbonyl, aralkoxycarbonyl, alkanoyl, aralkanoyl, aroyl, cycloalkylcarbonyl, heterocyclylcarbonyl, heterocyclyl-alkanoyl, 6-(dibenzylcarbamoyl)-4-oxohexanoyl or an acyl group of an α-amino acid in which the amino group is substituted by alkoxycarbonyl, aralkoxycarbonyl, diaralkylcarbamoyl, diaralkylalkanoyl or aralkanoyl; $R^2$ represents alkyl, cycloalkylalkyl or aralkyl; $R^3$ represents hydrogen or alkyl; $R^4$ represents alkyl; and one of $R^5$ and $R^6$ represents hydrogen and the other represents hydrogen, alkyl, aryl, aralkyl, 1-alkoxycarbonyl-2-phenylethyl, 1-alkoxycarbonyl-2-(imidazol-4-yl)ethyl, 2-(imidazol-1-yl)ethyl, indanyl, heterocyclyl-alkyl, carboxyalky, alkoxycarbonylalkyl, aryloxycarbonylalkyl, aralkoxycarbonylalkyl or a group of the formula -A-N($R^a$)($R^b$) in which A represents alkylene and $R^a$ and $R^b$ each represent alkyl or $R^a$ and $R^b$ together represent a pentamethylene group in which one methylene group can be replaced by NH, N-alkyl, N-alkanoyl, N-aralkoxycarbonyl, O, S, SO or $SO_2$; or $R^5$ and $R^6$ together with the nitrogen atom to which they are attached represent a 1,2,3,4-tetrahydroisoquinoline ring; one of W and X represents hydrogen and the other represents hydroxy or amino or W and X together represent hydroxyimino and Y represents hydrogen or, where one of W and X represents hydrogen and the other represents hydroxy, Y can also represent hydroxy,
and pharmaceutically acceptable acid addition salts thereof, which process comprises

(a) for the manufacture of a compound of formula I in which $R^1$ represents alkoxycarbonyl or aralkoxycarbonyl, one of W and X represents hydrogen and the other represents hydroxy and Y represents hydrogen or hydroxy, treating a compound of the general formula

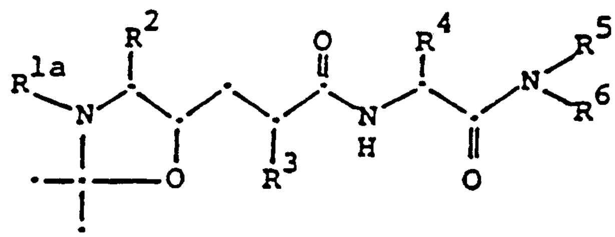

II

or

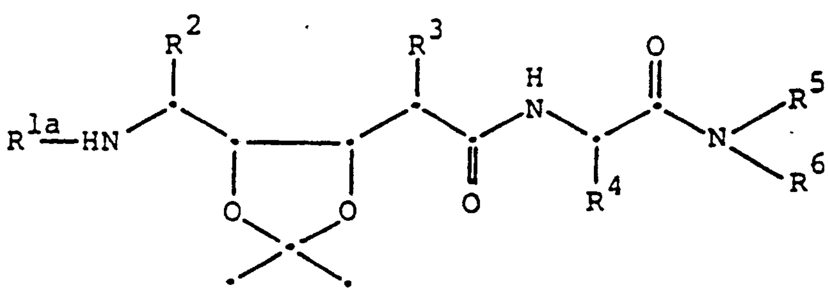

III

wherein $R^{1a}$ represents alkoxycarbonyl or aralkoxycarbonyl and $R^2$, $R^3$, $R^4$, $R^5$ and $R^6$ have the significance given earlier,
with an acid, or

(b) for the manufacture of a compound of formula I in which one of W and X represents hydrogen and the other represents hydroxy and Y represents hydrogen or hydroxy, reacting a compound of the general formula

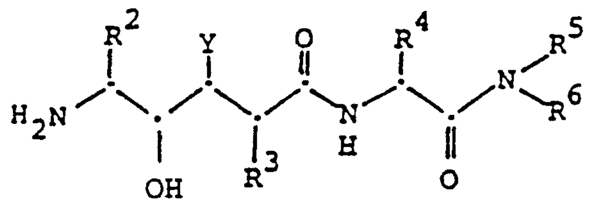

IV

wherein $R^2$, $R^3$, $R^4$, $R^5$, $R^6$ and Y have the significance given earlier in this claim, with an acylating agent which introduces a group $R^1$ as defined earlier, or

(c) for the manufacture of a compound of formula I in which one of W and X represents hydrogen and the other represents hydroxy and Y represents hydrogen, cleaving off the trialkylsilyl protecting group form a compound of the general formula

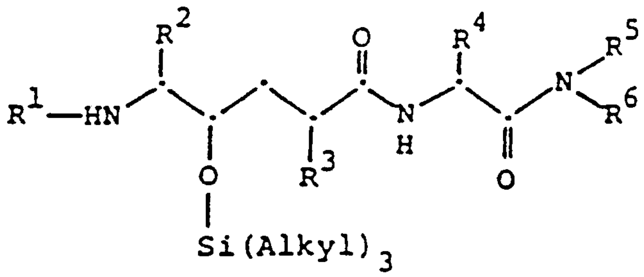

V

wherein $R^1$, $R^2$, $R^3$, $R^4$, $R^5$ and $R^6$ have the significance given earlier in this claim, or

(d) for the manufacture of a compound of formula I in which $R^1$ represents alkanoyl, one of W and X represents hydrogen and the other represents hydroxy and Y represents hydrogen, treating a compound of the general formula

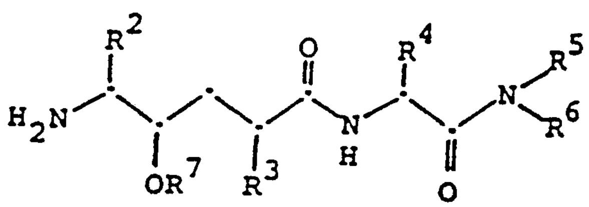

VI

wherein $R^2$, $R^3$, $R^4$, $R^5$ and $R^6$ have the significance given earlier in this claim and $R^7$ represents alkanoyl, with a base, or

(e) for the manufacture of a compound of formula I in which W and X together represent hydroxyimino and Y represents hydrogen, reacting a compound of the general formula

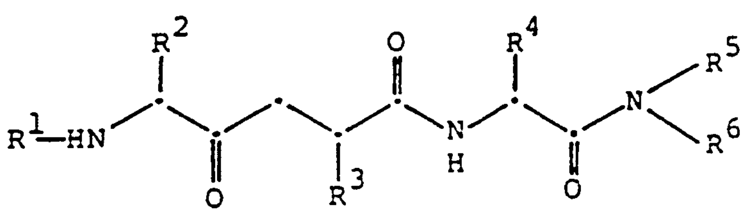

VII

wherein $R^1$, $R^2$, $R^3$, $R^4$, $R^5$ and $R^6$ have the significance given earlier in this claim, with hydroxylamine, or

(f) for the manufacture of a compound of formula I in which one of W and X represents hydrogen and

the other represents amino and Y represents hydrogen, reducing a compound of formula I in which W and X together represent hydroxyimino and Y represents hydrogen, or

(g) for the manufacture of a compound of formula I in which $R^1$ represents N-(diaralkylcarbamoyl or aralkanoyl)-histidyl, one of $R^5$ and $R^6$ represents hydrogen and the other represents hydrogen, alkyl, aryl, aralkyl, 1-alkoxycarbonyl-2-phenylethyl, 1-alkoxycarbonyl-2-(imidazol-4-yl)ethyl, 2-(imidazol-1-yl)ethyl), indanyl, heterocyclyl-alkyl, carboxyalkyl, alkoxycarbonylalkyl, aryloxycarbonylalkyl or a group of the formula -A-N($R^a$)($R^b$) in which A represents alkylene and $R^a$ and $R^b$ each represent alkyl or $R^a$ and $R^b$ together represent a pentamethylene group in which one methylene group can be replaced by NH, N-alkyl, N-alkanoyl, O, S, SO or $SO_2$ or $R^5$ and $R^6$ together with the nitrogen atom to which they are attached represents a 1,2,3,4-tetrahydroisoquinoline ring, one of W and X represents hydrogen and the other represents hydroxy and Y represents hydrogen or hydroxy, hydrogenolyzing a compound of the general formula

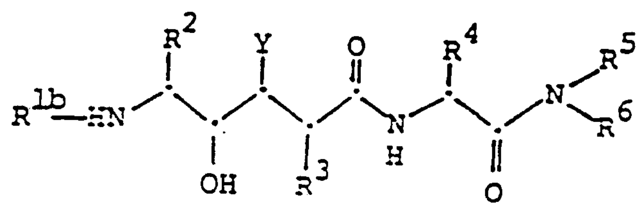

VIII

wherein $R^{1b}$ represents 3-(aralkoxymethyl)-N-(diaralkylcarbamoyl or aralkanoyl)-histidyl and $R^2$, $R^3$, $R^4$, $R^5$, $R^6$ and Y have the significance given earlier in this claim,

or

(h) for the manufacture of a compound of formula I in which $R^1$ represents alkoxycarbonyl, alkanoyl, aralkanoyl, aroyl, cycloalkylcarbonyl, heterocyclylcarbonyl, heterocyclyl-alkanoyl, 6-(dibenzylcarbamoyl)-4-oxohexanoyl or an acyl group of an α-amino acid in which the amino group is substituted by alkoxycarbonyl, diaralkylcarbamoyl, diaralkylalkanoyl or aralkanoyl and one of $R^5$ and $R^6$ represents hydrogen and the other represents carboxyalkyl or a group of the formula -A-N($R^a$)($R^b$) in which $R^a$ and $R^b$ together represent a pentamethylene group in which one methylene group is replaced by NH, hydrogenolyzing a corresponding compound of formula I which one of $R^5$ and $R^6$ represents hydrogen and the other represents aralkoxycarbonylalkyl or a group of the formula -A-N($R^a$)($R^b$) in which $R^a$ and $R^b$ together represent a pentamethylene group in which one methylene group is replaced by N-aralkoxycarbonyl, and

(i) if desired, converting a compound of formula I obtained into a pharmaceutically acceptable acid addition salt.

2. A process according to claim 1, wherein compounds of formula I wherein $R^1$ represents alkoxycarbonyl, aralkoxycarbonyl, alkanoyl, aralkanoyl, aroyl, cycloalkylcarbonyl, heterocyclylcarbonyl, heterocyclyl-alkanoyl, 6-(dibenzylcarbamoyl)-4-oxohexanoyl or an acyl group of a natural α-amino acid in which the amino group is substituted by aralkoxycarbonyl, diaralkylcarbamoyl, diaralkylalkanoyl or aralkanoyl; one of $R^5$ and $R^6$ represents hydrogen and the other represents hydrogen, alkyl, aryl, aralkyl, 1-alkoxycarbonyl-2-phenylethyl, 1-alkoxycarbonyl-2-(imidazol-4-yl)ethyl, 2-(imidazol-1-yl)ethyl, indanyl, heterocyclyl-alkyl or a group of the formula -A-N($R^a$)($R^b$) in which A represents alkylene and $R^a$ and $R^b$ each represent alkyl or $R^a$ and $R^b$ together represent a pentamethylene group in which one methylene group can be replaced by NH, N-alkyl, N-alkanoyl, O, S, SO or $SO_2$; or $R^5$ and $R^6$ together with the nitrogen atom to which they are attached represent a 1,2,3,4-tetrahydroisoquinoline ring; one of W and X represents hydrogen and the other represents hydroxy and Y represents hydrogen; and wherein the term "aryl" used alone or in combination means a phenyl or naphthyl group which optionally carries one or more substituents selected from alkyl, alkoxy and halogen; and their pharmaceutically acceptable acid addition salts are manufactured according to process embodiments (a), (b), (c), (d), (g) and/or (i).

3. A process according to claim 1 or claim 2, wherein $R^1$ represents alkoxycarbonyl, aralkoxycarbonyl, alkanoyl or aroyl or an acyl group of an α-amino acid in which the amino group is substituted by alkoxycarbonyl, aralkoxycarbonyl, diaralkylcarbamoyl, diaralkylalkanoyl or aralkyl.

4. A process according to claim 3, wherein $R^1$ represents tert.butoxycarbonyl, benzyloxycarbonyl, acetyl, tert.butylacetyl, 4-methylvaleryl, p-toluoyl, N-benzyloxycarbonylasparaginyl or N-benzyloxycarbonyl-ß-cyanoalanyl.

5. A process according to any one of claims 1 to 4, wherein $R^2$ represents isobutyl, cyclohexylmethyl or benzyl.

6. A process according to any one of claims 1 to 5, wherein $R^3$ represents alkyl.

7. A process according to claim 6, wherein $R^3$ represents methyl or isopropyl.

8. A process according to any one of claims 1 to 7, wherein $R^4$ represents isobutyl or sec.butyl.

9. A process according to any one of claims 1 to 8, wherein one of $R^5$ and $R^6$ represents hydrogen and the other represents hydrogen, alkyl, aralkyl, 1-alkoxy carbonyl-2-phenylethyl, 1-alkoxycarbonyl-2-(imidazol-4-yl)ethyl, 2-(imidazol-1-yl)ethyl, indanyl, heterocyclylalkyl or a group of the formula -A-N($R^a$) ($R^b$) in which A represents alkylene and $R^a$ and $R^b$ each represent alkyl or $R^a$ and $R^b$ together represent a pentamethylene group in which one methylene group can be replaced by NH, N-alkyl, N-alkanoyl, N-aralkoxycarbonyl, O, S, SO or $SO_2$; or $R^5$ and $R^6$ together represent a 1,2,3,4-tetrahydroisoquinoline ring.

10. A process according to claim 9, wherein one of $R^5$ and $R^6$ represents hydrogen and the other represents hydrogen, isobutyl, benzyl, 2-phenylethyl, 2-(4-hydroxyphenyl)ethyl, 1-methoxycarbonyl-2-phenylethyl, 1-methoxycarbonyl-2-(imidazol-4-yl)ethyl, 2-(imidazol-1-yl)ethyl, 2-indanyl, 2-(2-pyridyl)ethyl, 2-(dimethylamino)ethyl, 2-morpholinoethyl or 2-[4-(benzyloxycarbonyl)-1-piperazinyl]ethyl or $R^5$ and $R^6$ together with the nitrogen atom to which they are attached represent a 1,2,3,4-tetrahydroisoquinoline ring.

11. A process according to any one of claims 1 to 10, wherein one of W and X represents hydrogen and the other represents hydroxy and Y represents hydrogen or hydroxy.

12. A process according to any one of claims 1 to 11, wherein $R^1$ represetns tert.butoxycarbonyl, benzyloxycarbonyl, acetyl, tert.butylacetyl, 4-methylvaleryl, p-toluoyl, N-benzyloxycarbonylasparaginyl or N-benzyloxycarbonyl-ß-cyanoalanyl, $R^2$ represents isobutyl, cyclohexylmethyl or benzyl, $R^3$ represents methyl or isopropyl, $R^4$ represents isobutyl or sec.butyl and one of $R^5$ and $R^6$ represents hydrogen and the other represents hydrogen, isobutyl, benzyl, 2-phenylethyl, 2-(4-hydroxyphenyl)ethyl, 1-methoxycarbonyl-2-phenylethyl, 1-methoxycarbonyl-2-(imidazol-4-yl)ethyl, 2-(imidazol-1-yl)ethyl, 2-indanyl, 2-(2-pyridyl)ethyl, 2-(dimethyl amino)ethyl, 2-morpholinoethyl of 2-[4-(benzyloxycarbonyl)-1-piperazinyl]ethyl or $R^5$ and $R^6$ together with the nitrogen atom to which they are attached represent a 1,2,3,4-tetrahydroisoquinoline ring and one of W and X represents hydrogen and the other represents hydroxy and Y represents hydrogen or hydroxy.

13. A process according to claim 2, wherein $N^2$-[5(S)-(tert.butoxyformamido)-6-cyclohexyl-4(S)-hydroxy-2(S)-isopropylhexanoyl]-$N^1$-phenethyl-L-isoleucinamide is prepared.

14. A process according to claim 2, wherein N-[N-[5(S)-(tert.butoxyformamido)-6-cyclohexyl-4(S)-hydroxy-2(S)-isopropylhexanoyl]-L-isoleucyl]-L-phenylalanine methyl ester is prepared.

15. A process according to claim 2, wherein $N^2$-[5(S)-(tert.butoxyformamido)-6-cyclohexyl-4(S)-hydroxy-2(S)-isopropylhexanoyl]-$N^1$-benzyl-L-isoleucinamide is prepared.

16. A process according to claim 2, wherein $N^2$-[5(S)-(tert.butoxyformamido)-6-cyclohexyl-4(S)-hydroxy-2(S)-isopropylhexanoyl]-$N^1$-[2-(2-pyridyl)ethyl]-L-isoleucinamide is prepared.

17. A process according to claim 2, wherein $N^2$-[5(S)-(tert.butoxyformamido)-6-cyclohexyl-4(S)-hydroxy-2(S)-isopropylhexanoyl]-$N^1$-(2-indanyl)-L-isoleucinamide is prepared.

18. A process according to claim 2, wherein $N^2$-[5(S)-(tert.butoxyformamido)-6-cyclohexyl-4(S)-hydroxy-2(S)-isopropylhexanoyl]-$N^1$-(2-morpholinoethyl)-L-isoleucinamide is prepared.

19. A process according to claim 2, wherein $N^2$-[5(S)-(tert.butoxyformamido)-6-cyclohexyl-2(S)-isopropyl-6-phenylhexanoyl]-$N^1$-phenethyl-L-isoleucinamide is prepared.

20. A process according to claim 2, wherein $N^2$-[5(S)-benzyloxyformamido-6-cyclohexyl-4(S)-hydroxy-2-(S)-isopropylhexanoyl]-$N^1$-phenethyl-L-isoleucinamide is prepared.

21. A process according to claim 2, wherein $N^2$-[5(S)-[[N-(benzyloxycarbonyl)-L-asparaginyl]amino]-6-cyclohexyl-4(S)-hydroxy-2(S)-isopropylhexanoyl]-$N^1$-phenethyl-L-isoleucinamide is prepared.

22. A process according to claim 2, wherein $N^2$-[5(S)-(tert.butylacetamido)-6-cyclohexyl-4(S)-hydroxy-2-(S)-isopropylhexanoyl]-$N^1$-phenethyl-L-isoleucinamide is prepared.

23. A process according to claim 1, wherein $N^2$-[5(S)-[[N-(benzyloxycarbonyl)-ß-cyano-L-alanyl]amino]-6-cyclohexyl-4(S)-hydroxy-2(S)-isopropylhexanoyl]-$N^1$-phenethyl-L-isoleucinamide is prepared.

24. A process according to claim 1, wherein $N^1$-[2-[4-(benzyloxycarbonyl)-1-piperazinyl]ethyl]-$N^2$-[5(S)-(tert.butoxyformamido)-6-cyclohexyl-4(S)-hydroxy-2(S)-isopropylhexanoyl]-L-isoleucinamide is prepared.

25. A process according to claim 1, wherein $N^2$-[5(S)-(tert.butoxyformamido)-6-cyclohexyl-4(S)-hydroxy-2(S)-isopropylhexanoyl]-$N^1$-[2-(4hydroxyphenyl)ethyl]-L-isoleucinamide is prepared.

26. A process according to claim 1, wherein $N^1$-[2-[4-(benzyloxycarbonyl)-1-piperazinyl]ethyl]-$N^2$-[5(S)-(tert.butoxyformamido)-6-cyclohexyl-3(R),4(R)-dihydroxy-2(R)-isopropylhexanoyl]-L-isoleucinamide is prepared.

27. A process for the manufacture of a medicament, particularly to be used in the treatment or prophylaxis of viral infections, which process comprises bringing a compound of formula I set forth in claim 1 or a pharmaceutically acceptable acid addition salt thereof into a galenical dosage form.

28. The use of a compound of formula I set forth in claim 1 or a pharmaceutically acceptable acid addition salt thereof for the manufacture of a medicament for the treatment or prophylaxis of viral infections.

Claims for the following Contracting State: GR

1. A process for the manufacture of compounds of the general formula

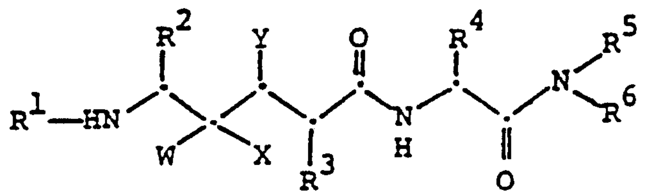

I

wherein $R^1$ represents alkoxycarbonyl, aralkoxycarbonyl, alkanoyl, aralkanoyl, aroyl, cycloalkylcarbonyl, heterocyclylcarbonyl, heterocyclyl-alkanoyl, 6-(dibenzylcarbamoyl)-4-oxohexanoyl or an acyl group of an α-amino acid in which the amino group is substituted by alkoxycarbonyl, aralkoxycarbonyl, diaralkylcarbamoyl, diaralkylalkanoyl or aralkanoyl; $R^2$ represents alkyl, cycloalkylalkyl or aralkyl; $R^3$ represents hydrogen or alkyl; $R^4$ represents alkyl; and one of $R^5$ and $R^6$ represents hydrogen and the other represents hydrogen, alkyl, aryl, aralkyl, 1-alkoxycarbonyl-2-phenylethyl, 1-alkoxycarbonyl-2-(imidazol-4-yl)ethyl, 2-(imidazol-1-yl)ethyl, indanyl, heterocyclyl-alkyl, carboxyalky, alkoxycarbonylalkyl, aryloxycarbonylalkyl, aralkoxycarbonylalkyl or a group of the formula -A-N($R^a$)($R^b$) in which A represents alkylene and $R^a$ and $R^b$ each represent alkyl or $R^a$ and $R^b$ together represent a pentamethylene group in which one methylene group can be replaced by NH, N-alkyl, N-alkanoyl, N-aralkoxycarbonyl, O, S, SO or $SO_2$; or $R^5$ and $R^6$ together with the nitrogen atom to which they are attached represent a 1,2,3,4-tetrahydroisoquinoline ring; one of W and X represents hydrogen and the other represents hydroxy or amino or W and X together represent hydroxyimino and Y represents hydrogen or, where one of W and X represents hydrogen and the other represents hydroxy, Y can also represent hydroxy,
and pharmaceutically acceptable acid addition salts thereof, which process comprises

(a) for the manufacture of a compound of formula I in which $R^1$ represents alkoxycarbonyl or aralkoxycarbonyl, one of W and X represents hydrogen and the other represents hydroxy and Y represents hydrogen or hydroxy, treating a compound of the general formula

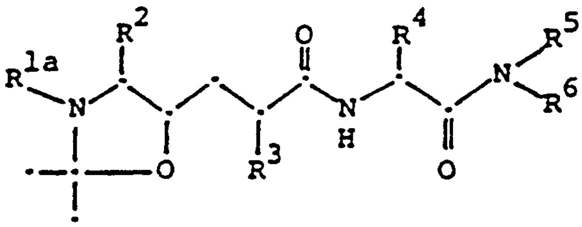

II

or

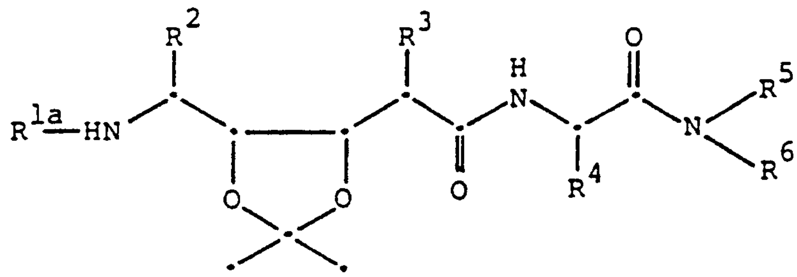

III

wherein $R^{1a}$ represents alkoxycarbonyl or aralkoxycarbonyl and $R^2$, $R^3$, $R^4$, $R^5$ and $R^6$ have the significance given earlier,
with an acid, or

(b) for the manufacture of a compound of formula I in which one of W and X represents hydrogen and the other represents hydroxy and Y represents hydrogen or hydroxy, reacting a compound of the general formula

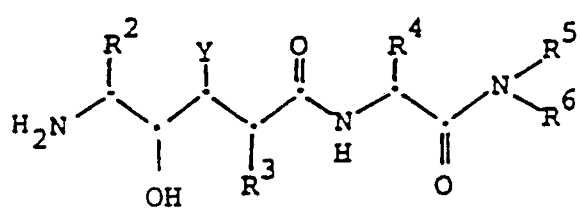

IV

wherein $R^2$, $R^3$, $R^4$, $R^5$, $R^6$ and Y have the significance given earlier in this claim,
with an acylating agent which introduces a group $R^1$ as defined earlier, or

(c) for the manufacture of a compound of formula I in which one of W and X represents hydrogen and the other represents hydroxy and Y represents hydrogen, cleaving off the trialkylsilyl protecting group form a compound of the general formula

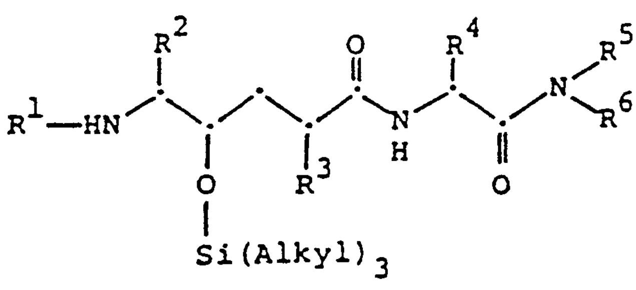

V

wherein $R^1$, $R^2$, $R^3$, $R^4$, $R^5$ and $R^6$ have the significance given earlier in this claim,

or

(d) for the manufacture of a compound of formula I in which $R^1$ represents alkanoyl, one of W and X represents hydrogen and the other represents hydroxy and Y represents hydrogen, treating a compound of the general formula

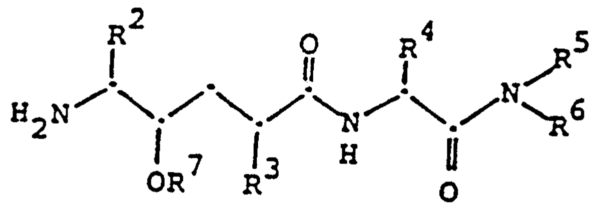

VI

wherein $R^2$, $R^3$, $R^4$, $R^5$ and $R^6$ have the significance given earlier in this claim and $R^7$ represents alkanoyl, with a base, or

(e) for the manufacture of a compound of formula I in which W and X together represent hydroxyimino and Y represents hydrogen, reacting a compound of the general formula

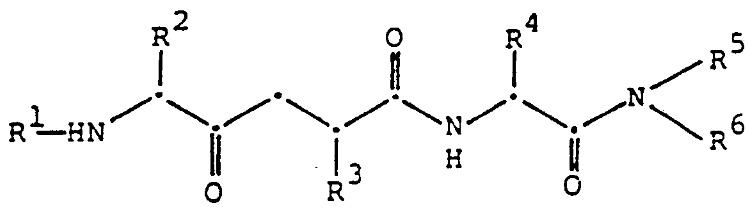

VII

wherein $R^1$, $R^2$, $R^3$, $R^4$, $R^5$ and $R^6$ have the significance given earlier in this claim, with hydroxylamine, or

(f) for the manufacture of a compound of formula I in which one of W and X represents hydrogen and the other represents amino and Y represents hydrogen, reducing a compound of formula I in which W and X together represent hydroxyimino and Y represents hydrogen, or

(g) for the manufacture of a compound of formula I in which $R^1$ represents N-(diaralkylcarbamoyl or aralkanoyl)-histidyl, one of $R^5$ and $R^6$ represents hydrogen and the other represents hydrogen, alkyl, aryl, aralkyl, 1-alkoxycarbonyl-2-phenylethyl, 1-alkoxycarbonyl-2-(imidazol-4-yl)ethyl, 2-(imidazol-1-yl)ethyl), indanyl, heterocyclyl-alkyl, carboxyalkyl, alkoxycarbonylalkyl, aryloxycarbonylalkyl or a group of the formula $-A-N(R^a)(R^b)$ in which A represents alkylene and $R^a$ and $R^b$ each represent alkyl or $R^a$ and $R^b$ together represent a pentamethylene group in which one methylene group can be replaced by NH, N-alkyl, N-alkanoyl, O, S, SO or $SO_2$ or $R^5$ and $R^6$ together with the nitrogen atom to which they are attached represents a 1,2,3,4-tetrahydroisoquinoline ring, one of W and X represents hydrogen and the other represents hydroxy and Y represents hydrogen or hydroxy, hydrogenolyzing a compound of the general formula

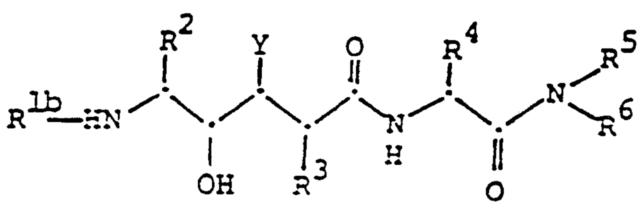

VIII

wherein $R^{1b}$ represents 3-(aralkoxymethyl)-N-(diaralkylcarbamoyl or aralkanoyl)-histidyl and $R^2$, $R^3$, $R^4$, $R^5$, $R^6$ and Y have the significance given earlier in this claim,

or

(h) for the manufacture of a compound of formula I in which $R^1$ represents alkoxycarbonyl, alkanoyl, aralkanoyl, aroyl, cycloalkylcarbonyl, heterocyclylcarbonyl, heterocyclyl-alkanoyl, 6-(dibenzylcarbamoyl)-4-oxohexanoyl or an acyl group of an α-amino acid in which the amino group is substituted by alkoxycarbonyl, diaralkylcarbamoyl, diaralkylalkanoyl or aralkanoyl and one of $R^5$ and $R^6$ represents hydrogen and the other represents carboxyalkyl or a group of the formula -A-N($R^a$)($R^b$) in which $R^a$ and $R^b$ together represent a pentamethylene group in which one methylene group is replaced by NH, hydrogenolyzing a corresponding compound of formula I which one of $R^5$ and $R^6$ represents hydrogen and the other represents aralkoxycarbonylalkyl or a group of the formula -A-N($R^a$)($R^b$) in which $R^a$ and $R^b$ together represent a pentamethylene group in which one methylene group is replaced by N-aralkoxycarbonyl, and

(i) if desired, converting a compound of formula I obtained into a pharmaceutically acceptable acid addition salt.

2. A process according to claim 1, wherein compounds of formula I wherein $R^1$ represents alkoxycarbonyl, aralkoxycarbonyl, alkanoyl, aralkanoyl, aroyl, cycloalkylcarbonyl, heterocyclylcarbonyl, heterocyclylalkanoyl, 6-(dibenzylcarbamoyl)-4-oxohexanoyl or an acyl group of a natural α-amino acid in which the amino group is substituted by aralkoxycarbonyl, diaralkylcarbamoyl, diaralkylalkanoyl or aralkanoyl; one of $R^5$ and $R^6$ represents hydrogen and the other represents hydrogen, alkyl, aryl, aralkyl, 1-alkoxycarbonyl-2-phenylethyl, 1-alkoxycarbonyl-2-(imidazol-4-yl)ethyl, 2-(imidazol-1-yl)ethyl, indanyl, heterocyclyl-alkyl or a group of the formula -A-N($R^a$)($R^b$) in which A represents alkylene and $R^a$ and $R^b$ each represent alkyl or $R^a$ and $R^b$ together represent a pentamethylene group in which one methylene group can be replaced by NH, N-alkyl, N-alkanoyl, O, S, SO or $SO_2$; or $R^5$ and $R^6$ together with the nitrogen atom to which they are attached represent a 1,2,3,4-tetrahydroisoquinoline ring; one of W and X represents hydrogen and the other represents hydroxy and Y represents hydrogen; and wherein the term "aryl" used alone or in combination means a phenyl or naphthyl group which optionally carries one or more substituents selected from alkyl, alkoxy and halogen; and their pharmaceutically acceptable acid addition salts are manufactured according to process embodiments (a), (b), (c), (d), (g) and/or (i).

3. A process according to claim 1 or claim 2, wherein $R^1$ represents alkoxycarbonyl, aralkoxycarbonyl, alkanoyl or aroyl or an acyl group of an α-amino acid in which the amino group is substituted by alkoxycarbonyl, aralkoxycarbonyl, diaralkylcarbamoyl, diaralkylalkanoyl or aralkyl.

4. A process according to claim 3, wherein $R^1$ represents tert.butoxycarbonyl, benzyloxycarbonyl, acetyl, tert.butylacetyl, 4-methylvaleryl, p-toluoyl, N-benzyloxycarbonylasparaginyl or N-benzyloxycarbonyl-ß-cyanoalanyl.

5. A process according to any one of claims 1 to 4, wherein $R^2$ represents isobutyl, cyclohexylmethyl or benzyl.

6. A process according to any one of claims 1 to 5, wherein $R^3$ represents alkyl.

7. A process according to claim 6, wherein $R^3$ represents methyl or isopropyl.

8. A process according to any one of claims 1 to 7, wherein $R^4$ represents isobutyl or sec.butyl.

9. A process according to any one of claims 1 to 8, wherein one of $R^5$ and $R^6$ represents hydrogen and the other represents hydrogen, alkyl, aralkyl, 1-alkoxy carbonyl-2-phenylethyl, 1-alkoxycarbonyl-2-(imidazol-4-yl)ethyl, 2-(imidazol-1-yl)ethyl, indanyl, heterocyclylalkyl or a group of the formula -A-N($R^a$)($R^b$) in which A represents alkylene and $R^a$ and $R^b$ each represent alkyl or $R^a$ and $R^b$ together represent a pentamethylene group in which one methylene group can be replaced by NH, N-alkyl, N-alkanoyl, N-aralkoxycarbonyl, O, S, SO or $SO_2$; or $R^5$ and $R^6$ together represent a 1,2,3,4-tetrahydroisoquinoline ring.

10. A process according to claim 9, wherein one of $R^5$ and $R^6$ represents hydrogen and the other represents hydrogen, isobutyl, benzyl, 2-phenylethyl, 2-(4-hydroxyphenyl)ethyl, 1-methoxycarbonyl-2-phenylethyl, 1-methoxycarbonyl-2-(imidazol-4-yl)ethyl, 2-(imidazol-1-yl)ethyl, 2-indanyl, 2-(2-pyridyl)ethyl, 2-(dimethylamino)ethyl, 2-morpholinoethyl or 2-[4-(benzyloxycarbonyl)-1-piperazinyl]ethyl or $R^5$ and $R^6$ together with the nitrogen atom to which they are attached represent a 1,2,3,4-tetrahydroisoquinoline ring.

11. A process according to any one of claims 1 to 10, wherein one of W and X represents hydrogen and the other represents hydroxy and Y represents hydrogen or hydroxy.

12. A process according to any one of claims 1 to 11, wherein $R^1$ represents tert.butoxycarbonyl, benzyloxycarbonyl, acetyl, tert.butylacetyl, 4-methylvaleryl, p-toluoyl, N-benzyloxycarbonylasparaginyl or N-benzyloxycarbonyl-ß-cyanoalanyl, $R^2$ represents isobutyl, cyclohexylmethyl or benzyl, $R^3$ represents methyl or isopropyl, $R^4$ represents isobutyl or sec.butyl and one of $R^5$ and $R^6$ represents hydrogen and the other represents hydrogen, isobutyl, benzyl, 2-phenylethyl, 2-(4-hydroxyphenyl)ethyl, 1-methoxycarbonyl-2-phenylethyl, 1-methoxycarbonyl-2-(imidazol-4-yl)ethyl, 2-(imidazol-1-yl)ethyl, 2-indanyl, 2-(2-pyridyl)ethyl, 2-(dimethyl amino)ethyl, 2-morpholinoethyl of 2-[4-(benzyloxycarbonyl)-1-piperazinyl]ethyl or $R^5$ and $R^6$ together with the nitrogen atom to which they are attached represent a 1,2,3,4-tetrahydroisoquinoline ring and one of W and X represents hydrogen and the other represents hydroxy and Y represents hydrogen or hydroxy.

13. A process according to claim 2, wherein $N^2$-[5(S)-(tert.butoxyformamido)-6-cyclohexyl-4(S)-hydroxy-

2(S)-isopropylhexanoyl]-$N^1$-phenethyl-L-isoleucinamide is prepared.

14. A process according to claim 2, wherein N-[N-[5(S)-(tert.butoxyformamido)-6-cyclohexyl-4(S)-hydroxy-2(S)-isopropylhexanoyl]-L-isoleucyl]-L-phenylalanine methyl ester is prepared.

15. A process according to claim 2, wherein $N^2$-[5(S)-(tert.butoxyformamido)-6-cyclohexyl-4(S)-hydroxy-2(S)-isopropylhexanoyl]-$N^1$-benzyl-L-isoleucinamide is prepared.

16. A process according to claim 2, wherein $N^2$-[5(S)-(tert.butoxyformamido)-6-cyclohexyl-4(S)-hydroxy-2(S)-isopropylhexanoyl]-$N^1$-[2-(2-pyridyl)ethyl]-L-isoleucinamide is prepared.

17. A process according to claim 2, wherein $N^2$-[5(S)-(tert.butoxyformamido)-6-cyclohexyl-4(S)-hydroxy-2(S)-isopropylhexanoyl]-$N^1$-(2-indanyl)-L-isoleucinamide is prepared.

18. A process according to claim 2, wherein $N^2$-[5(S)-(tert.butoxyformamido)-6-cyclohexyl-4(S)-hydroxy-2(S)-isopropylhexanoyl]-$N^1$-(2-morpholinoethyl)-L-isoleucinamide is prepared.

19. A process according to claim 2, wherein $N^2$-[5(S)-(tert.butoxyformamido)-6-cyclohexyl-2(S)-isopropyl-6-phenylhexanoyl]-$N^1$-phenethyl-L-isoleucinamide is prepared.

20. A process according to claim 2, wherein $N^2$-[5(S)-benzyloxyformamido-6-cyclohexyl-4(S)-hydroxy-2-(S)-isopropylhexanoyl]-$N^1$-phenethyl-L-isoleucinamide is prepared.

21. A process according to claim 2, wherein $N^2$-[5(S)-[[N-(benzyloxycarbonyl)-L-asparaginyl]-amino]-6-cyclohexyl-4(S)-hydroxy-2(S)-isopropylhexanoyl]-$N^1$-phenethyl-L-isoleucinamide is prepared.

22. A process according to claim 2, wherein $N^2$-[5(S)-(tert.butylacetamido)-6-cyclohexyl-4(S)-hydroxy-2-(S)-isopropylhexanoyl]-$N^1$-phenethyl-L-isoleucinamide is prepared.

23. A process according to claim 1, wherein $N^2$-[5(S)-[[N-(benzyloxycarbonyl)-ß-cyano-L-alanyl]amino]-6-cyclohexyl-4(S)-hydroxy-2(S)-isopropylhexanoyl[-$N^1$-phenethyl-L-isoleucinamide is prepared.

24. A process according to claim 1, wherein $N^1$-[2-[4-(benzyloxycarbonyl)-1-piperazinyl]ethyl]-$N^2$-[5(S)-(tert.butoxyformamido)-6-cyclohexyl-4(S)-hydroxy-2(S)-isopropylhexanoyl]-L-isoleucinamide is prepared.

25. A process according to claim 1, wherein $N^2$-[5(S)-(tert.butoxyformamido)-6-cyclohexyl-4(S)-hydroxy-2(S)-isopropylhexanoyl]-$N^1$-[2-(4hydroxyphenyl)ethyl]-L-isoleucinamide is prepared.

26. A process according to claim 1, wherein $N^1$-[2-[4-(benzyloxycarbonyl)-1-piperazinyl]ethyl]-$N^2$-[5(S)-(tert.butoxyformamido)-6-cyclohexyl-3(R),4(R)-dihydroxy-2(R)-isopropylhexanoyl]-L-isoleucinamide is prepared.

27. A process for the manufacture of a medicament, particularly to be used in the treatment or prophylaxis of viral infections, which process comprises bringing a compound of formula I set forth in claim 1 or a pharmaceutically acceptable acid addition salt thereof into a galenical dosage form.

28. The use of a compound of formula I set forth in claim 1 or a pharmaceutically acceptable acid addition salt thereof for the manufacture of a medicament for the treatment or prophylaxis of viral infections.

29. Compounds of the general formulae

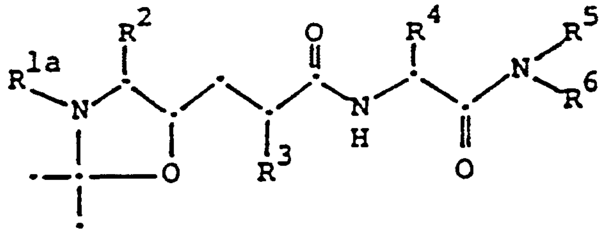

II

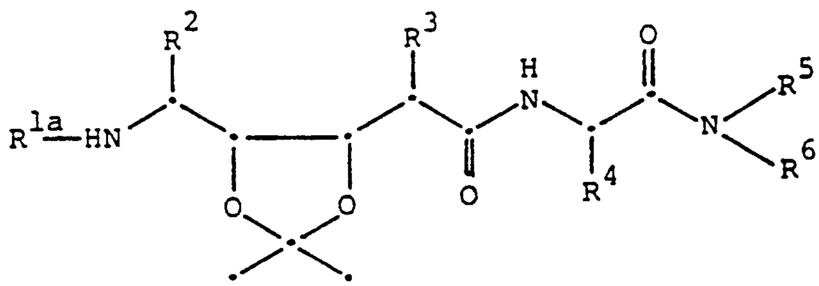

III

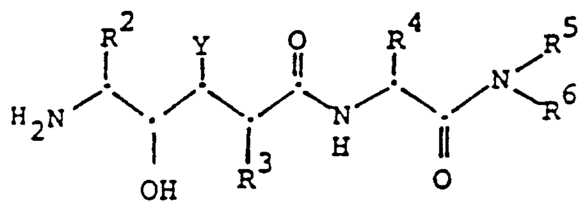

IV

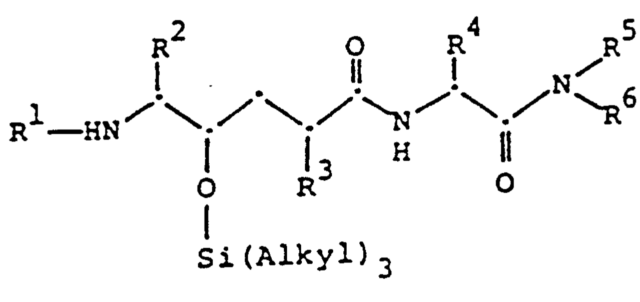

V

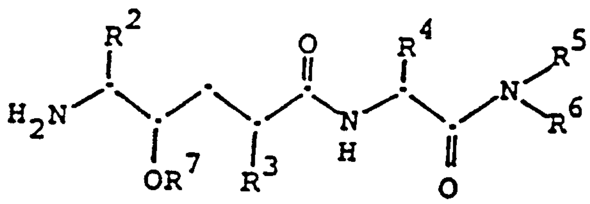

VI

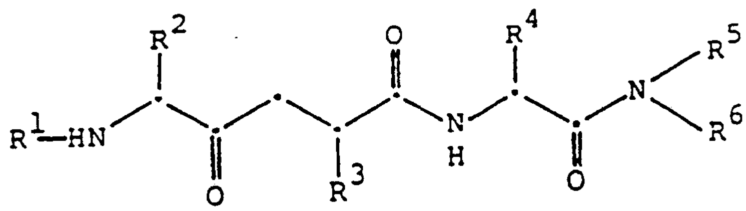

VII

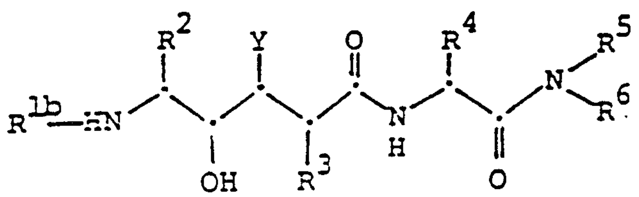

VIII

wherein $R^1$ represents alkoxycarbonyl, aralkoxycarbonyl, alkanoyl, aralkanoyl, aroyl, cycloalkylcarbonyl, heterocyclylcarbonyl, heterocyclyl-alkanoyl, 6-(dibenzylcarbamoyl)-4-oxohexanoyl or an acyl group of an α-amino acid in which the amino group is substituted by alkoxycarbonyl, aralkoxycarbonyl, diaralkylcarbamoyl, diaralkylalkanoyl or aralkanoyl; $R^{1a}$ represents alkoxycarbonyl or aralkoxycarbonyl, $R^{1b}$ represents 3-(aralkoxymethyl)-N-(diarylcarbamoyl or aralkanoyl)-histidyl; $R^2$ represents alkyl, cycloalkylalkyl or aralkyl; $R^3$ represents hydrogen or alkyl; $R^4$ represents alkyl; and one of $R^5$ and $R^6$ represents hydrogen and the other represents hydrogen, alkyl, aryl, aralkyl, 1-alkoxycarbonyl-2-phenylethyl, 1-alkoxycarbonyl-2-(imidazol-4-yl)ethyl, 2-(imidazol-1-yl)ethyl, indanyl, heterocyclyl-alkyl, carboxyalkyl, alkoxycarbonylalkyl, aryloxycarbonylalkyl, aralkoxycarbonylalkyl or a group of the formula -A-N($R^a$)($R^b$) in which A represents alkylene and $R^a$ and $R^b$ each represent alkyl or $R^a$ and $R^b$ together represent a pentamethylene group in which one methylene group can be replaced by NH, N-alkyl, N-alkanoyl, N-aralkoxycarbonyl, O, S, SO or $SO_2$; or $R^5$ and $R^6$ together with the nitrogen atom to which they are attached represent a 1,2,3,4-tetrahydroisoquinoline ring, $R^7$ represents alkanoyl and Y represents hydrogen or hydroxy.